# EUROPEAN PATENT SPECIFICATION

(11) **EP 0 989 983 B1**
(45) Date of publication and mention of the grant of the patent: **11.08.2004**
(21) Application number: 98928886.5
(22) Date of filing: 15.06.1998
(51) Int. Cl.: C07D 401/14, A61K 31/55, C07D 401/04, A61K 31/495, A61K 31/44

(54) **BENZPYRIDO CYCLOHEPTANE COMPOUNDS USEFUL FOR INHIBITION OF FARNESYL PROTEIN TRANSFERASE**
BENZPYRIDO CYCLOHEPTAN DERIVATE MIT FARNESYL PROTEIN TRANSFERASE INHIBIERENDER WIRKUNG
COMPOSES A BASE DE CYCLOHEPTANE BENZPYRIDO SERVANT D'INHIBITEURS DE FARNESYL PROTEINE TRANSFERASE

(30) Priority: 17.06.1997 US 877741
(43) Date of publication of application: 05.04.2000
(73) Proprietor: SCHERING CORPORATION, Kenilworth, New Jersey 07033-0530 (US); PHARMACOPEIA, INC., Princeton, NJ 08540 (US)
(72) Inventor: COOPER, Alan, B., West Caldwell, NJ (US); DOLL, Ronald, J., Maplewood, NJ 07040 (US); GIRIJAVALLABHAN, Viyyoor, M., Parsippany, NJ 07054 (US); GANGULY, Ashit, Upper Montclair, NJ 07043 (US); READER, John, C., Cambridge CB1 6JS (GB); BALDWIN, John, J., Gwynedd Valley, PA 19437 (US); HUANG, Chia-yu, Plainsboro, NJ 08536 (US)
(74) Representative: Ritter, Stephen David
(86) International application number: PCT/US1998/011495
(87) International publication number: WO 1998/057960

(56) References cited:
- WO-A-95/10515
- WO-A-95/10516
- WO-A-96/31477
- WO-A-96/31478
- WO-A-96/31505
- BISHOP W R ET AL: "NOVEL TRICYCLIC INHIBITORS OF FARNESYL PROTEIN TRANSFERASE BIOCHEMICAL CHARACTERIZATION AND INHIBITION OF RAS MODIFICATION IN TRANSFECTED COS CELLS" JOURNAL OF BIOLOGICAL CHEMISTRY, vol. 270, no. 51, 22 December 1995, pages 30611-30618, XP002050604
- NJOROGE F G ET AL: "NOVEL TRICYCLIC AMINOACETYL AND SULFONAMIDE INHIBITORS OF RAS FARNESYL PROTEIN TRANSFERASE" BIOORGANIC & MEDICINAL CHEMISTRY LETTERS, vol. 6, no. 24, 1996, pages 2977-2982, XP002056550
- BUSS J E ET AL: "FARNESYL TRANSFERASE INHIBITORS: THE SUCCESSES AND SURPRISES OF A NEW CLASS OF POTENTIAL CANCER CHEMOTHERAPEUTICS" CHEMISTRY AND BIOLOGY, vol. 118, no. 2, December 1995, pages 787-791, XP002056549
- NJOROGE F G ET AL: "DISCOVERY OF NOVEL NONPEPTIDE TRICYCLIC INHIBITORS OF RAS FARNESYL PROTEIN TRANSFERASE" BIOORGANIC & MEDICINAL CHEMISTRY, vol. 5, no. 1, 1997, pages 101-113, XP002056551

## Description

The present invention relates to inhibitors of famesyl protein transferase.

### BACKGROUND

Bishop *et al.,* J. Biol. Chem. (1995) 270, pp 30611-30618 and *Buss et al.,* Chem. and Biol. (1995), 2, pp 787-791 discloses the 8-chloro, piperidyl-substituted compound SCH 44342 as an inhibitor of famesyl protein transferase:

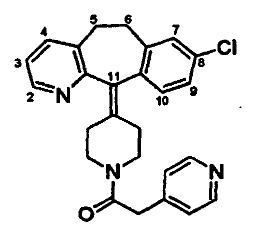

Tricyclic aminoacetyl and sulfonamide inhibitors of famesyl protein transferase are disclosed in Njoroge *et al.,* Bioorg. Med. Chem. Lett., (1996) 6, pp 2977-2982.

Further tricyclic compounds useful for the inhibition of farnesyl protein transferase are disclosed in WO 96/31478 and WO 96/30515, which are both directed to 3,8-disubstituted tricyclic compounds and WO 96/31477, which is directed to 3,8-disubsituted compounds that contain a -C=O linker group at the C-11 position of the tricyclic ring.

Tricyclic compounds useful for the inhibition of farnesyl protein transferase are also disclosed in WO 95/10516, WO 95/10515, and Njoroge *et. al.,* Bioorg. Med. Chem. Lett., (1997), 5, pp 101-113. WO 95/10516 discloses, in the specific examples, a 3,4,8-tri-halo substituted compound.

In view of the current interest in inhibitors of farnesyl protein transferase, a welcome contribution to the art would be further compounds useful for the inhibition of famesyl protein transferase. Such a contribution is provided by this invention.

### SUMMARY OF THE INVENTION

This invention provides compounds useful for the inhibition of farnesyl protein transferase (FPT). The compounds of this invention are represented by the formula: 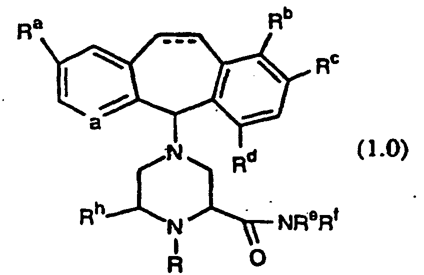 or a pharmaceutically acceptable salt or solvate thereof, wherein:
a represents N or NO-;
R^{a}, R^{c}, and R^{d} are halo and R^{b} is H;
the dotted line (---) represents an optional double bond;
R is selected from the group consisting of H, -S(O)₂R¹, -S(O)₂NR¹R², -C(O)R¹, and -C(O)NR¹R², wherein R¹ and R² are independently selected from the group consisting of H, alkyl, aryl, arylalkyl, heteroaryl, heteroarylalkyl, (C₃-C₇) cycloalkyl, cycloalkylalkyl, heterocycloalkyl, substituted alkyl, substituted aryl, substituted arylalkyl, substituted heteroaryl, substituted heteroarylalkyl, substituted (C₃-C₇) cycloalkyl, substituted cycloalkylalkyl, substituted heterocycloalkyl, wherein said substituted groups have one or more substituents selected from: alkyl, alkoxy, aralkyl, heteroarylalkyl, -NO₂, alkyloxyalkyl, alkyloxyalkyloxyalkyl, C₃-C₇ cycloalkyl, aryl, -CN, heteroaryl, heterocycloalkyl, =O, -OH, amino, substituted amino, nitro and halo;
R^{e} and R^{f} are independently selected from H, alkyl, alkyloxyalkyl, alkyloxyalkyloxyalkyl, aryl, arylalkyl, heteroaryl, heteroarylalkyl, (C₃-C₇) cycloalkyl, cycloalkylalkyl, heterocycloalkyl, substituted alkyl, substituted alkyloxyalkyl, substituted alkyloxyalkyloxyalkyl, substituted aryl, substituted arylalkyl, substituted heteroaryl, substituted heteroarylalkyl, substituted (C₃-C₇) cycloalkyl, substituted cycloalkylalkyl, substituted heterocycloalkyl, wherein said substituted groups have one or more substituents selected from: alkyl, alkoxy, aralkyl, heteroarylalkyl, -NO₂, alkyloxyalkyl, alkyloxyalkyloxyalkyl, C₃-C₇ cycloalkyl, aryl, -CN, heteroaryl, heterocycloalkyl, =O, -OH, amino, substituted amino, nitro and halo; or R^{e} is selected from the group consisting of H, alkyl and aryl and R^{f} is represented by ―(CH₂)ₙ―R¹⁵, wherein n is an integer from 0 to 8 and R¹⁵ is selected from -C(O)NH₂, -SO₂NH₂, aryl, heteroaryl, cycloalkyl, heterocycloalkyl, optionally substituted by alkyl, alkoxy, aralkyl, heteroarylalkyl, -NO₂, alkyloxyalkyl, alkyloxyalkyloxyalkyl, C₃-C₇ cycloalkyl, aryl, - CN, heterocycloalkyl, =O, -OH, amino, substituted amino, nitro and halo;
or R¹⁵ is 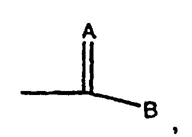 wherein B is OH or NH₂ and A is NH, O, NOH or NCN, or R¹⁵ is NR¹⁶R¹⁷, wherein R¹⁶ is H or alkyl and R¹⁷ is H, alkyl, SO₂CH₃, or C(O)NH₂; or R^{e} and R^{f} together with the nitrogen to which they are bound, form a 5 or 6 membered heterocycloalkyl ring which is optionally substituted by OH, NH₂, NHR¹⁶, NHR¹⁷, NR¹⁶R¹⁷, or (CH₂)ₙR¹⁸R¹⁹, wherein R¹⁶ and R¹⁷ are as defined above, R¹⁸ is H or C₁-C₆ alkyl, and R¹⁹ is selected from H, C₁-C₆ alkyl, substituted alkyl, arylalkyl, acyl (e.g., acetyl, benzoyl, etc.), carboxamido, alkyloxycarbonyl (e.g., methoxycarbonyl), arylalkyloxycarbonyl (e.g., benzyloxycarbonyl), amido derivatives derived from amino acids (e.g., glycine,alnine, serine,etc.), imidate (e.g., phenoxyimidate), cyanide, imidamido (e.g., C(=NH)NH₂, (C=NSO₂NH₂)NH₂, etc.), sulfonamido (e.g., SO₂NH₂, SO₂N(CH₃)₂) sulfonyl (e.g., SO₂CH₃, SO₂C₆H₅, SO₂CH₂C₆H₅, etc.), phosphinate (e.g., P(=O)(CH₃)₂), heterocyclyl and imidamido (e.g., (C=NC₆H₅)C₆H₅), (C=NH)C₆H₅ ,etc.), wherein n is as defined above; and R^{h} is H or =O;
with the further proviso that when R^{h} is H and R^{b} and R^{d} are both H, R^{e} is H and R^{f} is 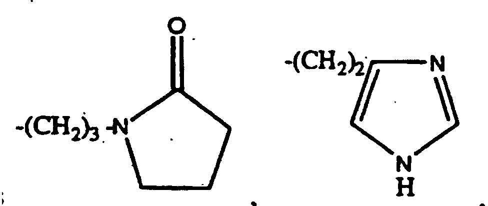 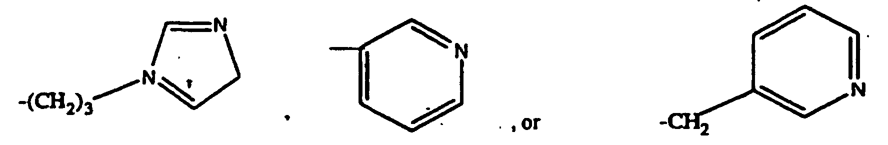 wherein "alkyl'', "alkyloxyalkyl", "alkyloxyalkyloxyalkyl", "cycloalkyl", "heterocycloalkyl", "aryl", "halo" and "heteroaryl" are defined below.

The compounds of this invention: (i) potently inhibit farnesyl protein transferase, but not geranylgeranyl protein transferase I, in vitro; (ii) block the phenotypic change induced by a form of transforming Ras which is a farnesyl acceptor but not by a form of transforming Ras engineered to be a geranylgeranyl acceptor; (iii) block intracellular processing of Ras which is a farnesyl acceptor but not of Ras engineered to be a geranylgeranyl acceptor; and (iv) block abnormal cell growth in culture induced by transforming Ras.

The compounds of this invention inhibit farnesyl protein transferase and the farnesylation of the oncogene protein Ras. Thus, this invention further provides a method of inhibiting farnesyl protein transferase, (e.g., ras farnesyl protein transferase) in mammals, especially humans, by the administration of an effective amount of the tricyclic compounds' described above. The administration of the compounds of this invention to patients, to inhibit farnesyl protein transferase, is useful in the treatment of the cancers described below.

This invention provides the use of a compound of formula (1.0) for the manufacture of a medicament for inhibiting or treating the abnormal growth of cells, including transformed cells, by administering an effective amount of a compound of this invention. Abnormal growth of cells refers to cell growth independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; and (3) benign and malignant cells of other proliferative diseases in which aberrant Ras activation occurs.

This invention also provides the use of a compound of formula (1.0) for the manufacture of a medicament for inhibiting or treating tumor growth by administering an effective amount of the tricyclic compounds, described herein, to a mammal (e.g., a human) in need of such treatment. In particular, this invention provides the use of a compound of formula (1.0) for the manufacture of a medicament for inhibiting or treating the growth of tumors expressing an activated Ras oncogene by the administration of an effective amount of the above described compounds. Examples of tumors which may be inhibited or treated include, but are not limited to, lung cancer (e.g., lung adenocarcinoma), pancreatic cancers (e.g., pancreatic carcinoma such as, for example, exocrine pancreatic carcinoma), colon cancers (e.g., colorectal carcinomas, such as, for example, colon adenocarcinoma and colon adenoma), myeloid leukemias (for example, acute myelogenous leukemia (AML)), thyroid follicular cancer, myelodysplastic syndrome (MDS), bladder carcinoma, epidermal carcinoma, breast cancer and prostate cancer

It is believed that this invention also provides the use of a compound of formula (1.0) in the manufacture of a medicament for inhibiting or treating proliferative diseases, both benign and malignant, wherein Ras proteins are aberrantly activated as a result of oncogenic mutation in other genes--i.e., the Ras gene itself is not activated by mutation to an oncogenic form--with said inhibition or treatment being accomplished by the administration of an effective amount of the tricyclic compounds described herein, to a mammal (e.g., a human) in need of such treatment. For example, the benign proliferative disorder neurofibromatosis, or tumors in which Ras is activated due to mutation or overexpression of tyrosine kinase oncogenes (e.g., neu, src, abl, lck, and fyn), may be inhibited or treated by the tricyclic compounds described herein.

The tricyclic compounds useful in this invention inhibit or treat the abnormal growth of cells. Without wishing to be bound by theory, it is believed that these compounds may function through the inhibition of G-protein function, such as ras p21, by blocking G-protein isoprenylation, thus making them useful in the treatment of proliferative diseases such as tumor growth and cancer. Without wishing to be bound by theory, it is believed that these compounds inhibit ras farnesyl protein transferase, and thus show antiproliferative activity against ras transformed cells.

### DETAILED DESCRIPTION OF THE INVENTION

As used herein, the following terms are used as defined below unless, otherwise indicated:
MH⁺-represents the molecular ion plus hydrogen of the molecule in the mass spectrum;
Bu-represents butyl;
Boc represents a tert-butoxycarbonyl group
Et-represents ethyl;
Me-represents methyl;
Ph-represents phenyl;
benzotriazol-1-yloxy represents 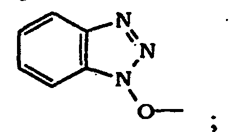
1-methyl-tetrazol-5-ylthio represents 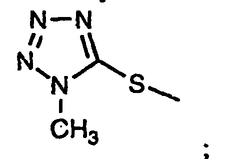
alkyl-(including the alkyl portions of alkoxy, alkylamino and dialkylamino)-represents straight and branched carbon chains and contains from one to twenty carbon atoms, preferably one to six carbon atoms;
alkyloxyalkyl represents alkyl bound to an oxygen atom, which in turn is bound to alkyl.
alky oxyalkyloxyalkyl represents alkyl bound to oxygen, which in turn is bound to alkyl, which in turn is bound to oxygen, which in turn, is bound to alkyl (e.g., CH₂CH₂OCH₂CH₂OCH₂CH₃)
alkanediyl-represents a divalent, straight or branched hydrocarbon chain having from 1 to 20 carbon atoms, preferably 1 to 6 carbon atoms, the two available bonds being from the same or different carbon atoms thereof, e.g., methylene, ethylene, ethylidene, -CH₂CH₂CH₂-, -CH₂CHCH₃, -CHCH₂CH₃, etc.
cycloalkyl-represents saturated carbocyclic rings branched or unbranched of from 3 to 20 carbon atoms, preferably 3 to 7 carbon atoms;
heterocycloalkyl-represents a saturated, branched or unbranched carbocyclic ring containing from 3 to 15 carbon atoms, preferably from 4 to 6 carbon atoms, which carbocyclic ring is interrupted by 1 to 3 hetero groups selected from -O-, -S- or - NR¹⁰- , wherein R¹⁰ is H, alkyl, aryl, or arylalkyl (suitable heterocycloalkyl groups including 2- or 3-tetrahydrofuranyl, 2- or 3- tetrahydrothienyl, 2-, 3- or 4-piperidinyl, 2- or 3-pyrrolidinyl, 1-, 2- or 3-piperizinyl, 2- or 4-dioxanyl, etc.);
alkenyl-represents straight and branched carbon chains having at least one carbon to carbon double bond and containing from 2 to 12 carbon atoms, preferably from 2 to 6 carbon atoms and most preferably from 3 to 6 carbon atoms;
alkynyl-represents straight and branched carbon chains having at least one carbon to carbon triple bond and containing from 2 to 12 carbon atoms, preferably from 2 to 6 carbon atoms;
aryl (including the aryl portion of aryloxy and aralkyl)-represents a carbocyclic group containing from 6 to 15 carbon atoms and having at least one aromatic ring (e.g., aryl is a phenyl ring), with all available substitutable carbon atoms of the carbocyclic group being intended as possible points of attachment, said carbocyclic group being optionally substituted (e.g., 1 to 3) with one or more of halo, alkyl, hydroxy, alkoxy, phenoxy, CF₃, amino, alkylamino, dialkylamino, -COOR¹⁰ or -NO₂; and
halo-represents fluoro, chloro, bromo and iodo; and
heteroaryl-represents cyclic groups, optionally substituted, and having at least one heteroatom selected from O, S or N, said heteroatom interrupting a carbocyclic ring structure and having a sufficient number of delocalized pi electrons to provide aromatic character, with the aromatic heterocyclic groups preferably containing from 2 to 14 carbon atoms, e.g., triazolyl, 2-, 3- or 4-pyridyl or pyridyl N-oxide (optionally substituted with R³ and R⁴), wherein pyridyl N-oxide can be represented as:

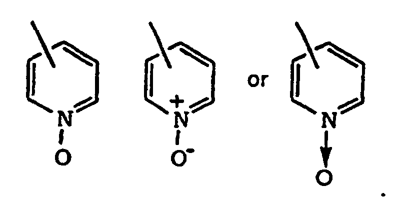

The following solvents and reagents are referred to herein by the abbreviations indicated: tetrahydrofuran (THF); CDI (carbonyl di-imidazole )ethanol (EtOH); methanol (MeOH); acetic acid (HOAc or AcOH); ethyl acetate (EtOAc); N,N-dimethylformamide (DMF); trifluoroacetic acid (TFA); trifluoroacetic anhydride (TFAA); 1-hydroxybenzotriazole (HOBT); m-chloroperbenzoic acid (MCPBA); triethylamine (Et₃N); diethyl ether (Et₂O); ethyl chloroformate (ClCO₂Et); 1-(3-dimethylaminopropyl)-3-ethyl carbodiimide hydrochloride (DEC); disobutylaluminum hydride (DIABL); and 4-methylmorpholine (NMM).

The positions in the tricyclic ring system are:

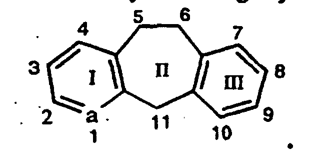

Preferred halo atoms for R^{a}, R^{b}, R^{c}, and R^{d} in Formula 1.0 are selected from: Br, Cl or I, with Br and Cl being preferred.

Compounds of Formula 1.0 include compounds of the formula:

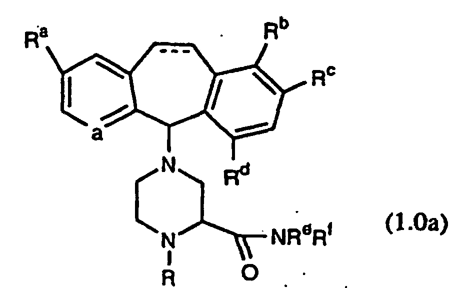

Compounds of Formula 1.0 include compounds of the formula:

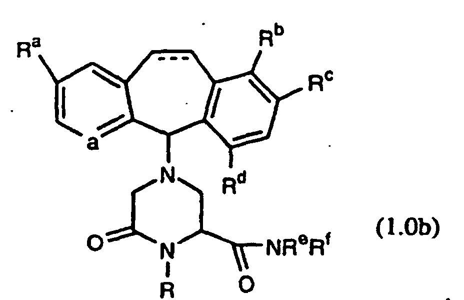

Preferably, for the compounds of this invention, R^{a}, R^{b}, R^{c} and R^{d} are selected from halo, preferably Br or Cl, and more preferably, R^{a} is Br and R^{c} is Cl. Preferably, only one of R^{a}, R^{b}, R^{c}, and R^{d} is H.

Preferably, in Formula (1.0a), R^{a} is Br, R^{c} is Cl, and R^{b} or R^{d} is halo. More preferably, in Formula (1.0a), R^{a} is Br, R^{c} is Cl, and R^{b} or R^{d} is Br.

Preferably, for the compounds of this invention, one of R^{e} Or R^{f} is H, and the other is -(CH₂)ₙ-R¹⁵, wherein R¹⁵ is selected from: alkyloxyalkyl, NHBoc,

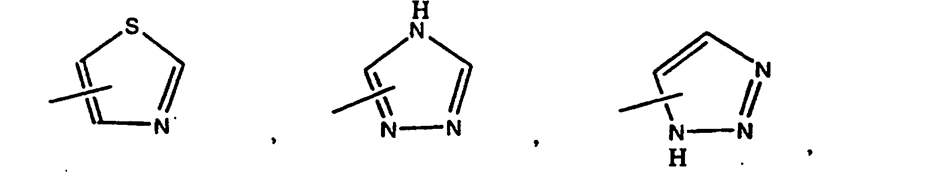

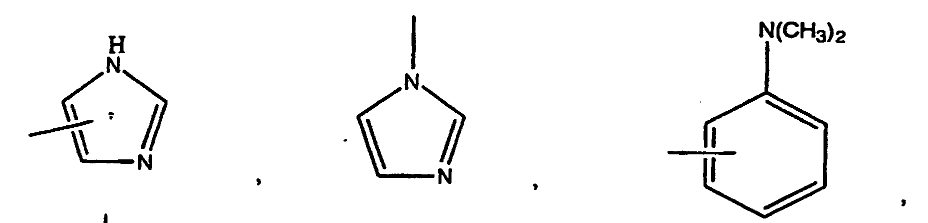

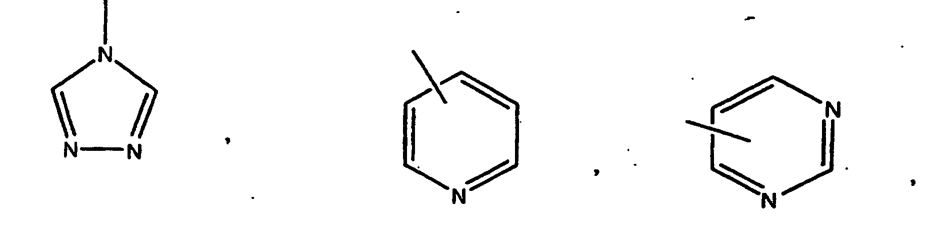

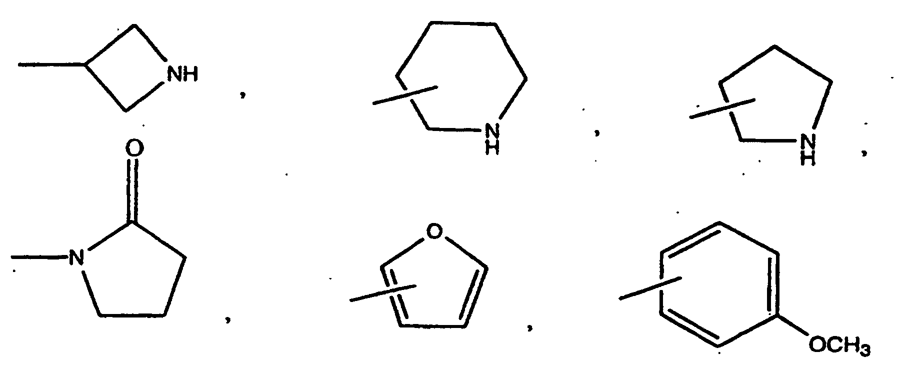

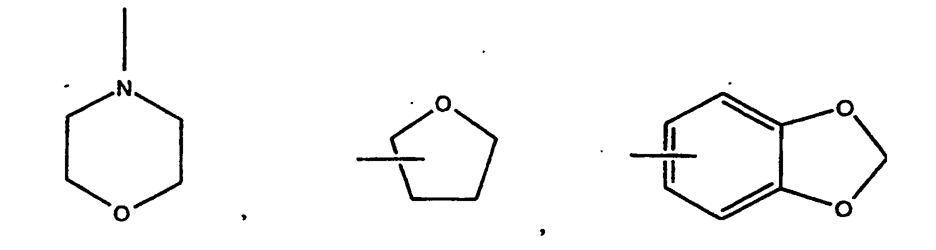

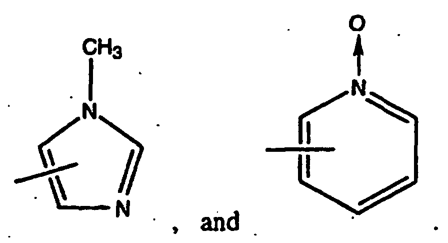

Preferably, for the compounds of this invention, the optional double bond between positions 5 and 6 (i.e., C5-C6) in the tricyclic system is absent.

Also, preferably, for the compounds of this invention, substituent a in Ring I represents N.

Thus, compounds of the invention include compounds of the formulas:

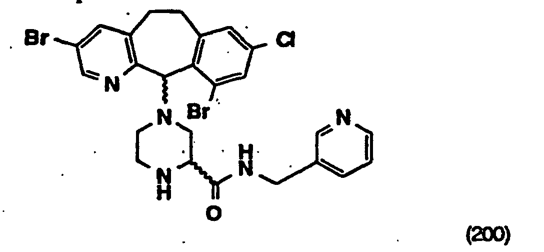

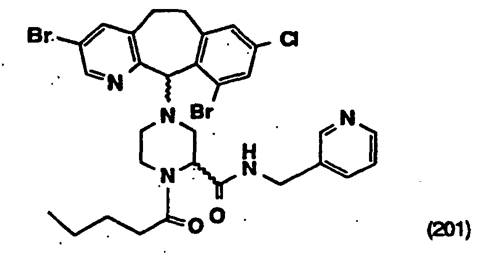

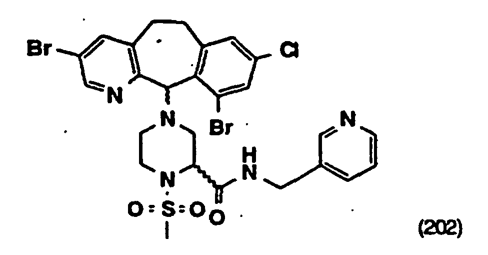

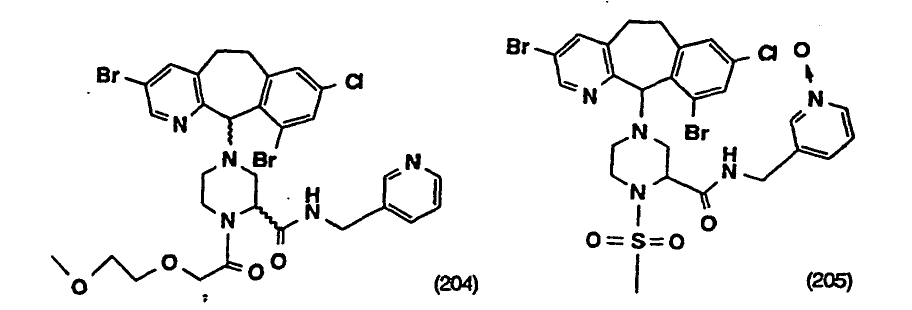

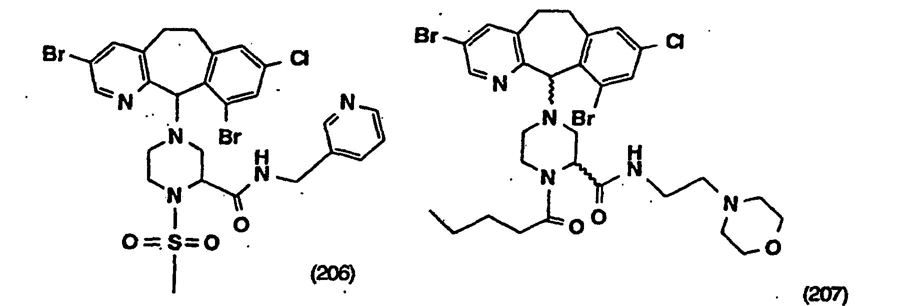

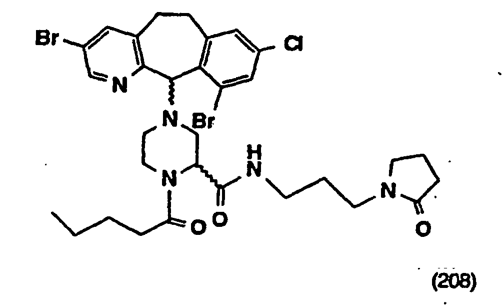

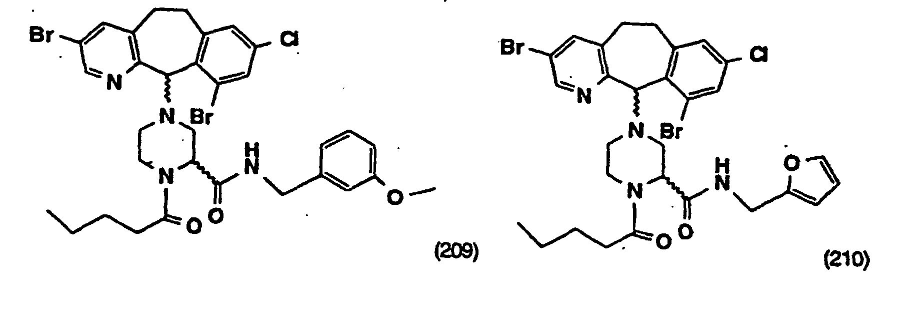

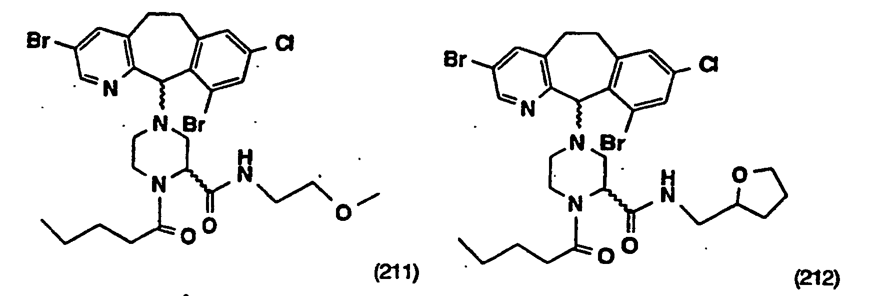

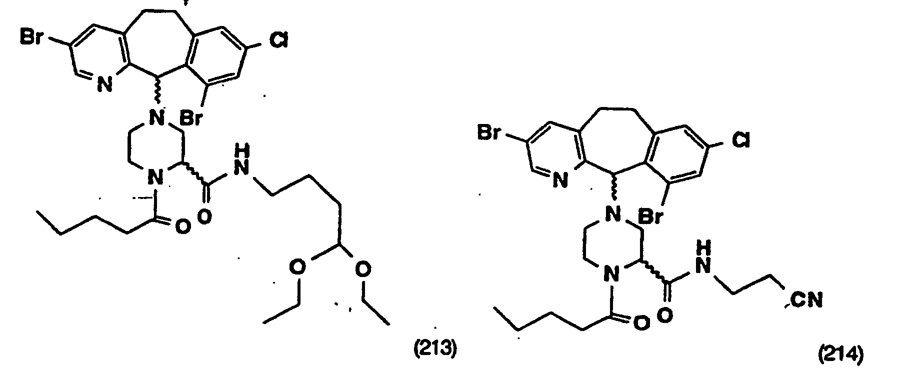

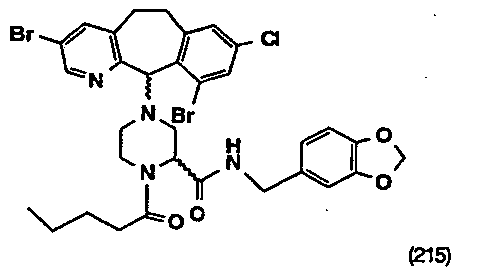

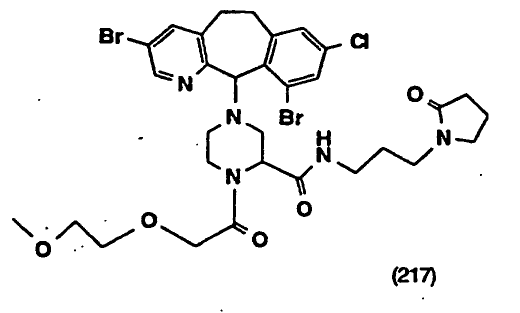

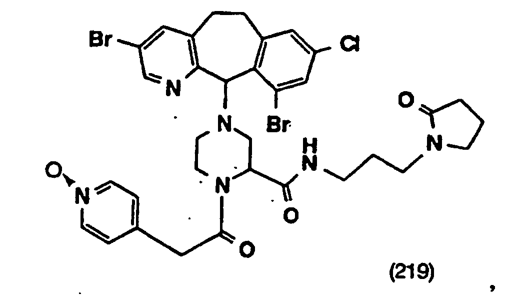

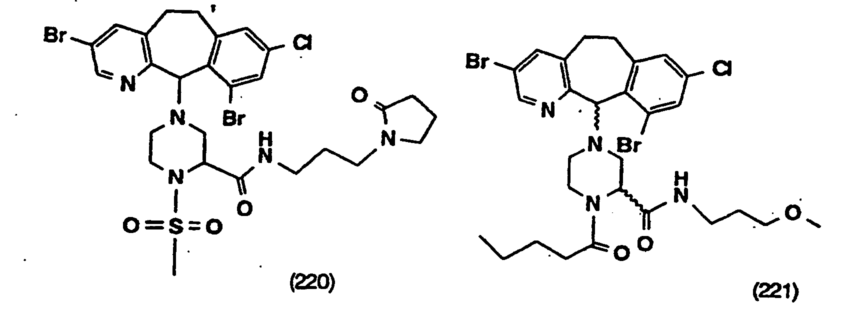

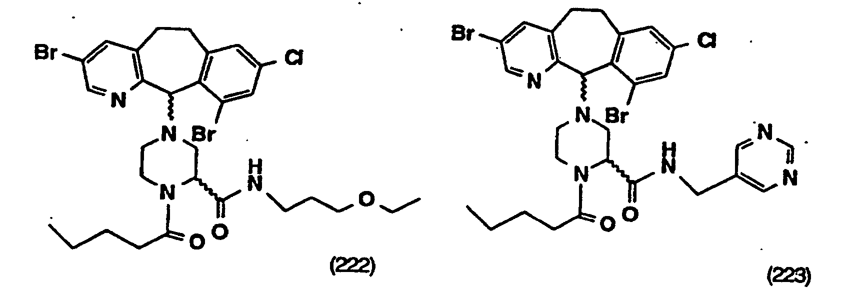

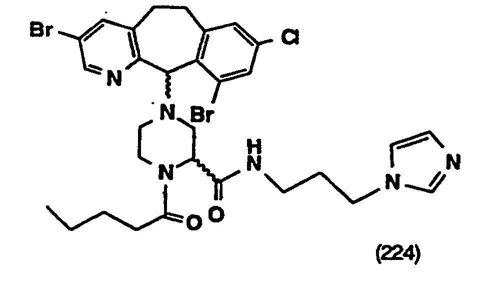

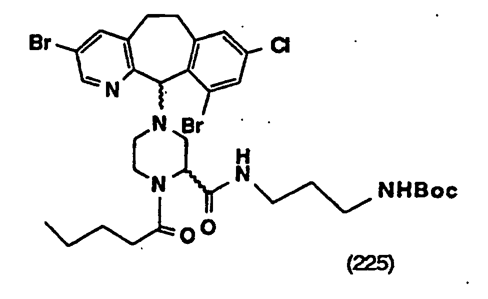

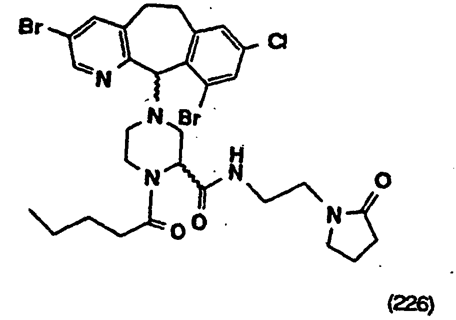

The following compounds, which may be prepared according to the methods disclosed in WO96/31478 and/or WO95/15016, are also within the scope of the present invention:

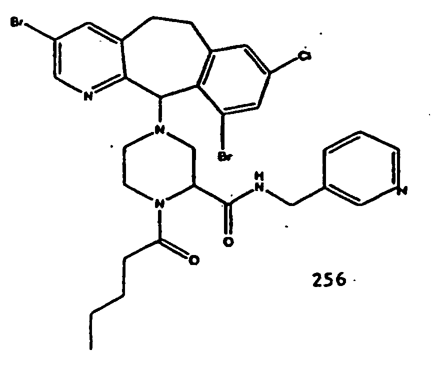

Lines drawn into the ring systems indicate that the indicated bond may be attached to any of the substitutable ring carbon atoms.

Certain compounds of the invention may exist in different isomeric (e.g., enantiomers and diastereoisomers) forms. The invention contemplates all such isomers both in pure form and in admixture, including racemic mixtures. Enol forms are also included.

Certain tricyclic compounds will be acidic in nature, e.g. those compounds which possess a carboxyl or phenolic hydroxyl group. These compounds may form pharmaceutically acceptable salts. Examples of such salts may include sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic tricyclic compounds also form pharmaceutically acceptable salts, e.g., acid addition salts. For example, the pyrido-nitrogen atoms may form salts with strong acid, while compounds having basic substituents such as amino groups also form salts with weaker acids. Examples of suitable acids for salt 'formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous NaOH, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all acid arid base salts are considered equivalent to the free forms of the corresponding compounds for purposes of the invention.

Compounds of the invention may be prepared according to the procedures described in WO 96/30363, published October 3, 1996, WO96/31478, published October 10, 1996, WO 95/10516 published April 20, 1995, copending Application Serial No. 08/410,187 filed March 24, 1995, copending Application Serial No. 08/577,951 filed December 22, 1995, and copending Application Serial No. 08/615,760 filed March 13, 1996; the disclosures of each being incorporated herein by reference thereto; and according to the procedures described below.

Compounds of the fomula 1.0a can be prepared from a tricyclic hydroxy compound (2.0) as shown in Reaction Scheme 1.

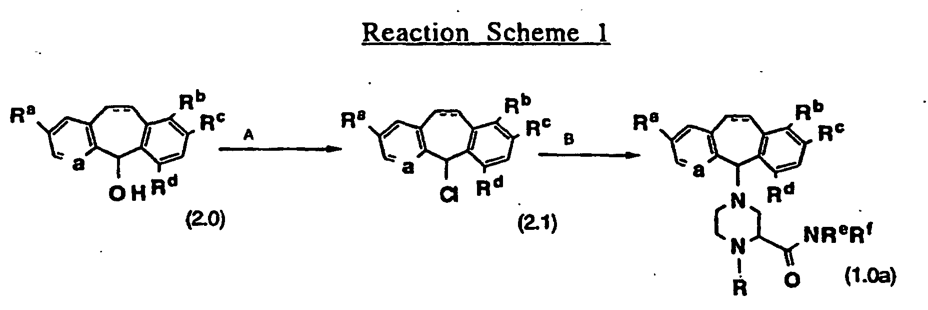

Compound (2.0), which may be prepared as described in WO 95/10516, published April 20, 1995 and in U.S. 5,151,423, can be chlorinated with a chlorinating agent, e.g., thionyl chloride, as described therein to form Compound (2.1). Compound (2.1) may then be reacted with a suitably. substituted piperazine to form Compound (1.0a) The reaction with the piperazine is carried out in a suitable solvent, e.g., DMF or THF in the presence of a base, e.g., triethylamine, at a temperature of from 0 to 80 C for 1 - 24 hours, then added to water, extracted with a suitable solvent, e.g., ethyl acetate, and chromatographed on silica gel.

Alternatively, compounds of the formula (1.0a) can be generally prepared as shown in Reaction Scheme 2.

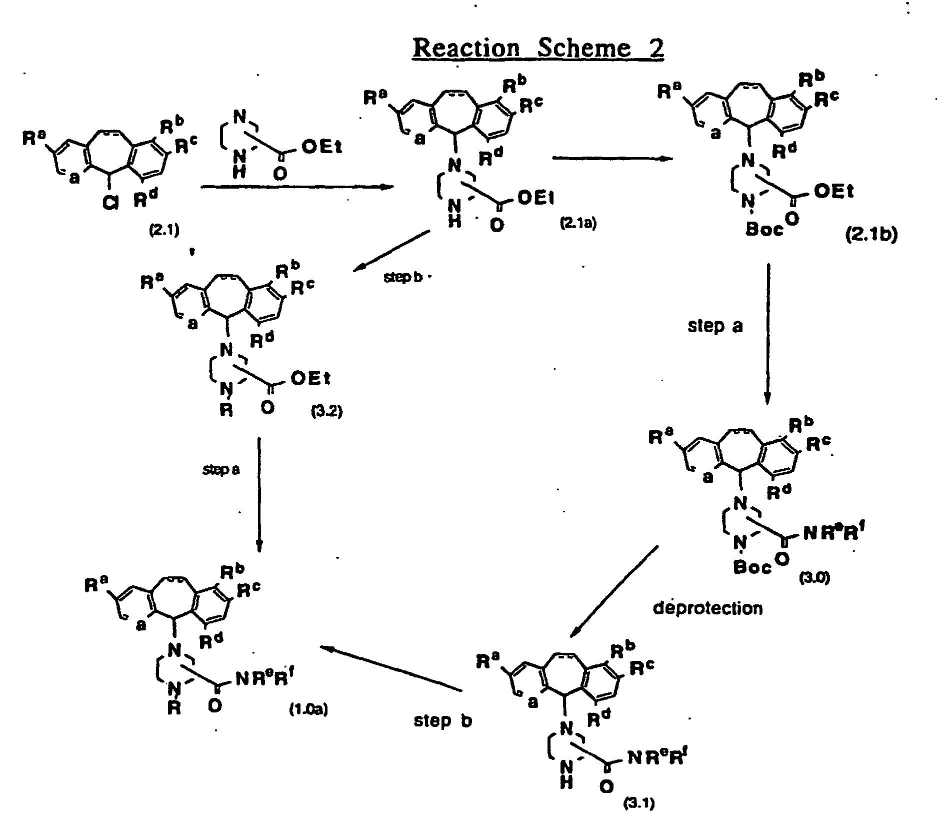

Compound (2.1) is reacted with ethylpiperazine-3-carboxylate to form Compound (2.1a). The reaction with the piperazine is carried out in a suitable solvent, e.g., DMF or THF in the presence of a base, e.g., triethylamine, at a temperature of from 0 to 80 °C for 1 - 24 hours, then added to water, extracted with a suitable solvent, e.g., ethyl acetate, and chromatographed on silica gel Compound (2.1a) can then be reacted with a suitable protecting group, such as the Boc group, utilizing di-tert.butyldicarbonate to form Compound (2.1b). Compound (2.1b) is subjected to step a (replacement of OEt with NR^{e}R^{f}), discussed below, to form Compound (3.0). Compound (3.0) can then be subjected to a standard deprotection reaction to remove the Boc group, thus forming Compound (3.1). Compound (3.1) can then optionally be subjected to reaction step (b)
(substitution of the nitrogen of the piperazine with R) if it is desired that R be other than H. Steps (a) and (b) may be carried out in any sequence. When Compound (2.1 a) is subjected to reaction step b first (i.e., forming Compound (3.2)), the use of a protecting group is generally not required.

Step (a) is carried out by reacting Compound (2.1b) or Compound (3,2) with LiOH in water with a suitable co-solvent, such as methanol, to convert the -C(O)OEt group to -C(O)OH. This carboxylic acid intermediate can then be reacted with a suitable NHR^{e}R^{f} amine compound (wherein R^{e} and R^{f} are as defined above) in the presence of HOBt and N-methylmorpholine and a coupling agent, such as DEC or DCC in a suitable solvent, e.g., DMF at a temperature of from 0 to 80 °C for 1 - 24 hours, then added to water, extracted with a suitable solvent, e.g., ethyl acetate, and chromatographed on silica gel.

Step (b) can be carried out in the following manner, depending on the R substituent:

For compounds of the formula (1.0a) wherein R and the nitrogen atom to which it is attached together comprise an amide, e.g., where R is -C(O)-alkyl, step (b) can be carried out by reacting the amine (2.1 a or 3.1) with a carboxylic acid of the formula R^{A}-C(O)-OH, wherein R^{A}-C(O)- is R, in the presence of a coupling agent such as DEC, CDI or DCC. The reaction is typically carried out in a suitable organic solvent such as DMF, THF or CH₂Cl₂ at a temperature of -10° to 100°C, preferably at 0° to 50°C, and most preferably at about room temperature. When the coupling agent is DCC or DEC, the reaction is preferably conducted in the presence of HOBT and N-methylmorpholine.

Alternatively, the amine (2.1a or 3.1) can be reacted with a compound of the formula R-L, wherein R is as defined above and L is a leaving group, such as Cl, Br, I , -O-C(O)-R^{B} wherein R^{B} is C₁-C₆ alkyl or phenyl, or a sulfonate group of the formula -OSO₂-R^{C}, wherein R^{C} is selected from C₁-C₆ alkyl, phenyl, CF₃, tolyl and p-bromophenyl, to form a compound of the formula (1.0a). The reaction is carried out in the presence of a base, preferably a tertiary amine base, such as Et₃N, DMAP, pyridine or Hünigs base.

For compounds of Formula 1.0a wherein: R is -C(O)-CH₂-R^{D}, wherein R^{D} is a piperidine of the formula: 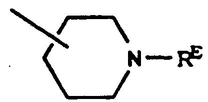 and R^{E} represents -C(O)NH₂ (i.e., a carboxamide), step (b) can be carried out by reacting the amine (2.1a or 3.1) with a protected piperidine, i.e., C(O)CH₂-piperidine-N-Boc in the presence of a coupling agent such as DEC, CDI or DCC. The reaction is typically carried out in a suitable organic solvent such as DMF, THF or CH₂Cl₂ at a temperature of -10° to 100°C, preferably at 0° to 50°C, and most preferably at about room temperature. When the coupling agent is DCC or DEC, the reaction is preferably conducted in the presence of HOBT and N-methylmorpholine. The above described Boc-piperidine compound is then deprotected with a suitable acid, such as trifluoroactic acid, to provide a piperidine wherein the nitrogen is unsubstituted. The above described unsubstituted piperidine is reacted with an excess of urea in water. This reaction can be run with about 4 to about 10 equivalents of urea relative to the unsubstitued piperidine starting reactant. Generally, about 10 equivalents of urea can be used. The reaction is run for about 3 to about 68 hours. Generally, the reaction can be run for about 60 to 70 hours. The reaction is usually run at the reflux temperature of the reaction mixture. This can range from about 98 to about 100°C. The amount of the unsubstitued piperidine starting reactant relative to water may typically vary from about 0.025g/ml to about 0.6g/ml, and can generally be about 0.1 g/ml.

For preparing compounds of the formula (1.0a) wherein R is -C(O)-NH-R^{G}, R^{G} being an alkyl, cycloalkyl, or heterocycloalkyl group, step (b) is carried out by reacting a compound of the formula (2.1a or 3.1) with an isocyanate of the formula RG-N=C=O, in a suitable solvent such as DMF, THF or CH₂Cl₂ using methods well known in the art.

Alternatively, the amine (2.1a or 3.1) is reacted with phosgene to form a chloroformate intermediate of the formula (5.0), as shown in Reaction Scheme 4. The chloroformate (5.0) is generally not isolated and is reacted with an amine of the formula R^{G}-NH₂, wherein R^{G} is as defined above, to form a compound of the formula (1.0a), wherein R is -C(O)-NH-R^{G}.

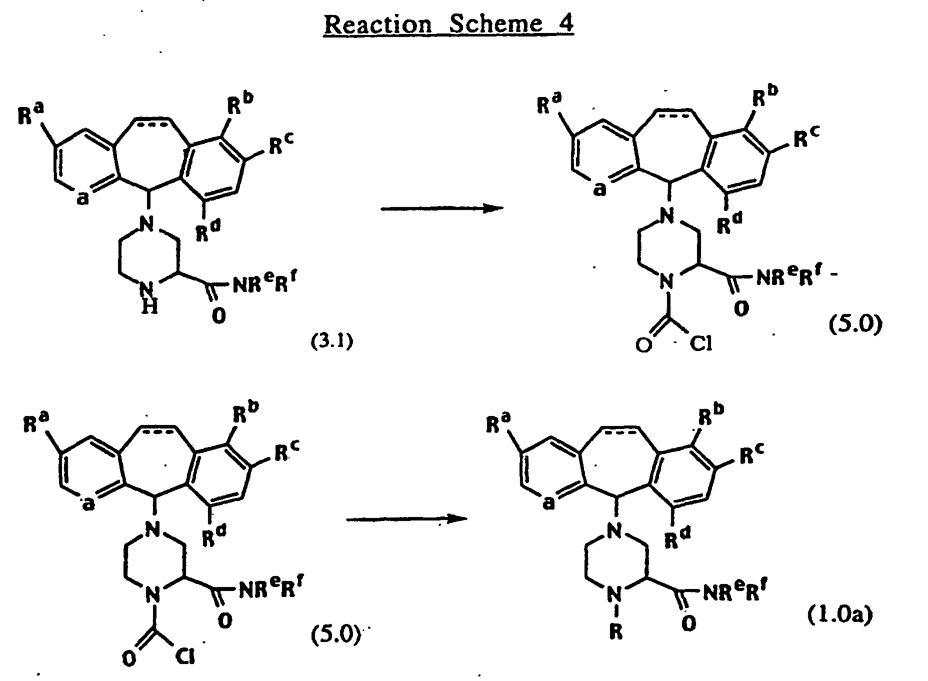

When R is S(O)₂R¹, step b can be carried out by dissolving Compound (3.1) in an appropriate solvent such as DMF of THF. A base is added such as triethylamine, and the appropriate alkylsulfonylchloride (R¹-S(O)₂Cl), prepared by methods known in the art, is added to the reaction mixture at 0°C to ambient temperature with stirring. After 1-24 hours, the reaction mixture is added to water and the product extracted with a suitable solvent such as ethylacetate. The crude reaction product can then be chromatographed on a silica gel column.

When R is S(O)₂NR¹R², step b can be carried out by dissolving Compound 3.1 in an appropriate solvent such as DMF of THF. A base is added such as triethylamine, and the appropriate alkylaminosulfonyl chloride (R¹R₂N-S(O)₂Cl), prepared by methods known in the art, is added to the reaction mixture at 0°C to ambient temperature with stirring. After 1-24 hours, the reaction mixture is added to water and the product extracted with a suitable solvent such as ethylacetate. The crude reaction product can then be chromatographed on a silica gel column.

Alternatively, compounds of the formula (1.0a) can be generally prepared as shown in Reaction Scheme 5.

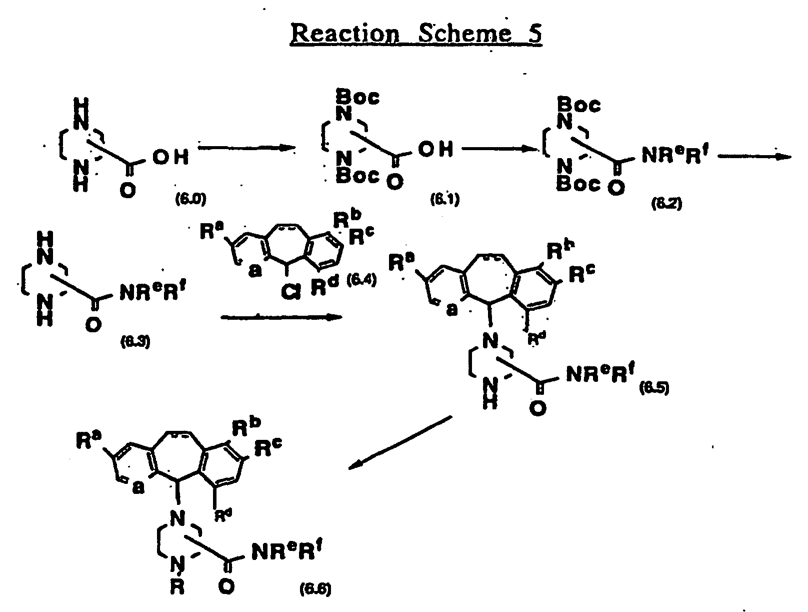

Compound (6.0) is reacted with di-tert-butyl-dicarbonate to form Compound (6.1). Compound (6.1) is reacted with NHR^{e}R^{f} in the presence of DEC and HOBt to form Compound (6.2). Compound (6.2) is treated with TFA to form Compound (6.3). Compound (6.3) is reacted with the tricyclic compound (6.4) to form Compound (6.5). Compound (6.5) is subjected to the treatment(s) described above for step (b) to obtain Compound (6.6).

The following examples illustrate methods by which compounds of formula (1.0) may be made.

Examples marked with an asterisk (*) are not within the scope of the invention, and are provided by way of illustrating analogous procedures by which compounds of the present invention may be made.

Compounds of formula (1.0a) in the R-enantiomeric form may be prepared as shown below in reaction schemes 6 and 7.

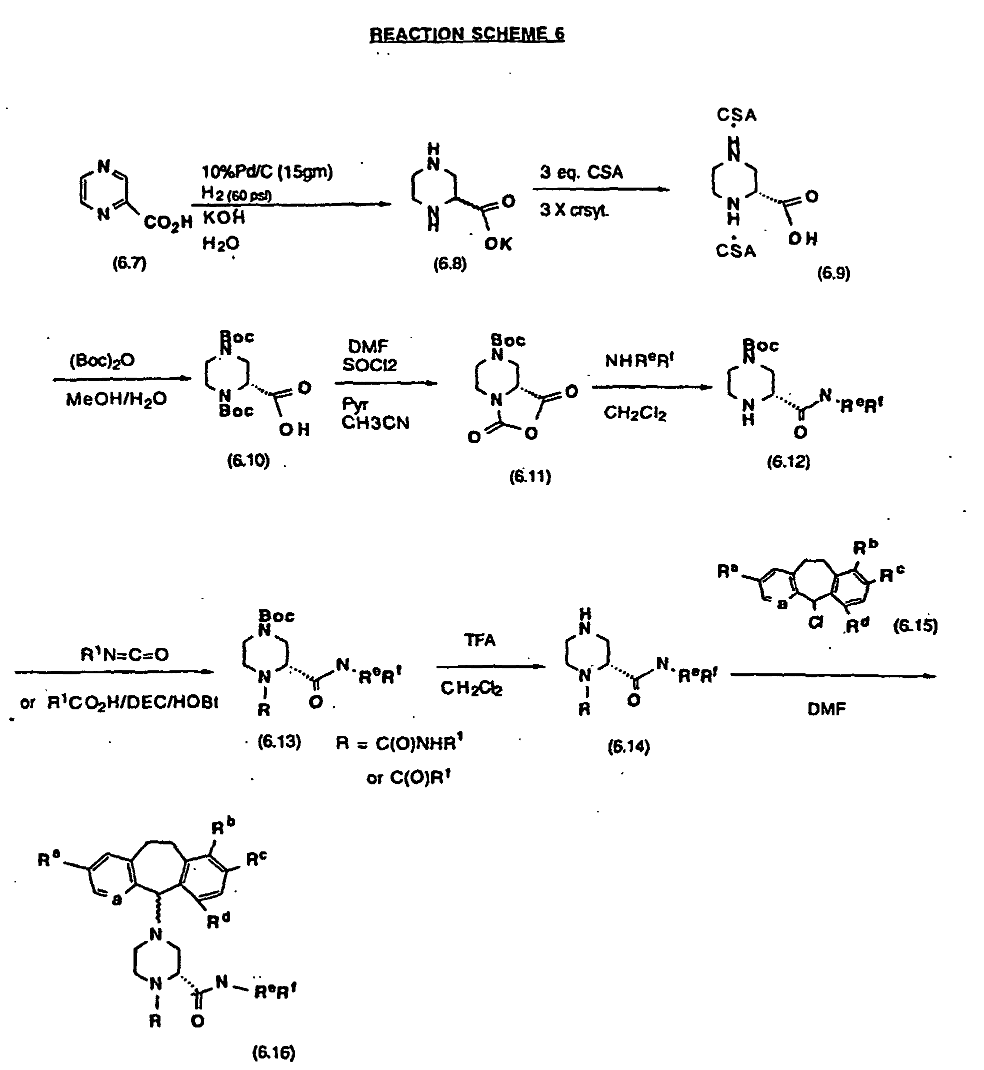

Compound (6.7) is hydrogenated under pressure, e.g., at a pressure of 30 to 100 psi, preferably 50 psi, in the presence of a Pd/C catalyst, e.g., 10% Pd/C. The hydrogenation is carried out at temperatures of 20°C to 30°C in ethanol. The hyrdrogenated product is subsequently reacted with KOH in water at ambient temperature to yield Compound (6.8). Compound (6.8) is reacted with 3 equivalents of R(-)camphor sulfonic acid ("CSA") at ambient temperature and recrystalized several times in water to yield a dicamphor sulfonic salt, Compound (6.9). Compound (6.9) is reacted with (Boc)₂O (di-tert-butyl dicarbonate) in a methanol/H₂O solution at ambient temperature to yield Compound (6.10). Compound (6.10) is reacted with sulfonyl chloride in DMF at 0°C and subsequently reacted with acetonitrile in pyridine at 0°C and allowed to warm to ambient temperature to yield Compound (6.11). Compound (6.11) is reacted with NHR^{e}R^{f} in methylene chloride at ambient temperature to form Compound (6.12). Compound (6.12) is reacted with either an isocyanate, R¹-N=C=O, or a carboxylic acid, R¹CO₂H, wherein R¹ is as defined above in formula (1.0), to form Compound (6.13), where R= -C(O)NHR¹ or C(O)R¹. The reaction with R¹-N=C=O or R¹CO₂H is carried out in either DMF or dichloromethane at ambient temperature. When R¹CO₂H is used, the reaction is preferably carried out in the presence of a coupling agent, e.g., DEC. The reactions converting Compound (6.11) to Compound (6.12) and converting Compound (6.12) to Compound (6.13) may be carried out as a one pot synthesis. Compound (6.13) is deprotected with TFA in methylene chloride at ambient temperature to form Compound (6.14). Compound (6.14) is reacted with Compound (6.15) in DMF at ambient temperature to form Compound (6.16).

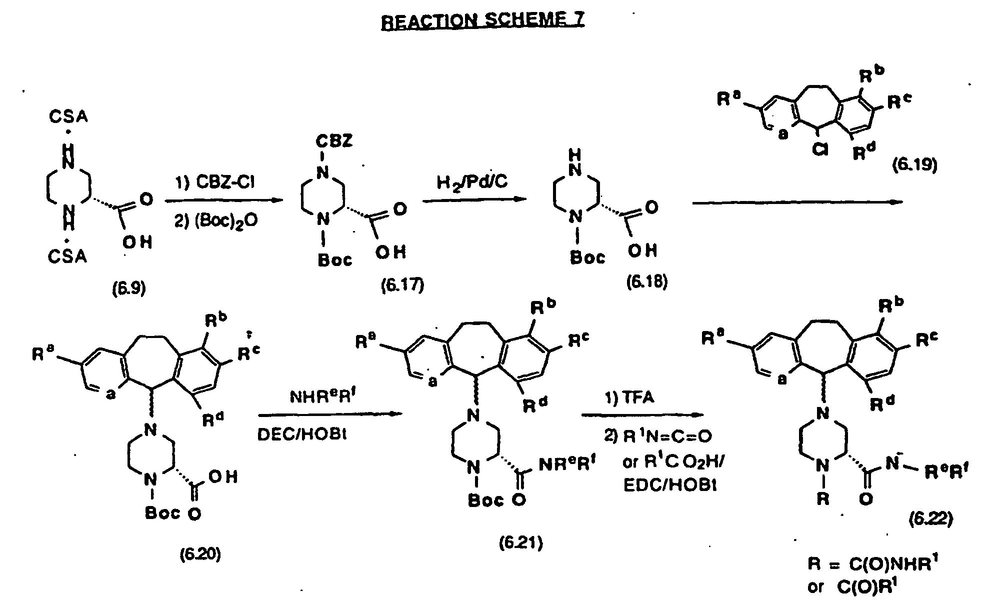

Compound (6.9), prepared as shown above in Scheme 6, is reacted with benzyl chloroformate (CBZ-Cl) in 50% dioxane water at ambient temperature to form a benzyloxycarbonyl compound, which is subsequently reacted with (Boc)₂O to form Compound (6.17). Compound (6.17) is hydrogenated under pressure, e.g., at a pressure of 30 to 100 psi, preferably 50 psi, in the presence of a Pd/C catalyst at ambient temperature to form Compound (6.18). Compound (6.18) is reacted with Compound (6.19) in DMF at ambient temperature to form Compound (6.20). Compound (6.20) is reacted with NHR^{e}R^{f} with DEC/HOBt in DMF at ambient temperature to form Compound (6.21). Compound (6.21) is deprotected with TFA at ambient temperature and then reacted with either an isocyanate, R¹-N=C=O, or a carboxylic acid, R¹CO₂H, where R¹ is as defined above in formula (1.0), to form Compound (6.22), where R is -C(O)NHR¹ or -C(O)R¹. The reaction with R¹-N=C=O or R¹CO₂H is carried out in either DMF or dichloromethane at ambient temperature. When R¹CO₂H is used, the reaction is preferably carried out in the presence of a coupling agent, e.g., DEC. The reactions converting Compound (6.20) to Compound (6.21) and converting Compound (6.21) to Compound (6.22) may be carried out as a one pot synthesis.

Compound (1.0b), wherein R is H, can be prepared according to the following reaction scheme:

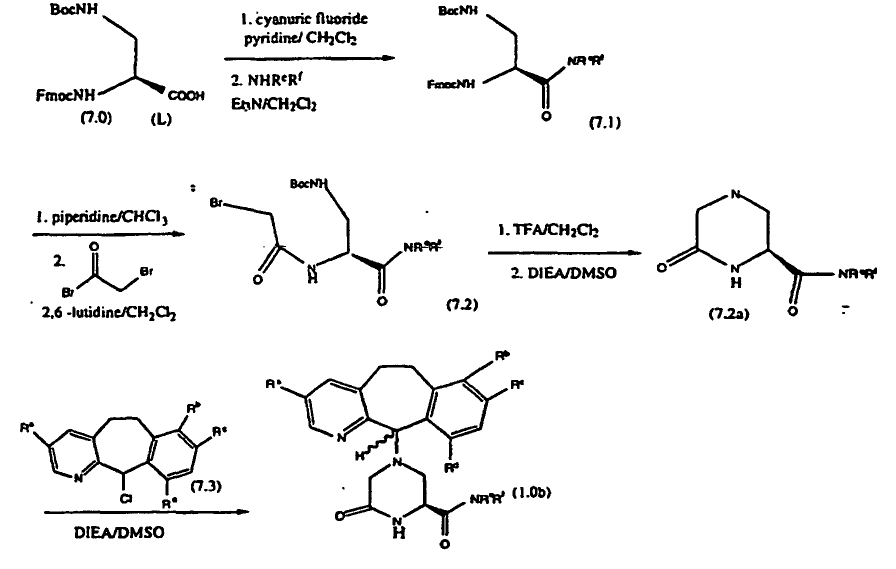

Compound (7.0) is treated with cyanuric fluoride and pyridine in CH₂Cl₂. The resulting acid fluoride is subsequently reacted with NHR^{e}R^{f} in the presence of triethylamine in CH₂Cl₂ to obtain Compound (7.1). Compound (7.1) is reacted with piperidine in CHCl₃ to yield an amine which is subsequently reacted with bromoacetyl bromide and 2,6-lutidine in CH₂Cl₂ to form Compound (7.2). Compound (7.2) is treated with trifluoroacetic acid in CH₂Cl₂, followed by treatment with diisopropylethylamine in DMSO to form the keto-substituted piperazine compound, Compound (7.2a). Compound (7.2a) is then reacted with tricyclic compound (7.3) to give Compound (1.0b), wherein R is H.

Compound (1.0b), wherein R is not H, can be prepared according to the following reaction scheme:

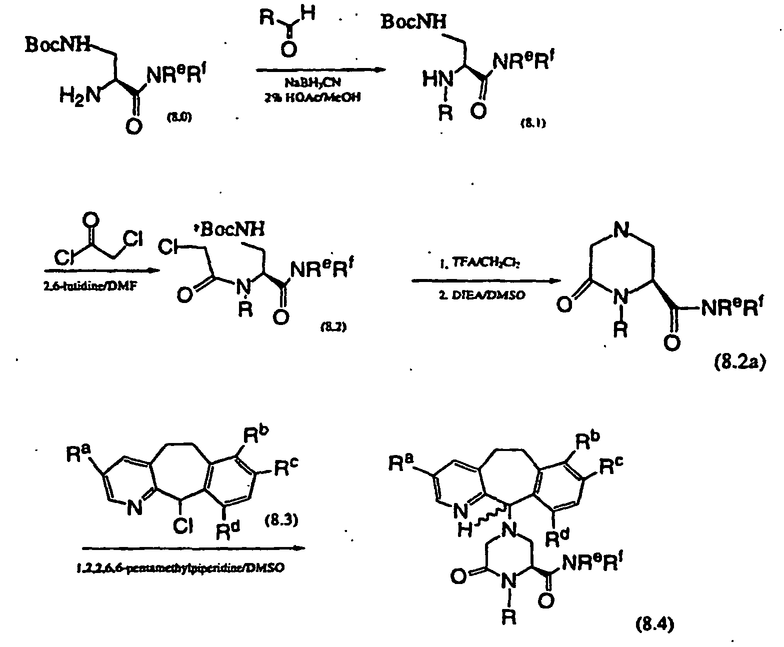

Compound (8.0) is reacted with R(O)H, e.g., valeraldehyde in 2% acetic acid/MeOH to obtain Compound (8.1). Compound (8.1) is reacted with chloroacetyl chloride in the presence of 2,6-lutidine in DMF to obtain Compound (8.2). Compound (8.2) is treated with trifluoroacetic acid in CH₂Cl₂, followed by treatment with diisopropylethylamine in DMSO to form the keto-substituted piperazine compound, Compound (8.2a). Compound (8.2a) is then reacted with tricyclic compound and reaction with the tricyclic compound (8.3) to obtain Compound (8.4).

Compounds of Formula 1.0 wherein substituent a is NO can be made from a tricyclic ketone by using procedures well known to those skilled in the art, as shown in the following scheme:

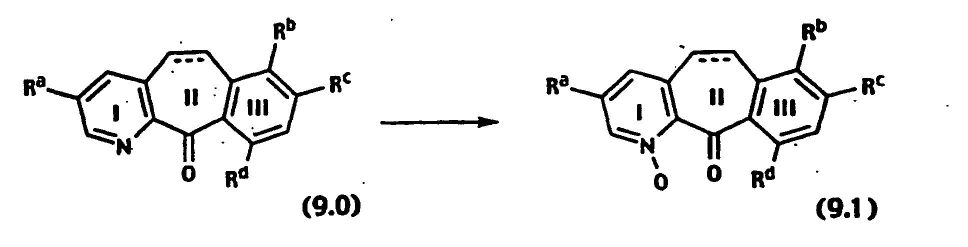

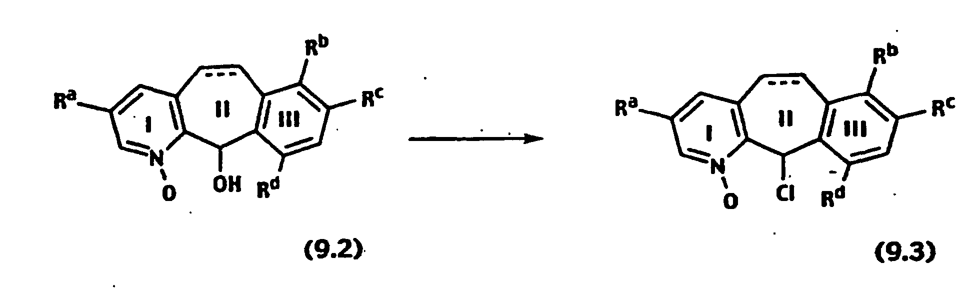

For example, the ketone (9.0) can be reacted with m-chloroperoxybenzoic acid in a suitable organic solvent, e.g., dichloromethane (usually anhydrous) or methylene chloride, at a suitable temperature, to produce NO-substituted compound (9.1). Generally, the organic solvent solution of ketone (9.0) is cooled to about 0°C before the m-chloroperoxybenzoic acid is added. The reaction is then allowed to warm to room temperature during the reaction period. Compound (9.1) can be recovered by standard separation means. For example, the reaction mixture can be washed with an aqueous solution of a suitable base, e.g., saturated sodium bicarbonate or NaOH (e.g., 1N NaOH), and then dried over anhydrous magnesium sulfate. The solution containing the product can be concentrated in vacuo. The product can be purified by standard means, e.g., by chromatography using silica gel (e.g., flash column chromatography). Compound (9.1) is converted to the hydroxy compound (9.2) by conventional means, e.g., by reacting it with NaBH₄ in methanol. Compound (9.2) is converted to the corresponding chlorinated compound (9.3) by conventional means, e.g., by reacting it with SOCl₂. Compound (9.3) is then reacted with a suitably substituted piperazine as described above, to give the desired compound.

### EXAMPLE 1*

The following compound was prepared according to the procedure described below

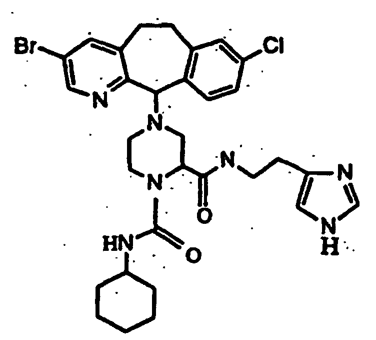

### Step 1A

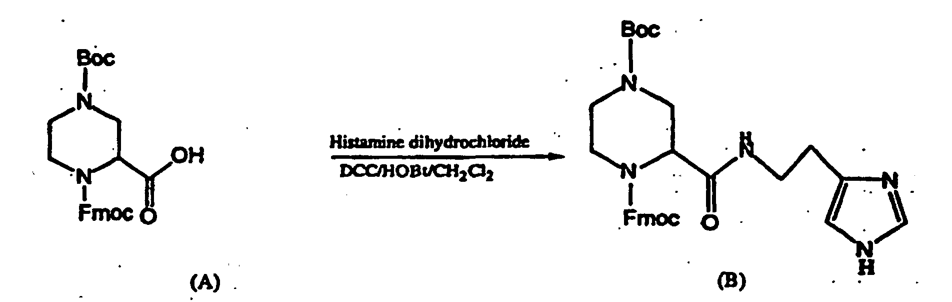

Compound A is prepared as follows:

Dissolve 5.25 g (25.85 mmol) of 2- piperazine carboxylic acid.2HCl in 160 ml of 1:1 dioxane/H2O, and adjust the pH to 11 with 50% NaOH (aq.). Slowly add (in portions) a solution of 7.21 g (29.28mmol) of BOC-ON in 40 ml of dioxane while maintaining the pH at 11 with 50% NaOH (aq.) during the addition. Stir at room temperature for 5 hours, then cool to 0 °C and adjust to pH/9.5 with 50% NaOH(aq.). Slowly add ( in portions) a solution of 7.34 g (28.37mmol) of FMOC-Cl in 40 mL of dioxane, maintaining a pH of 9.5 during the addition with 50% NaOH. Warm the mixture to room temperature and stir for 20 hours. Washed with Et₂O (3X150 ml). Dry the combined extracts over Na₂SO₄ and concentrate in vacuo to a volume of 150 ml. Chill at -20°C overnight, filter to collect the resulting solids, wash with hexane and dry the solids in vacuo to give 5.4 g of the product compound.

Preparation of compound **B**. To a stirred solution of the piperazine carboxylic acid derivative (**A**) (150 mg, 0.33 mmole) and histamine dihydrochloride (73 mg, 0.39 mmole) in CH₂Cl₂ (3 mL) was added DCC (81 mg, 0.39 mmole) and HOBt (53 mg, 0.39 mmole). The mixture was stirred overnight. Solvent was removed in vacuo and the residue was purified by column chromatography with 4-7% MeOH in CH₂Cl₂ to give 98 mg desired product in 55% yield. MS: m/z 546 (MH⁺).

### Step 1B

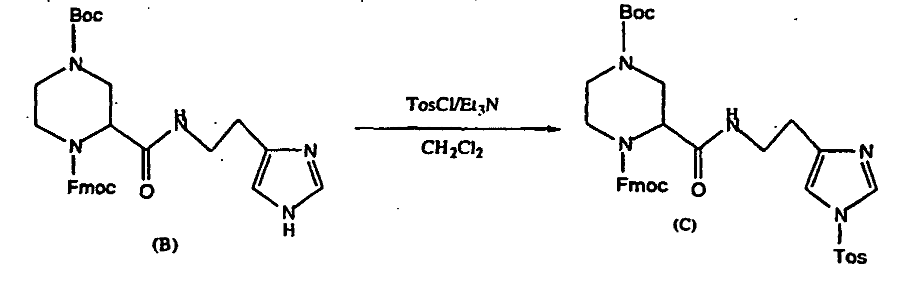

. Preparation of compound **C**. To a stirred solution of the amide (**B**) (54 mg, 0.098 mmole) in CH₂Cl₂ (2mL) was added triethylamine (14 uL, 0.098 mmole) and p-toluenesulfonyl chloride (19 mg, 0.098 mmole). The reaction was stirred overnight. The reaction was diluted with CH₂Cl₂ (20 mL) and washed with water (20 mL). Organic layer was dried (Na₂SO4) and solvent removed in vacuo to give 50 mg desired product in 73% yield. MS: m/z 700 (MH⁺).

### Step 1C

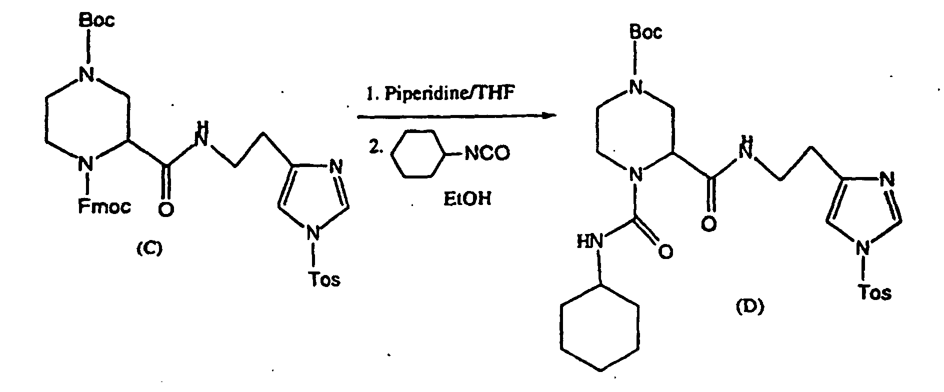

. Preparation of compound D. To a stirred solution of compound C (50 mg, 0.072 mmole) in THF (2mL) was added piperidine (1 mL). The reaction was stirred for 2 hours, and solvent was removed in vacuo. The residue was purified by column chromatography with 5% MeOH in CH₂Cl₂ to give 18 mg of the amine in 53% yield. MS: m/z 478 (MH⁺). This amine was dissolved in EtOH (2 mL) and cyclohexylisocyanate (54 uL, 0.37 mmole) was added. The reaction was stirred overnight. Solvent was removed in vacuo and the residue was purified by flash column chromatography with 4% MeOH in CH₂Cl₂ to give quantitative amount of desired product. MS: m/z 603 (MH⁺).

### Step 1D

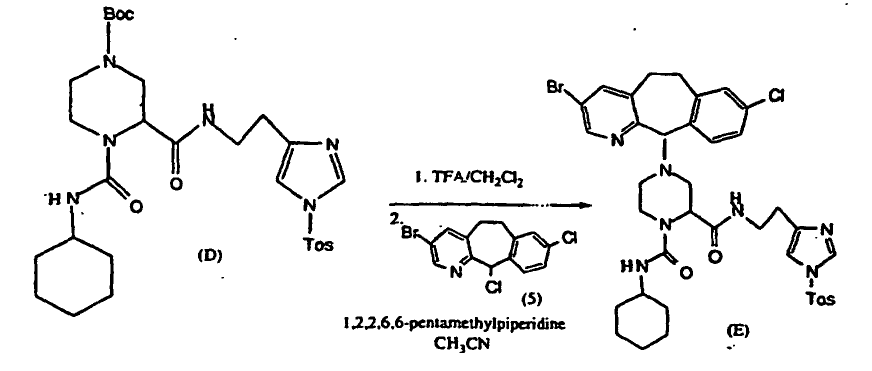

4. Preparation of **compound E**. To a stirred solution of the urea (**D**) (23 mg, 0.038 mmole) in CH₂Cl₂ (2mL) was added trifluoracetic acid (0.5 mL). The mixture was stirred for 20 min. and solvent was removed in vacuo. The residue was pumped on a high vacuum line for 2 hours and dissolved in CH₃CN (1 mL). To this solution was added 1,2,2,6,6-pentamethylpiperidine (34 uL, 0.19 mmole) and the tricyclic alkyl chloride (5) (13 mg, 0.038 mmol). The reaction was stirred overnight. A white precipitate formed. The mixture was dissolved in water (10 mL) and extracted with CH₂Cl₂ (15 mL x 2). The organic layer was dried (Na₂SO₄) and solvent was removed in vacuo. The residue was purified by flash column chromatography with 4% MeOH in CH₂Cl₂ to give 15.9 mg desired product in 52% yield. MS: m/z 810 (MH⁺).

### Step 1E

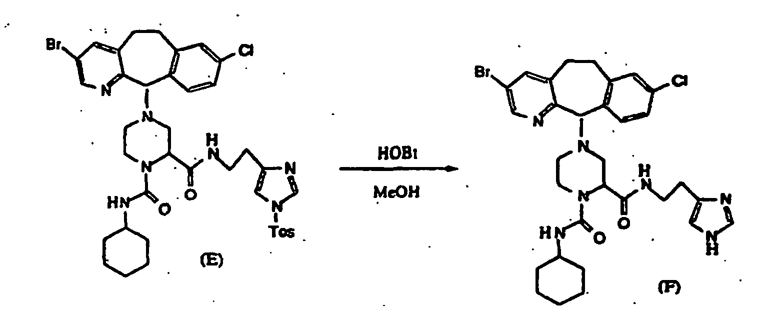

5. Preparation of **Compound F** To a stirred solution of **Compound E** (9.5 mg, 0.012 mmole) in MeOH (1 mL) was added HOBt (5 mg, 0.035 mmole). The reaction was stirred overnight. Solvent was removed in vacuo. The residue was purified by flash column chromatography with 5-7 % MeOH in CH₂Cl₂ to give 4.5 mg desired product in 58% yield. MS: m/z 656 (MH⁺).

### EXAMPLE 2*

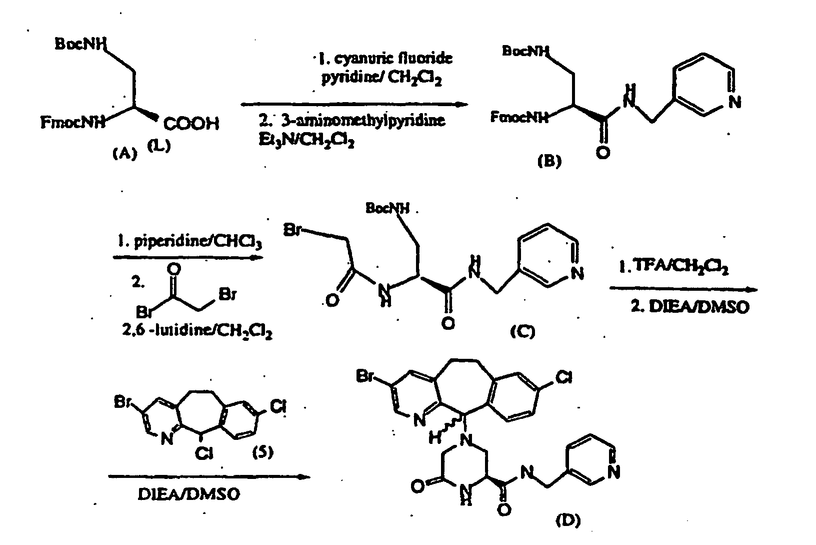

1. Preparation of compound **B**. To a stirred solution of the acid (**A**) (1.69g, 3.97 mmole) in CH₂Cl₂ (5 mL) was added pyridine (32 uL, 3.97 mmole) and cyanuric fluoride (669 uL, 7.93 mmole). The reaction was stirred overnight and water (10 mL) was added. The mixture was extracted with CH₂Cl₂ (15 mL x 2). The organic layer was dried (Na₂SO₄) and solvent removed in vacuo. The resulting acid fluoride was dissolved in CH₂Cl₂ (5 mL). To this solution was added triethylamine (732 uL, 5.25 mmole) and 3-amino-methylpyridine (428 uL, 4.2 mmole). The mixture was stirred for 3 hours and then diluted with CH₂Cl₂ (10 mL). the solution was washed with sat. NH₄Clsolution (5 mL). The organic layer was dried (Na₂SO₄) and solvent was removed in vacuo. The residue was purified by flash column chromatography with 2% MeOH in CH₂Cl₂ to give 1.4g desired product in 68% yield. MS: m/z 517 (MH⁺).
2. Preparation of compound **C**. To a stirred solution of compound(**B**) (581 mg, 1.13 mmole) in CHCl₃ (4 mL) was added piperidine (2mL). The mixture was stirred for 1 h and solvent was removed in vacuo. The residue was purified by flash column chromatography with 5-10% MeOH in CH₂Cl₂ to give 320 mg amine in 90% yield. The amine (68 mg, 0.231 mmole) was dissolved in CH₂Cl₂ (3 mL). To this solution was added 2,6-lutidine (46 uL, 0.39 mmole) and bromoacetyl bromide (30 uL, 0.35 mmole). The reaction was stirred for 15 min. The reaction was washed with 2% NH₄Cl solution. The aqueous phase was basified by NaHCO₃ and extracted with CH₂Cl₂. The organic layer was dried (Na₂SO₄) and evaporated to give 27 mg product in 28% yield. MS: m/z 415 (M⁺).
3. Preparation of **Compound D**. To a stirred solution of compound **C** (27 mg; 0.066 mmole) in CH₂Cl₂ (2mL) was added trifluoroacetic acid (1 mL). The reaction was stirred for 1 hour and solvent was removed in vacuo. The residue was pumped on a high vacuum for 3 hours then dissolved in DMSO (1 mL). To this solution was added diisopropylethylamine (69 uL, 0.39 mmole). The reaction was stirred for 2 hours, then tricyclic alkyl chloride 5 (27 mg, 0.079 mmole) and diisopropylethylamine (23 uL, 0.13 mmole) was added. The mixture was stirred overnight. Solvent was removed in vacuo. The residue was dissolved in EtOAc (10 mL) and washed with water (5 mL). The organic layer was dried (Na₂SO₄) and solvent removed in vacuo.. The residue was purified by flash column chromatography with 1 % MeOH in CH₂Cl₂ to give 4 mg product in 13% yield. MS: m/z 542 (MH⁺).

### Example 3*

The following compound was made, using the same procedure as Example 2, except that the R-isomer of Compound A of Example 2 was used:

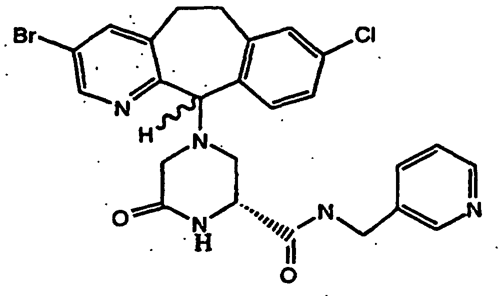

### EXAMPLE 4 *

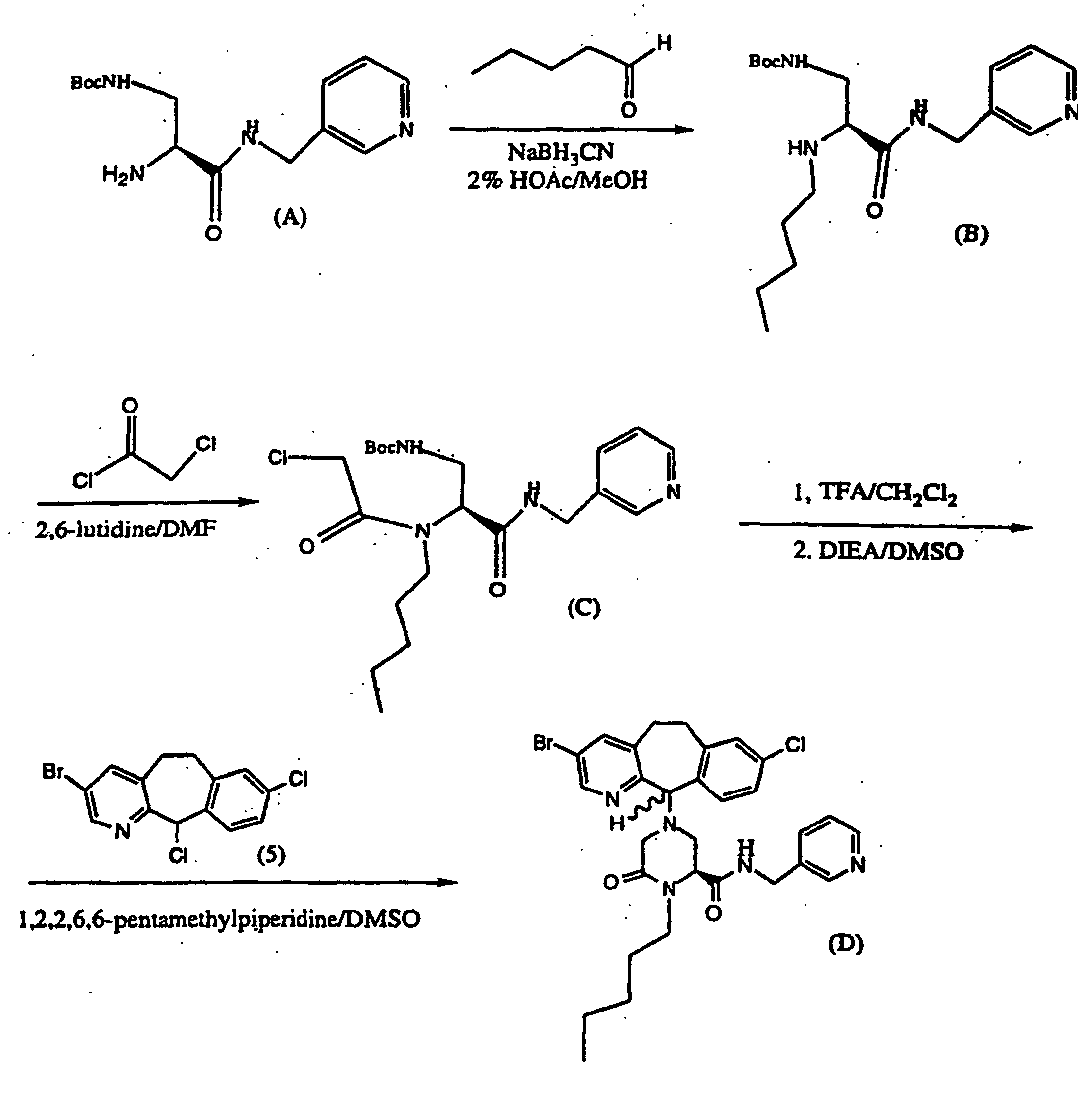

1. Preparation of **B**. To a stirred solution of amine **A** (276 mg, 0.939 mmole) in 2% HOAC/MeOH (2 mL) was added valeraldehyde (110 uL, 1 mmole) and NaBH₃CN. The mixture was stirred overnight. Solvent was removed in vacuo and 15% NaOH solution was added. The mixture was extracted with CH₂Cl₂ (10mL x 2). The organic layer was dried (Na₂SO₄) and solvent was removed in vacuo. The residue was purified by column chromatography with 2% MeOH in CH₂Cl₂ to give 220 mg product in 65% yield. MS: m/z 365 (MH⁺).
2. Preparation of **C**. To a stirred solution of the amine **B** (101 mg, 0.28 mmole) in DMF (2 mL) was added 2,6-lutidine (43 uL, 0.37 mmole) and chloroacetyl chloride (27 uL, 0.34 mmole). The reaction was stirred for 1 hour and solvent was removed in vacuo. The residue was dissolved in CH₂Cl₂ (10 mL) and washed with 0.5 N NaOH solution. Organic layer was dried (Na₂SO₄) and solvent was removed in vacuo to give 116 mg product in 94% yield. MS: m/z 441 (MH⁺).
3. Preparation of **Compound D**. To a stirred solution of compound **C** (116 mg, 0.26 mmole) in CH₂Cl₂ (3 mL) was added trifluoroacetic acid (1 mL). The reaction was stirred for 1 hour. Solvent was removed in vacuo. The residue was pumped on a high vacuum for 3 hours, then dissolved in DMSO (1mL). To this solution was added diisopropylethylamine (275 uL, 1.58 mmole). The reaction was stirred for 2 hours, then tricyclic alkyl chloride 5 (0.131 mmole) and 1,2,2,6,6-pentamethylpiperidine (94 uL, 0.52 mmole) was added. The mixture was stirred overnight. Solvent was removed in vacuo. The residue was dissolved in EtOAc (10 mL) and washed with water (5 mL). The organic layer was dried (Na₂SO₄) and solvent removed in vacuo. The residue was purified by flash column chromatography with 1 % MeOH in CH₂Cl₂ to give 5.4 mg product in 6.7% yield. MS: m/z 612 (MH⁺).

### EXAMPLE 5*

The following compound was prepared using the same procedures as in Example 4, except that the R isomer of Compound A in Example 4 was used:

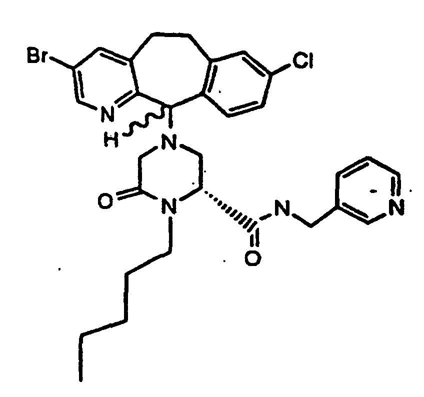

### EXAMPLE 6

### Procedure 1. Preparation of 3,10-DIBROMO-8-CHLORO-6,11-DIHYDRO-5H-BENZO[5,6]CYCLOHEPTA [1,2-b]PYRIDIN-11-OL

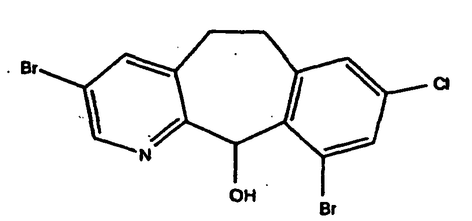

3,10-DIBROMO-8-CHLORO-5,6-DIHYDRO-11H-BENZO [5,6]CYCLOHEPTA[1,2-b]PYRIDIN-11-ONE (2gm, 5mm) was dissolved in 20 ml of methanol. Sodium borohydride (0.6 gm) was added and the reaction mixture stirred at ambient temperature. After 1 hour the reaction mixture was added to 20 ml of 1H hydrochloric acid and stirred for 5 minutes. 30 ml of 1N sodium hydroxide was added and the product extracted with methylenechloride three times. The methylenechloride layer was dried over magnesium sulfate, filtered, and evaporated to dryness under vacuo to obtain 1.98 gm of title product. FABMS (M/e+1)=402

### Procedure 2. Preparation of 3,10-DIBROMO-8,11-DICHLORO-6,11-DIHYDRO-5H-BENZO[5,6] CYCLOHEPTA[1,2-b]PYRIDINE

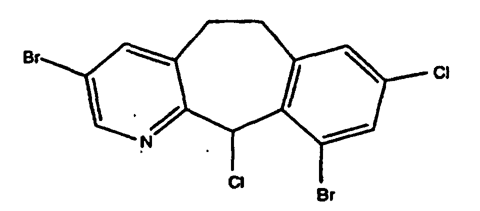

3,10-DIBROMO-8-CHLORO-6,11-DIHYDRO-5H-BENZO[5,6]CYCLOHEPTA[1,2-b]PYRIDIN-11-OL (1gm, 2.48 mm) was suspended in 20 ml of methylenechloride under a dry nitrogen atmosphere. Thionylchloride (1.63gm, 13.71mm) was added and the reaction mixture stirred for 2 hours. The crude reaction mixture was evaporated to dryness under vacuo to obtain 1.1 gm of product as the hydrochloride salt. FABMS (M/e+1)=420

### Procedure 3. Preparation of ETHYL 1-(3,10-DIBROMO-8-CHLORO-6,11-DIHYDRO-5H-BENZO[5,6] CYCLOHEPTA[1,2-b]PYRIDIN-11-YL)-3-PIPERAZINE-CARBOXYLATE

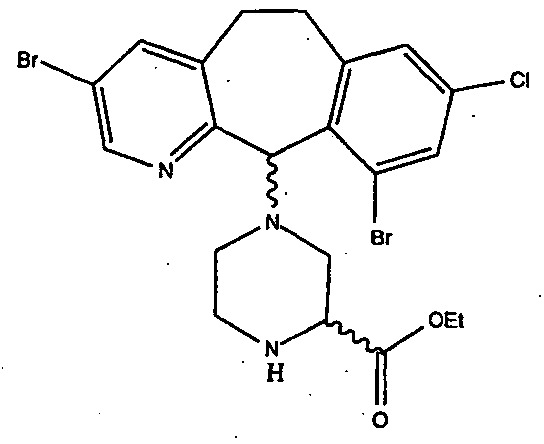

3,10-DIBROMO-8,11-DICHLORO-6,11-DIHYDRO-5H-BENZO[5,6]CYCLOHEPTA[1,2-b]PYRIDINE (1.05gm, 2.48mm) was dissolved in 20ml of dry N,N-dimethylformamide under a dry nitrogen atmosphere. Ethylpiperazine-3-carboxylate (1.177gm, 7.44mm) and diisopropylethylamine (1.28gm, 9.92mm) were added and the reaction mixture stirred at ambient temperature for 18 hours. The reaction mixture was added to 100 ml of brine and extracted with 3X150 ml of methylenechloride. The solvent was removed under vacuo to obtain a solid which was chromatographed on 100 g of flash silica gel using 50% ethylacetate/hexanes to obatin 0.895 g of the title product. FABMS (M/e+1)=542

### Procedure 4. Preparation of ETHYL 4-(3,10-DIBROMO-8-CHLORO-6,11-DIHYDRO-5H-BENZO[5,6] CYCLOHEPTA[1,2-b)PYRIDIN-11-YL)-1-(1-OXOPENTYL) 2-PIPERAZINECARBOXYLATE

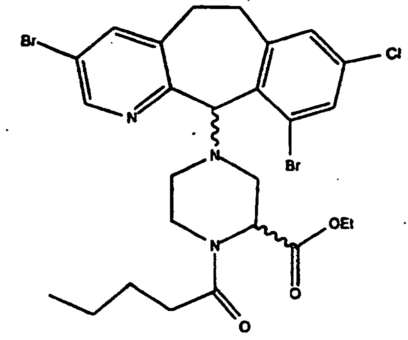

ETHYL 1-(3,10-DIBROMO-8-CHLORO-6,11-DIHYDRO-5H-BENZO[5,6]CYCLOHEPTA[1,2-b]PYRIDIN-11-YL)-3-PIPERAZINECARBOXYLATE (0.46gm, 0.85mm) was dissolved in 10 ml of dry N,N-dimethylformamide. Valeric acid (0.153gm, 1.5mm), 1-(3-dimethylaminopropyl)-3-ethylcarbodiimide hydrochloride (DEC) (0.288gm, 1.5mm), 1-hydroxybenzotriazole (HOBt) (0.203gm, 1.5mm), and N-methylmorpholine ( 0.5gm, 5mm) were added and the reaction mixture stirred at ambient temperature. After 48 hours, the reaction mixture was added to brine and extracted with 3X150ml of ethylacetate. The combined ethylacetate washes were combined and the solvent evaporated under vacuo to give a gum. The gum was chromatographed on 75gm of flash silica gel using 25% ethylacetate/hexanes as the eluent to obtain 0.45 gm of title product. FABMS (M/e+1)=626

### Procedure 5. Preparation of 4-(3,10-DIBROMO-8-CHLORO-6,11-DIHYDRO-5H-BENZO[5,6] CYCLOHEPTA[1,2-b]PYRIDIN-11-YL)-1-(1-OXOPENTYL)-2-PIPERAZINECARBOXYLIC ACID

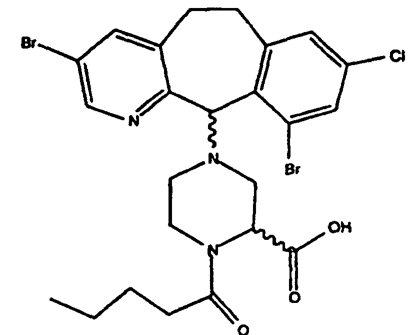

ETHYL 4-(3,10-DIBROMO-8-CHLORO-6,11-DIHYDRO-5H-BENZO[5,6]CYCLOHEPTA[1,2-b]PYRIDIN-11-YL)-1-(1-OXOPENTYL)-2-PIPERAZINECARBOXYLATE (0.4gm, 0.64mm) was dissolved in 10 mol of ethanol. 5 ml of 1M lithium hydroxide was added and the reaction mixture stirred for 24 hours. The pH was adjusted to 4.5 with 1.0% citric acid, diluted with 50 ml of water and extracted with 2X100ml of methylenechloride. The methylenechloride extracts were dried over magnesium sulfate, filtered, and evaporated to dryness to obtain 0.385 gm of title product. FABMS (M/e+1)=598

### Procedure 6. Preparation of 4-(3,10-DIBROMO-8-CHLORO-6,11-DIHYDRO-5H-BENZO[5,6] CYCLOHEPTA[1,2-b]PYRIDIN-11-YL)-N-(3-PYRIDINYLMETHYL)-1-(1-OXOPENTYL)-2-PIPERAZINECARBOXAMIDE

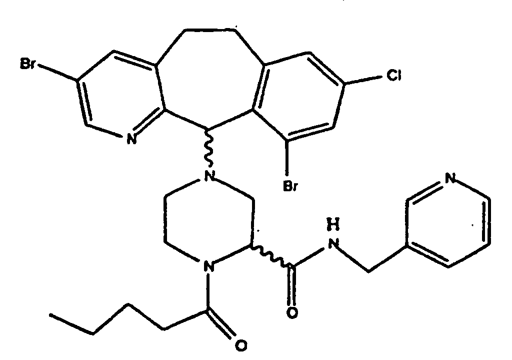

4-(3,10-DIBROMO-8-CHLORO-6,11-DIHYDRO-5H-BENZO[5,6]CYCLOHEPTA[1,2-b]PYRIDIN-11-YL)-1-(1-OXOPENTYL)-2-PIPERAZINECARBOXYLIC ACID (0.353gm, 0.59mm) was dissolved in dry N,N-dimethylformamide. 3-Aminomethylpyridine (0.127gm, 1.18mm), 1-(3-dimethylaminopropyl)-3-ethylcarbodiimide hydrochloride (DEC) (0.266gm, 1.18mm), 1-hydroxybenzotriazole (HOBt) (0.16gm, 1.18mm), and N-methylmorpholine ( 0.59gm, 5.9mm) were added and the reaction mixture stirred at ambient temperature under a dry nitrogen atmosphere for 18 hours. The reaction mixture was added to brine and the product extracted with 3X159 ml of ethylacetate. The ethylacetate layers were dried over magnesium sulfate and evaporated under vacuo. The crude product was chromatographed on a 50gm silica column using 2.5% methanol-2M ammonia/methylenechloride and increased to 10% to obtain 0.387gm of title product. FABMS (M/e+1)=691

### Procedure 7. Preparation of N,N-di-tert.butoxycarbonyl-3-carboxypiperazine.

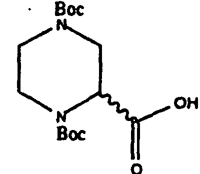

3-carboxypiperazine (10gm, 49.2mm) was dissolved in 200ml of 50% methanol/water and the pH adjusted to 9.5 with 50% sodium hydroxide. Di-tert.butyldicarbonate (21 gm) was added and the pH kept at 9.5 with 1N sodium hydroxide. The reaction was monitored by tlc and more di-tert.butyldicarbonate added if needed. The reaction mixture was acidified with conc. Hydrochloric acid to pH=7 and then with citric acid to pH 3.8 and extracted with methylenechloride to obtain 15.4 gm of solid product.

### Procedure 8. Preparation of N,N-di-tert.butoxycarbonyl-3-[4-pyrollidinoneaminopropionyl-piperazine

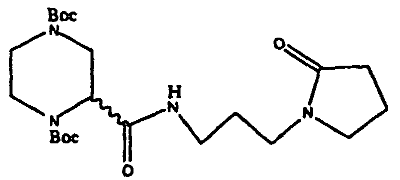

N,N-di-tert.butoxycarbonyl-3-carboxypiperazine (2gm, 6.2mm) was dissolved in 20 ml of N,N-dimethylformamide. 4-Pyrollidinoneaminopropane ( 12.4ml, 12.4mm), 1-(3-dimethylaminopropyl)-3-ethylcarbodiimide hydrochloride (DEC) (2.38gm, 12.4mm), 1-hydroxybenzotriazole (HOBt) (1.68gm, 12.4mm), and N-methylmorpholine ( 6.82ml, 62mm) were added and the reaction mixture stirred at ambient temperature under a dry nitrogen atmosphere for 18 hours. The reaction mixture was added to brine and the product extracted with 3X159 ml of ethylacetate. The ethylacetate layers were dried over magnesium sulfate and evaporated under vacuo. The crude product was chromatographed on a silica gel column using 5% methanol/methylenechloride as the eluent to obtain the title product which was treated with 30 ml of trifluoroacetic acid for 4 hr at ambient temperature. The trifluoroacetic acid was evaporated to obtain 6 gm of a light brown oil.

### Procedure 9. Preparation of 4-(3,10-DIBROMO-8-CHLORO-6,11-DIHYDRO-5H-BENZO[5,6] CYCLOHEPTA[1,2-b]PYRIDIN-11-YL)-N-[3-(2-OXO-1-PYRROLIDINYL)PROPYL]-2-PIPERAZINECARBOXAMIDE

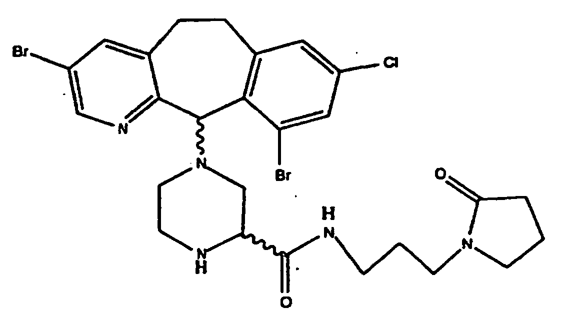

3-[4-pyrollidinone-aminopropiony]-piperazineditrifluoroacetic acid salt (6.2mm) is added to a solution of 3,10-DIBROMO-8,11-DICHLORO-6,11-DIHYDRO-5H-BENZO[5,6]CYCLOHEPTA[1,2-b]PYRIDINE (1gm, 2.48mm) in 30 ml of N,N-dimethylformamide and N-methylmorpholine (3.47ml) and stirred for 24 hours. The reaction mixture was added to 100 ml of brine and extracted with 3X150 ml of methylenechloride. The solvent was removed under vacuo to obtain a solid which was chromatographed on a flash silica gel using 2.5%-7.5% methanol/methylenechloride to obatin 0.4 g of the title product. FABMS (M/e+1)=641

### Procedure 10. Preparation of 4-(3,10-DIBROMO-8-CHLORO-6,11-DIHYDRO-5H-BENZO[5,6] CYCLOHEPTA[1,2-b]PYRIDIN-11-YL)-1-[(2-METHOXYETHOXY)ACETYL]-N-[3-(2-OXO-1-PYRROLIDINYL)PROPYL]-2-PIPERAZINECARBOXAMIDE

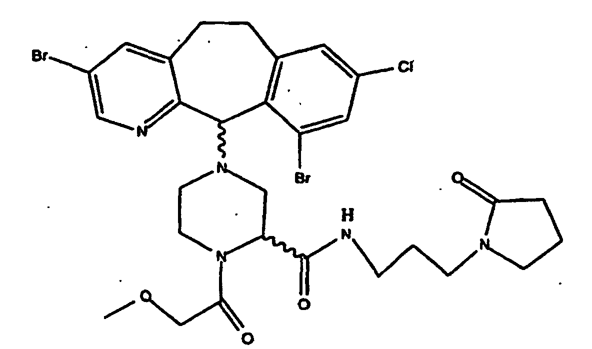

4-(3,10-DIBROMO-8-CHLORO-6,11-DIHYDRO-5H-BENZO[5,6]CYCLOHEPTA[1,2-b]PYRIDIN-11-YL)-N-[3-(2-OXO-1-PYRROLIDINYL)PROPYL]-2-PIPERAZINECARBOXAMIDE (0.064gm) was dissolved in 2ml of N,N-dimthylformamide. 1-(3-dimethylaminopropyl)-3-ethylcarbodiimide hydrochloride (DEC) (0.038gm), 1-hydroxybenzotriazole (HOBt) (0.027g), and N-methylmorpholine ( 0.11ml) were added and the reaction mixture stirred at ambient temperature under a dry nitrogen atmosphere for 18 hours. The reaction mixture was added to brine and the product extracted with ethylacetate. The ethylacetate layers were dried over magnesium sulfate and evaporated under vacuo. The crude product was chromatographed on a silica gel column using 5% methanol/methylenechloride as the eluent to obtain 0.059gm of the title product. FABMS (M/e+1)=713

### EXAMPLE 7

Using substantially the same reaction scheme of Example 1 or 6, and/or the reaction schemes described in the specification above, the following compounds were prepared:

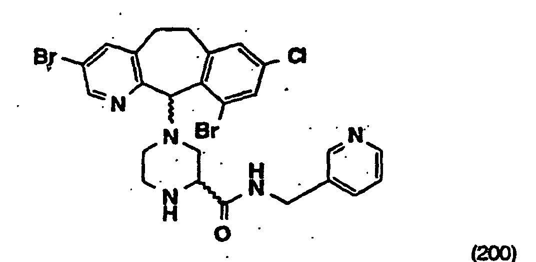

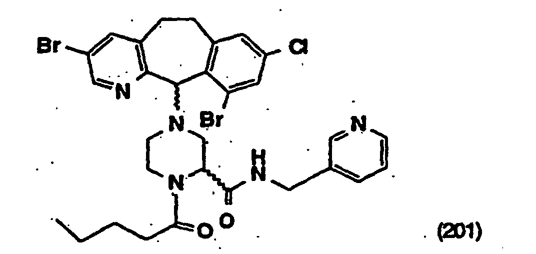

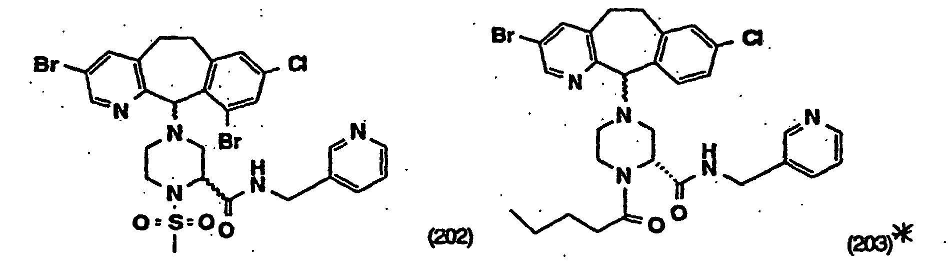

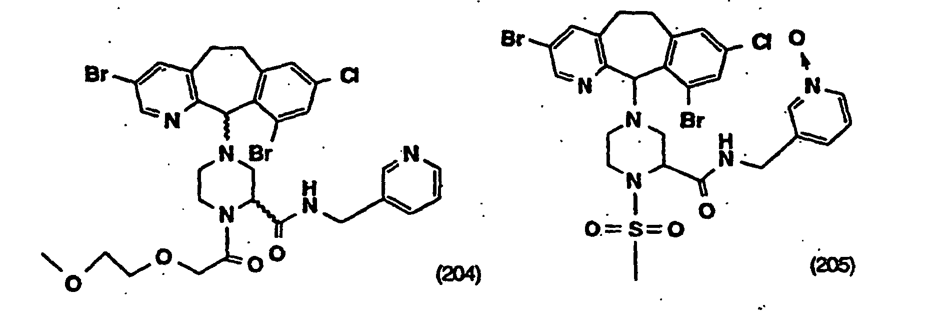

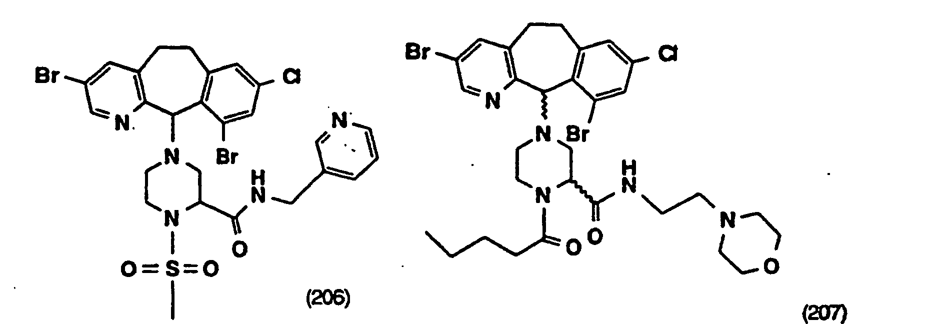

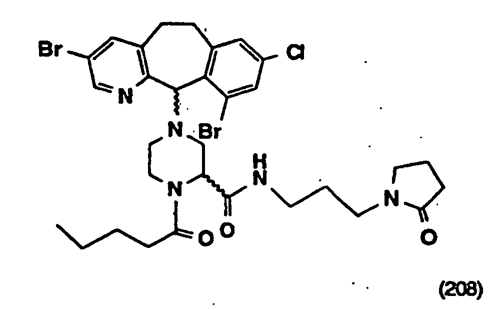

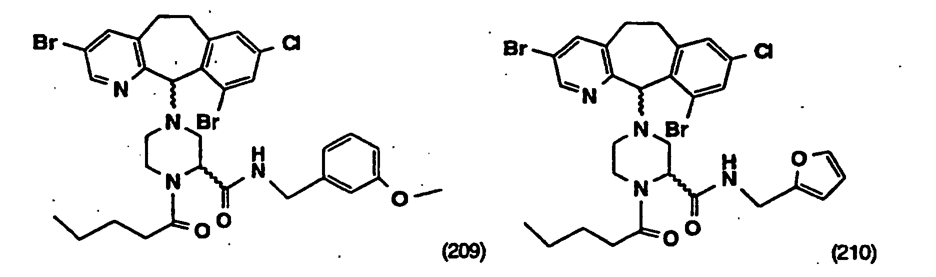

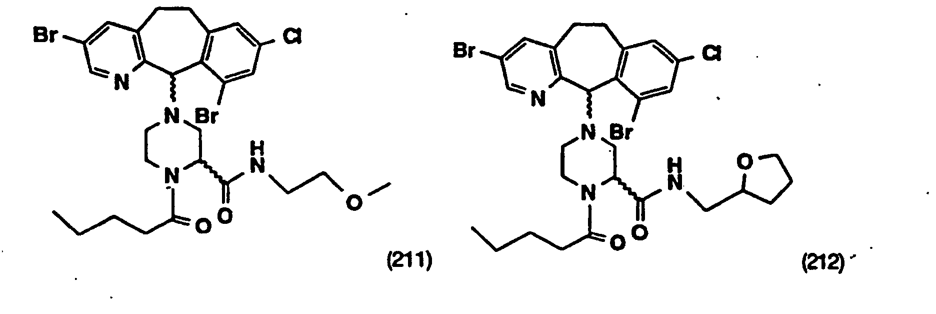

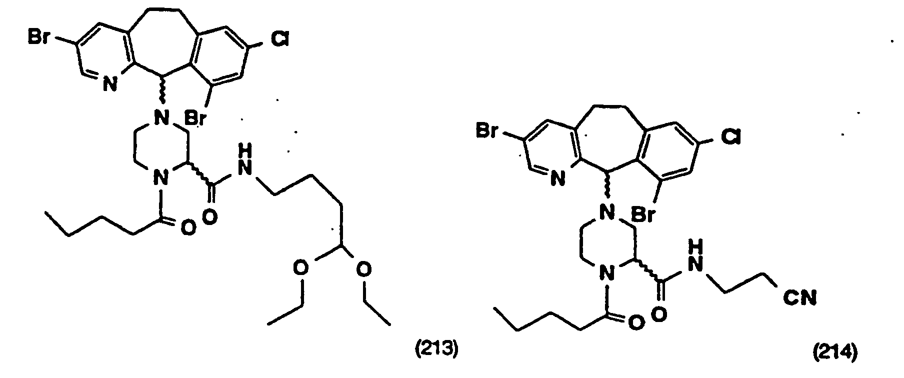

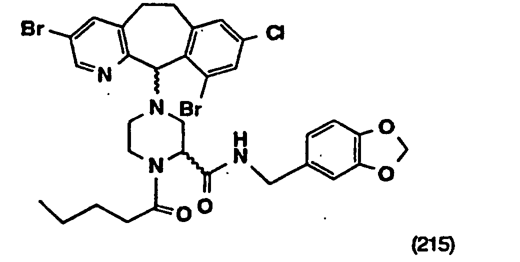

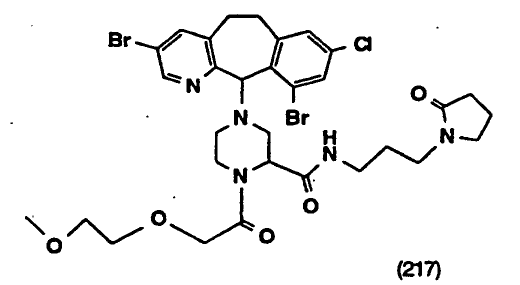

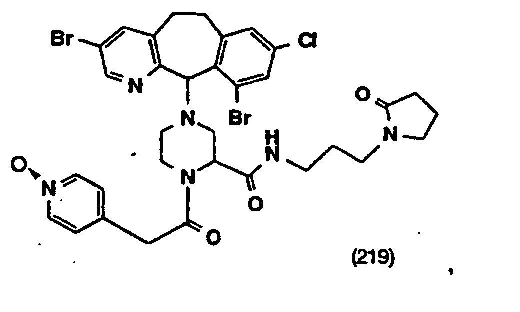

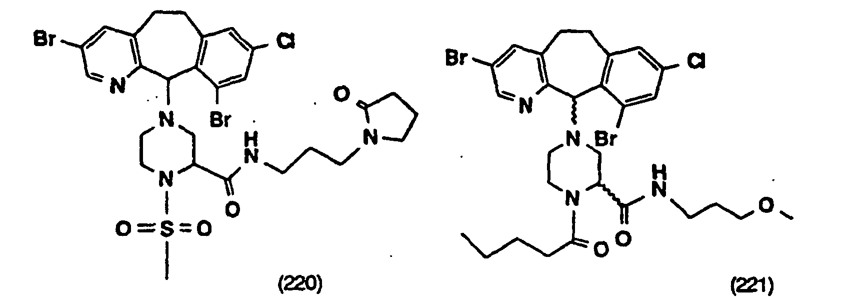

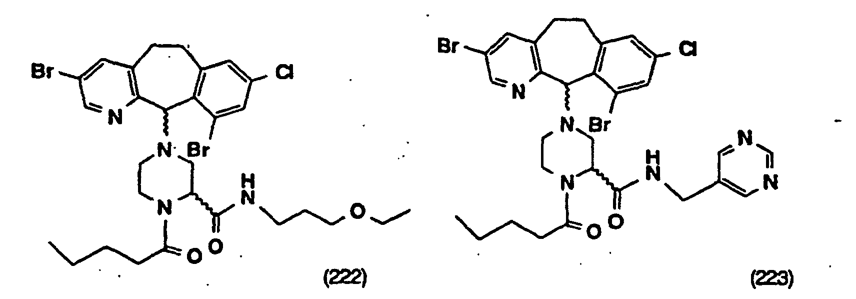

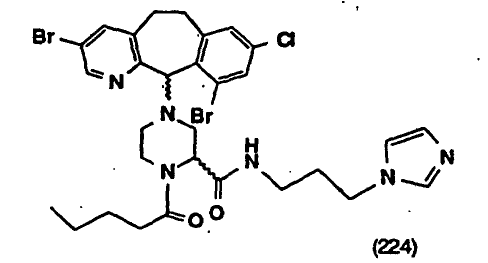

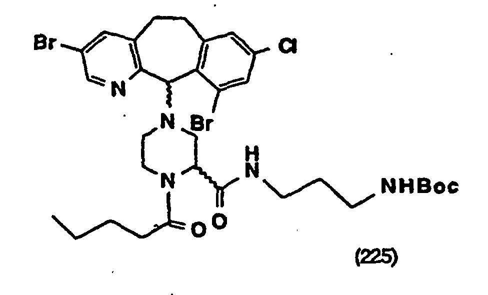

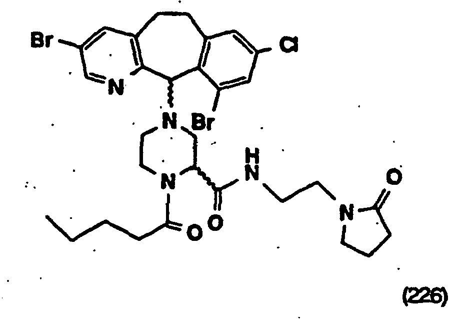

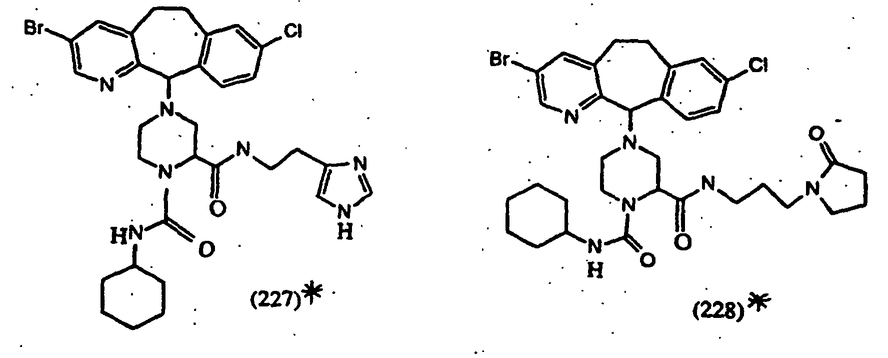

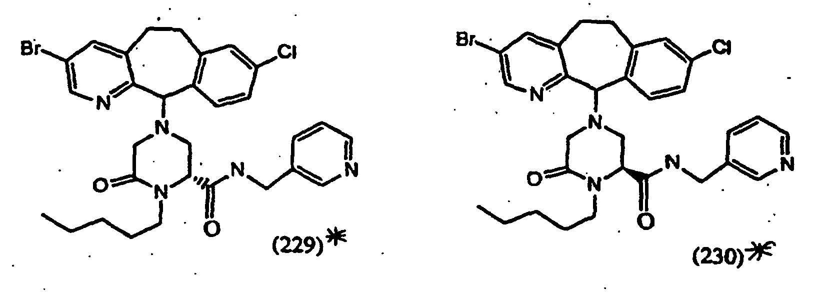

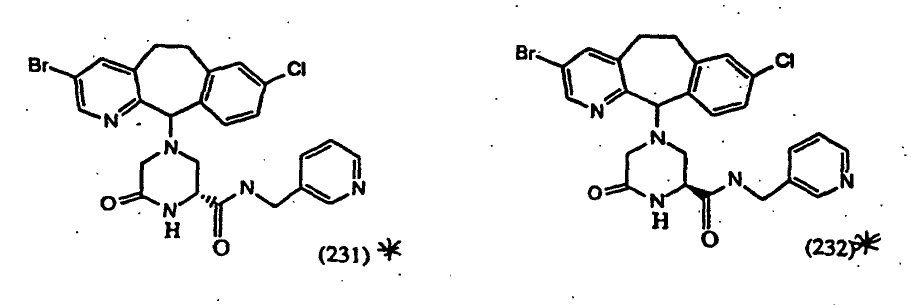

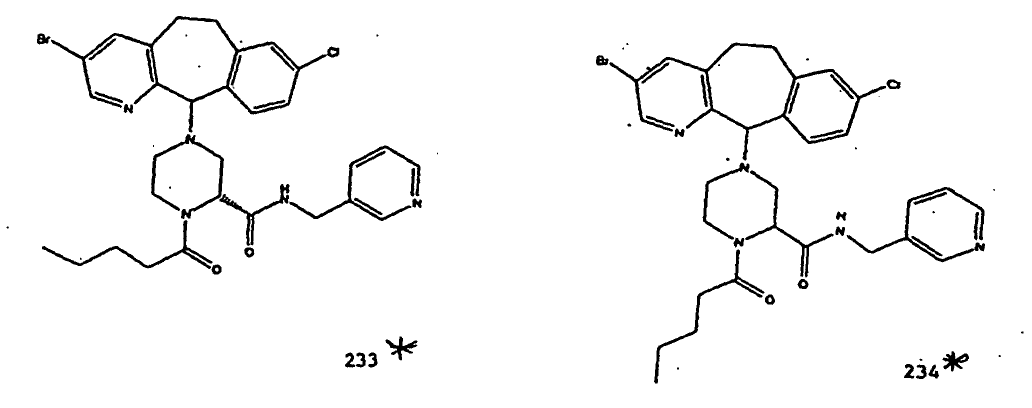

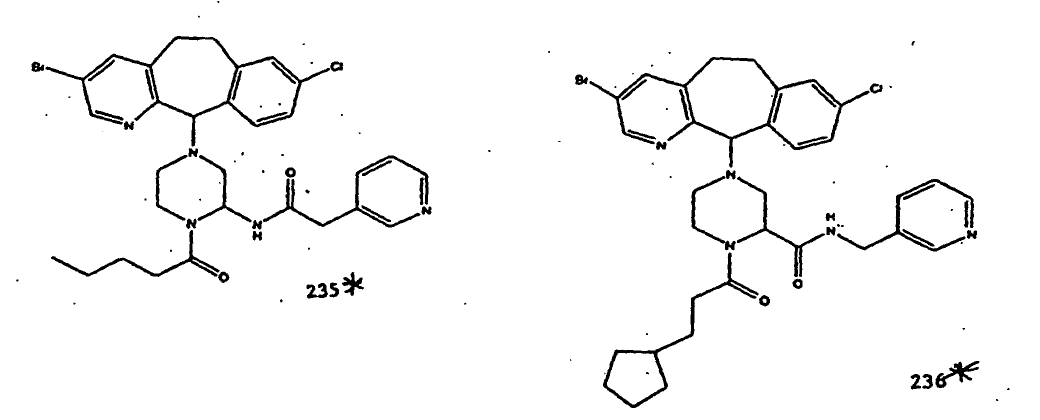

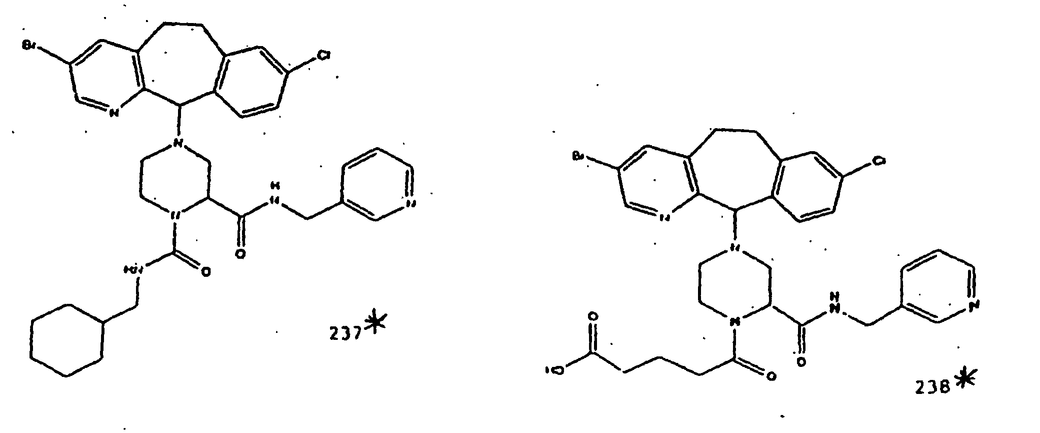

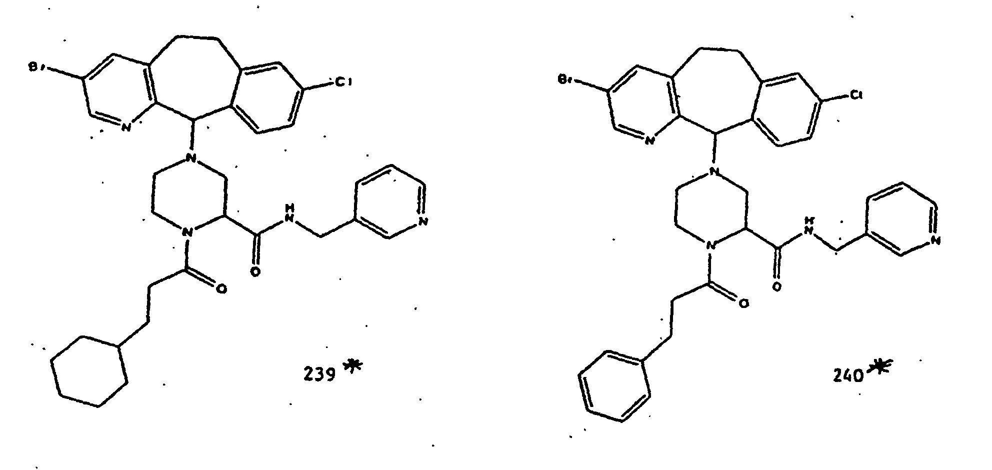

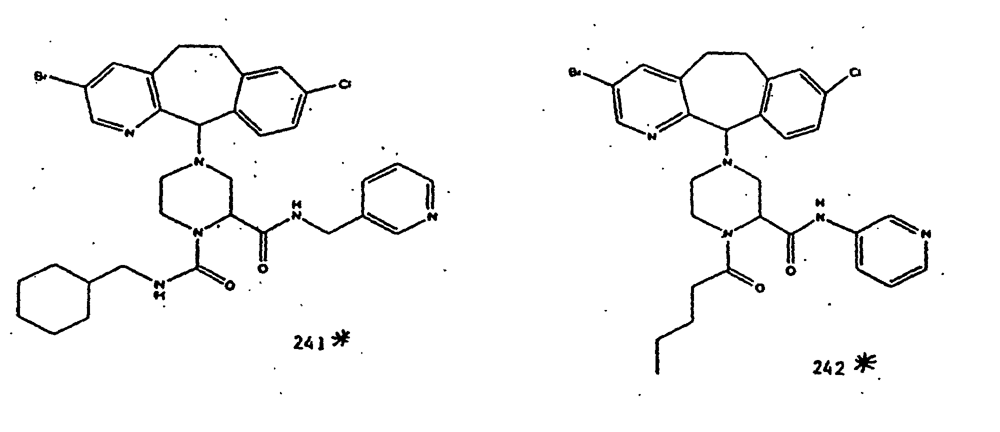

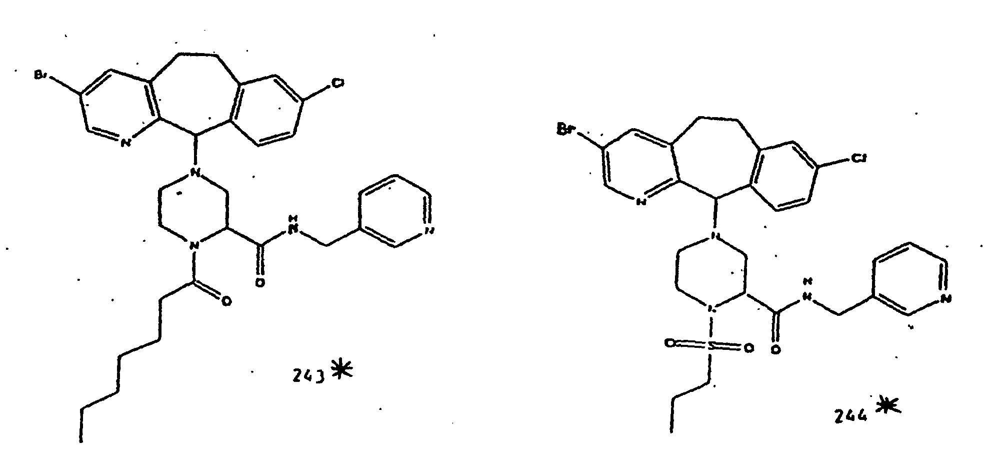

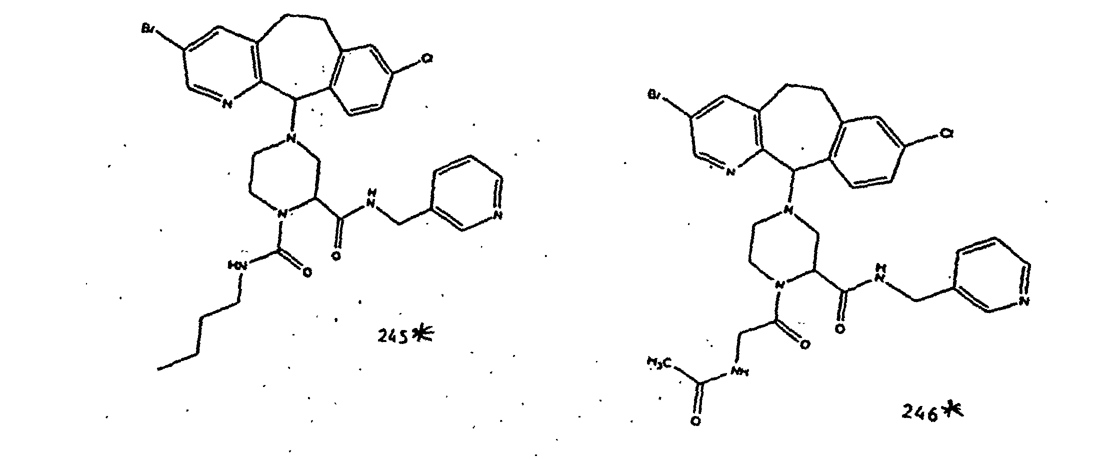

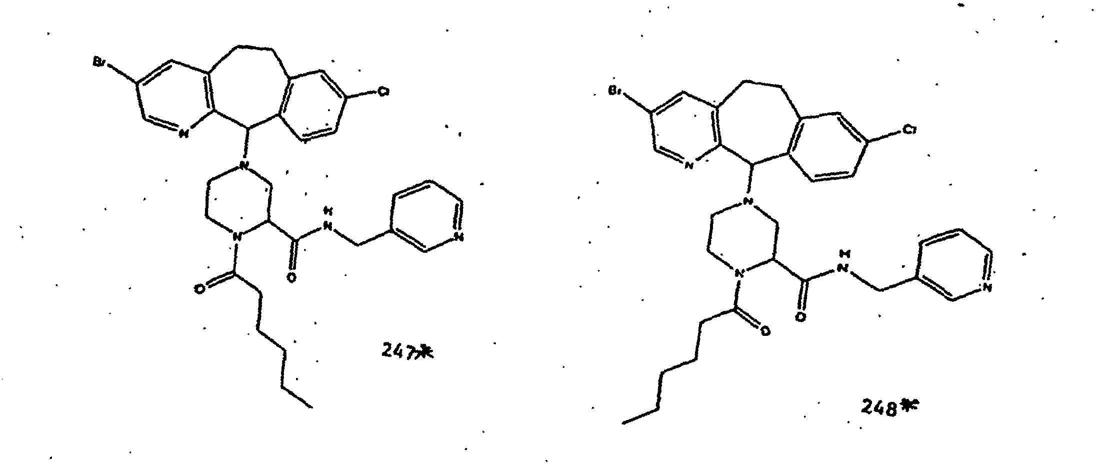

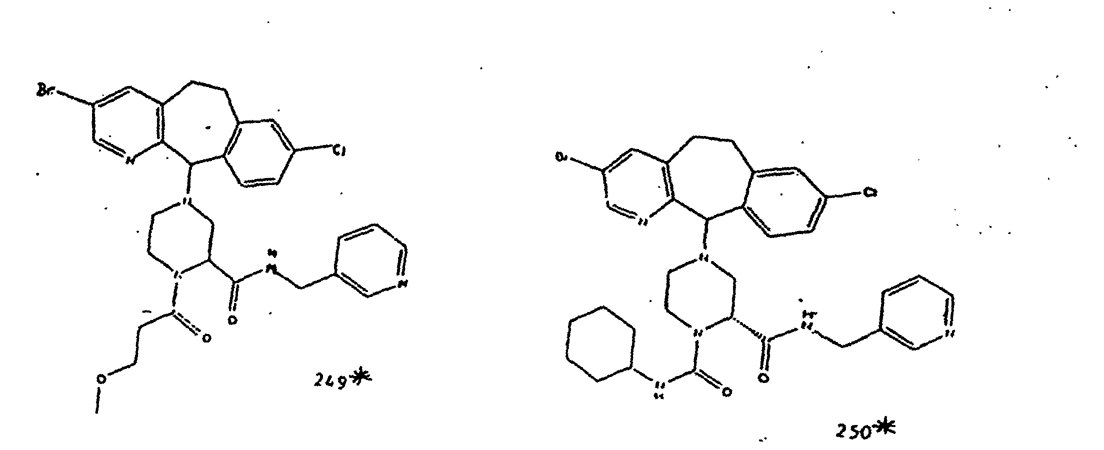

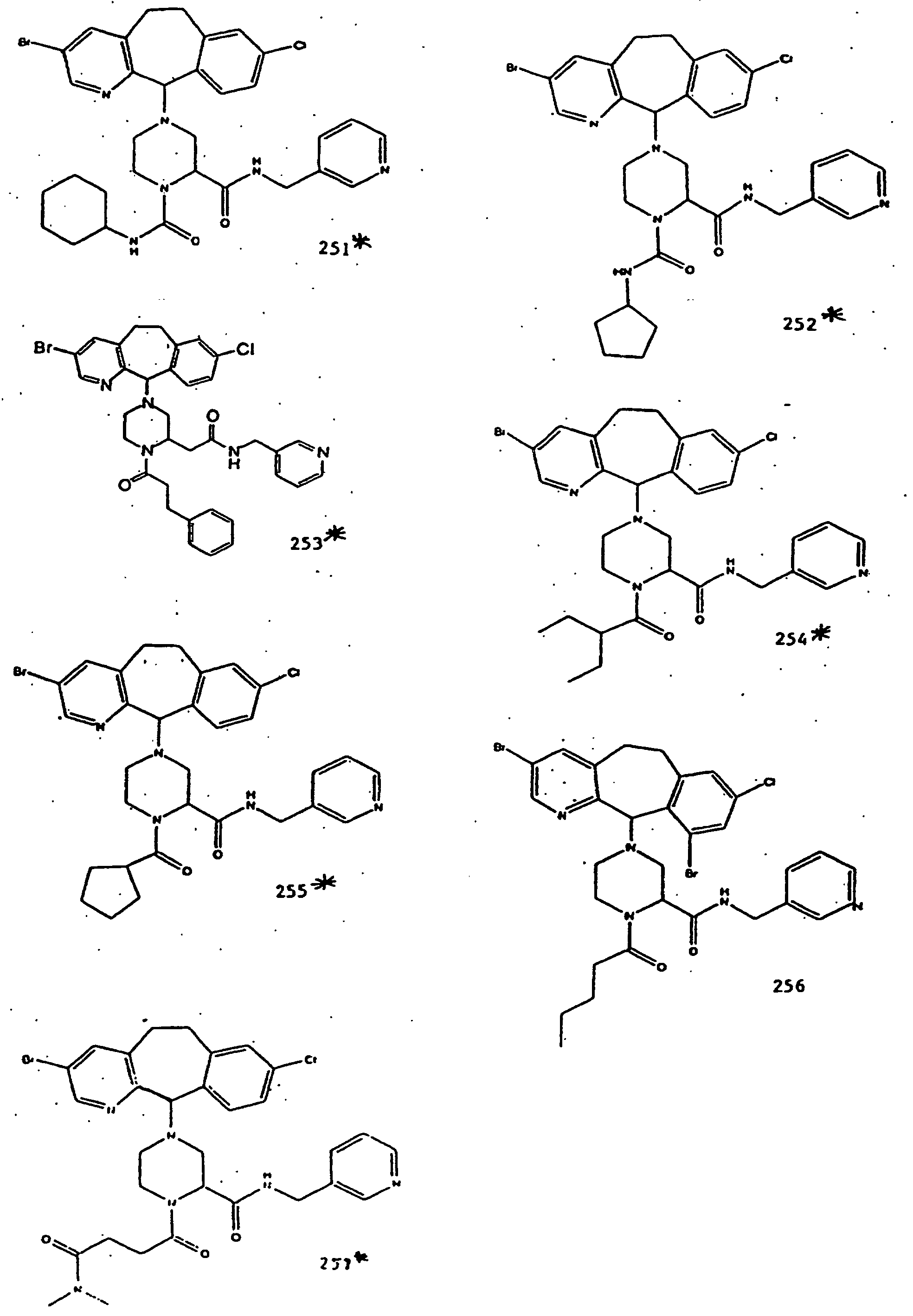

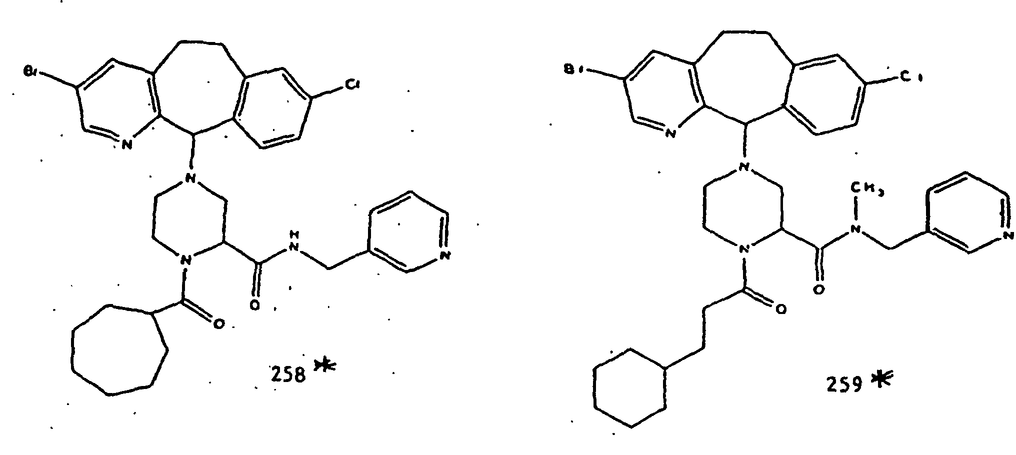

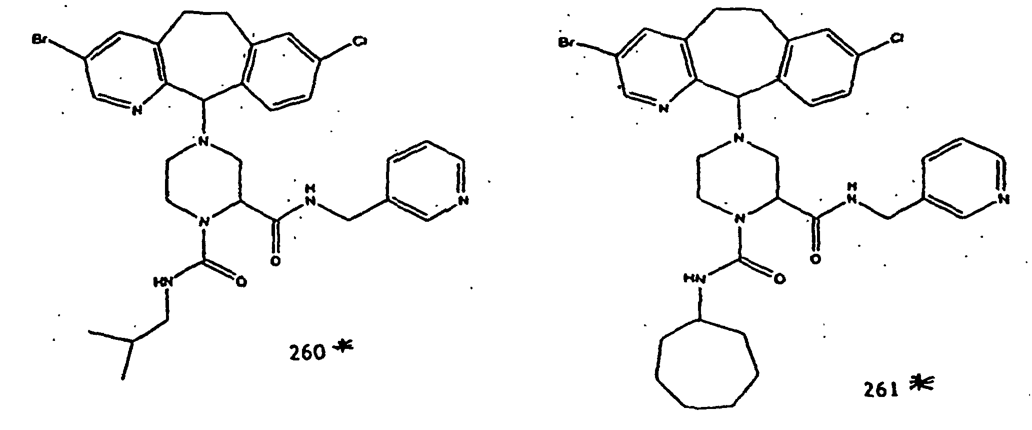

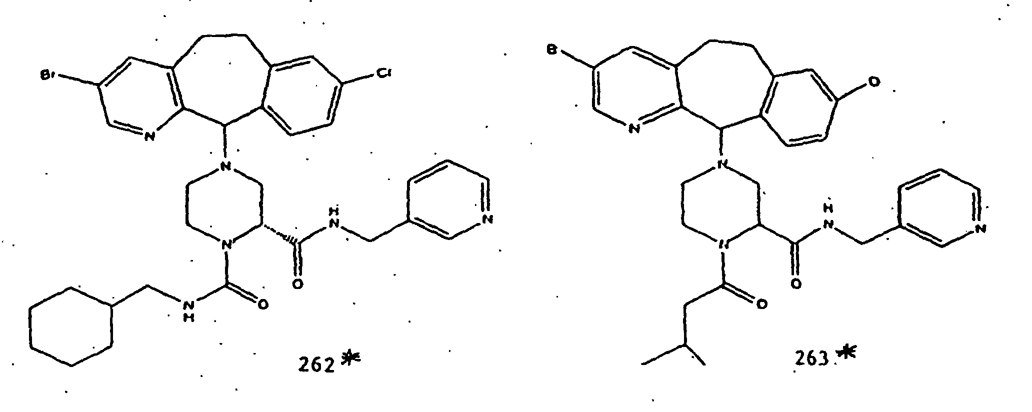

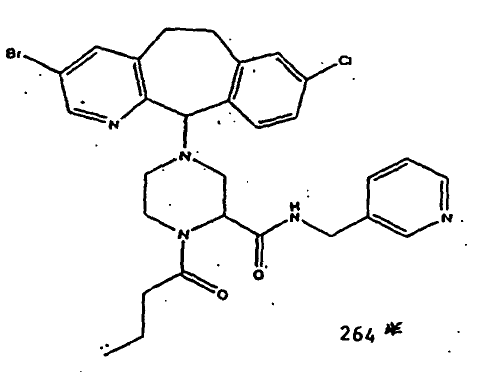

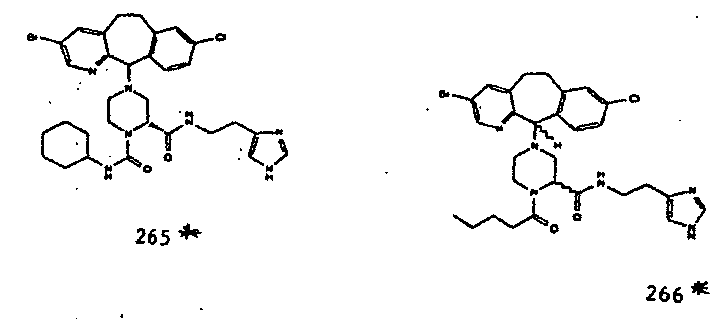

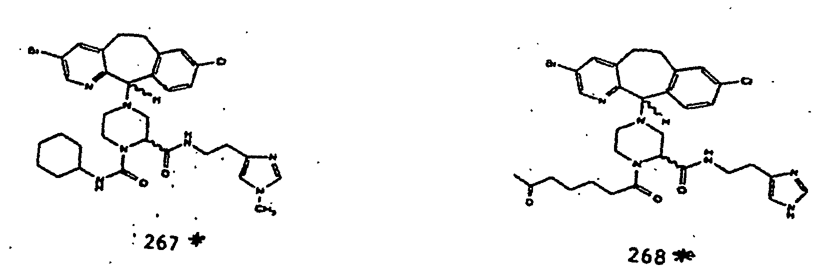

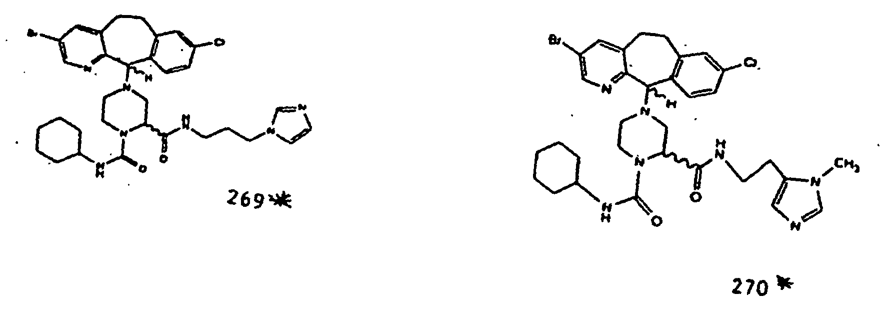

### ASSAYS

FPT IC₅₀ (inhibition of farnesyl protein transferase, in vitro enzyme assay). and COS Cell IC₅₀ (Cell-Based Assay) were determined following the assay procedures described in WO 95/10516, published April 20, 1995. GGPT IC₅₀ (inhibition of geranylgeranyl protein transferase, in vitro enzyme assay), Cell Mat Assay, and anti-tumor activity (in vivo anti-tumor studies) could be determined by the assay procedures described in WO 95/10516. The disclosure of WO 95/10516 is incorporated herein by reference thereto.

Additional assays can be carried out by following essentially the same procedure as described above, but with substitution of alternative indicator tumor cell lines in place of the T24-BAG cells. The assays can be conducted using either DLD-1-BAG human colon carcinoma cells expressing an activated K-ras gene or SW620-BAG human colon carcinoma cells expressing an activated K-ras gene. Using other tumor cell lines known in the art, the activity of the compounds of this invention against other types of cancer cells could be demonstrated.

### Soft Agar Assay:

Anchorage-independent growth is a characteristic of tumorigenic cell lines. Human tumor cells are suspended in growth medium containing 0.3% agarose and an indicated concentration of a farnesyl transferase inhibitor. The solution is overlayed onto growth medium solidified with 0.6% agarose containing the same concentration of farnesyl transferase inhibitor as the top layer. After the top layer is solidified, plates are incubated for 10-16 days at 37°C under 5% CO₂ to allow colony outgrowth. After incubation, the colonies are stained by overlaying the agar with a solution of MTT (3-[4,5-dimethylthiazol-2-yl]-2,5-diphenyltetrazolium bromide, Thiazolyl blue) (1 mg/mL in PBS). Colonies can be counted and the IC₅₀'s can be determined.

The results are given in the Table below ( "nM" represents nanomolar).

| Compound No, | FPT C₅₀ (nM) | COS Cell IC₅₀ (nM) | Soft Agar IC₅₀ | GGPTase IC₅₀ |
|---|---|---|---|---|
| 200 | 29 | | | |
| 201 | 3.2 | | | |
| 202 | 2.9 | | | |
| 203* | 3.3 | | | |
| 204 | 5.5 | | | |
| 205 | 77 | | | |
| 206 | 34 | | | |
| 207 | 55 | | | |
| 208 | 5.5 | | | |
| 209 | 47 | | | |
| 210 | 62 | | | |
| 211 | 17 | | | |
| 212 | 31 | | | |
| 213 | 180 | | | |
| 214 | 39 | | | |
| 215 | 140 | | | |
| 216 (Procedure 9 of Example 6) | >150 | | | |
| 217 | 17 | | | |
| 218 (Procedure 10 of Example 6) | 48 | | | |
| 219 | 11 | | | |
| 220 | 12 | | | |
| 221 | 50 | | | |
| 222 | 72 | | | |
| 223 | 12 | | | |
| 224 | 10 | | | |
| 225 | >130 | | | |
| 226 | 16 | | | |
| 227* (EXAMPLE 1) | 7.2 6.7 | 10-50 30 | | |
| 228* | 14, 12, 15 | 19.6±0.3 | 18 | |
| 229* | 11.5 | 59.5 | 140 | |
| 230* | 1200 | | | |
| 231* | 2200 | | | |
| 232* | >100,000 | | | |
| 233* | 4.2, 5.0 | 37±5 | 57±3 | |
| 234* | 33, 20 | | | |
| 235* | 93, 101 | 1100 | >1000 | |
| 236* | 26, 28 | | | |
| 237* | 216, 138 | | | |
| 238* | 7.4, 6.5 | >200 | >500 | |
| 239* | 22, 12, 28, 16 | 10-100 25-50 25-50 | 100-500 100 | |
| 240* | 35, 21 | 150-200 | >1000 | >50,000 |
| 241* | 11.5 9.5 9.0 | 53.5±8.5 | 83.5 ±2.5 | |
| 242* | 19 16 | 50-100 127 | 168±8 | 31.000 |
| 243* | 250 183 | | | |
| 244* | 35, 51, 49, 26 | 250-500 | 213±40 | |
| 245* | 314, 186 | | | |
| 246* | 1300 970 | | | |
| 247* | 8.2 6.7 9.0 | 75±2.5 | 87 | |
| 246* | 17 19 21 | | | |
| 249* | 19, 22, 23, 11, 24 | <100, 100 | 50-100 | 13000 79000 |
| 250* | 4.2 3.0 | 6.4±0.2 | 10-50 11 | |
| 251* | 6.3, 5.1, 4.8 5.7 | 8.6±0.2 | 38±14 | |
| 252* | 23 11 7.6 | 28.5±1.5 | | |
| 253* | 1000 910 | 2000 | | >500.000 |
| 254* | 870 1100 | | | |
| 255* | 23 12 15 | 49 94 | | |
| 256* | 6.7, 7.2 2.9 | 5.3±0 | 15.5±3.5 | |
| 257* | 12 14 | 9.5 12.5 | 52.5±14.5 | |
| 258* | 10 14 | 94 | | |
| 259* | 211 156 | 1000 100 | | |
| 260* | 7.1 8.9 | 25 | 64±14 | |
| 261* | 9.4 9.4 8.7 | 81 81 | | |
| 262* | 4.5 5.2 | 15.4 25 | 74.5±4.5 | |
| 263* | 7.8 6.8 | 10-100 50 | 50-100 50-100 174 | >100,000 |
| 264* | 415 290 | | | |
| 265* | 7.0 3.6 | 16 4 | 52 5.4 | |
| 266* | 13.5 9.5 | | 41 47 | |
| 267* | 27.3 | 108 | 307 | |
| 268* | 13.6 | 145 | | |
| 269* | 4.5±1.4 | 4 | | |
| 270* | 7.3±3nm | 0.4 | 1.5 | |

| | | | | |
|---|---|---|---|---|
| * not within the scope of the present invention | | | | |

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 70 percent active ingredient. Suitable solid carriers are known in the art, e.g. magnesium carbonate, magnesium stearate, talc, sugar, lactose. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection.

Liquid form preparations may also include solutions for intranasal administration.

Aerosol preparations suitable for inhalation may include solutions and solids in powder form, which may be in combination with a pharmaceutically acceptable carrier, such as an inert compressed gas.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in unit dosage form. In such form, the preparation is subdivided into unit doses containing appropriate quantities of the active component, e.g., an effective amount to achieve the desired purpose.

The quantity of active compound in a unit dose of preparation may be varied or adjusted from about 0.1 mg to 1000 mg, more preferably from about 1 mg. to 300 mg, according to the particular application.

The actual dosage employed may be varied depending upon the requirements of the patient and the severity of the condition being treated. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The amount and frequency of administration of the compounds of the invention and the pharmaceutically acceptable salts thereof will be regulated according to the judgment of the attending clinician considering such factors as age, condition and size of the patient as well as severity of the symptoms being treated. A typical recommended dosage regimen is oral administration of from 10 mg to 2000 mg/day preferably 10 to 1000 mg/day, in two to four divided doses to block tumor growth. The compounds are non-toxic when administered within this dosage range.

The following are examples of pharmaceutical dosage forms which contain a compound of the invention. The scope of the invention in its pharmaceutical composition aspect is not to be limited by the examples provided.

### Pharmaceutical Dosage Form Examples

### EXAMPLE A

### Tablets

| No. | Ingredients | mg/tablet | mg/tablet |
|---|---|---|---|
| 1. | Active compound | 100 | 500 |
| 2. | Lactose USP | 122 | 113 |
| 3. | Corn Starch, Food Grade, as a 10% paste in Purified Water | 30 | 40 |
| 4. | Corn Starch, Food Grade | 45 | 40 |
| 5. | Magnesium Stearate | 3 | 7 |
| Total | | 300 | 700 |

### Method of Manufacture

Mix Item Nos. 1 and 2 in a' suitable mixer for 10-15 minutes. Granulate the mixture with Item No. 3. Mill the. damp granules through a coarse screen (e.g., 1/4", 0.63 cm) if necessary. Dry the damp granules. Screen the dried granules if necessary and mix with Item No. 4 and mix for 10-15 minutes. Add Item No. 5 and mix for 1-3 minutes. Compress the mixture to appropriate size and weigh on a suitable tablet machine.

### EXAMPLE B

### Capsules

| No. | Ingredient | mg/capsule | mg/capsule |
|---|---|---|---|
| 1. | Active compound | 100 | 500 |
| 2. | Lactose USP | 106 | 123 |
| 3. | Corn Starch, Food Grade | 40 | 70 |
| 4. | Magnesium Stearate NF | 7 | 7 |
| Total | | 253 | 700 |

### Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a suitable encapsulating machine.

## Claims

1. A compound of the formula: 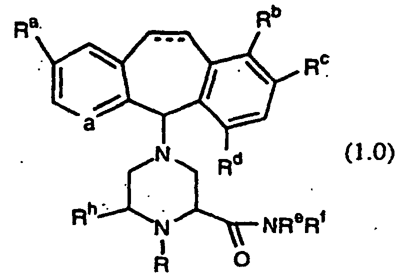 or a pharmaceutically acceptable salt or solvate thereof, wherein:
a represents N or NO⁻;
R^{a}, R^{c} , and R^{d} are halo and R^{b} is H;
the dotted line (---) represents an optional double bond;
R is selected from the group consisting of H, -S(O)₂R¹,
-S(O)₂NR¹R², -C(O)R¹, and -C(O)NR¹R², wherein R¹ and R² are independently selected from the group consisting of H, alkyl, aryl, arylalkyl, heteroaryl, heteroarylalkyl, (C₃-C₇) cycloalkyl, cycloalkylalkyl, heterocycloalkyl, substituted alkyl, substituted aryl, substituted arylalkyl, substituted heteroaryl, substituted heteroarylalkyl, substituted (C₃-C₇) cycloalkyl, substituted cycloalkylalkyl, substituted heterocycloalkyl, wherein said substituted groups have one or more substituents selected from: alkyl, alkoxy, aralkyl, heteroarylalkyl, -NO₂, alkyloxyalkyl, alkyloxyalkyloxyalkyl, C₃-C₇ cycloalkyl, aryl, -CN, heteroaryl, heterocycloalkyl, =O, -OH, amino, substituted amino, nitro and halo;
R^{e} and R^{f} are independently selected from H, alkyl, alkyloxyalkyl, alkyloxyalkyloxyalkyl, aryl, arylalkyl, heteroaryl, heteroarylalkyl, (C₃-C₇) cycloalkyl, cycloalkylalkyl, heterocycloalkyl, substituted alkyl, substituted alkyloxyalkyl, substituted alkyloxyalkyloxyalkyl, substituted aryl, substituted arylalkyl, substituted heteroaryl, substituted heteroarylalkyl, substituted (C₃-C₇) cycloalkyl, substituted cycloalkylalkyl, substituted heterocycloalkyl, wherein said substituted groups have one or more substituents selected from: alkyl, alkoxy, aralkyl, heteroarylalkyl, -NO₂, alkyloxyalkyl, alkyloxyalkyloxyalkyl, C₃-C₇ cycloalkyl, aryl, -CN, heteroaryl, heterocycloalkyl, =O, -OH, amino, substituted amino, nitro and halo; or R^{e} is selected from the group consisting of H, alkyl and aryl and R^{f} is represented by ―(CH₂)ₙ―R¹⁵, wherein n is an integer from 0 to 8 and R¹⁵ is selected from -C(O)NH₂, -SO₂NH₂, aryl, heteroaryl, cycloalkyl, heterocycloalkyl, optionally substituted by alkyl, alkoxy, aralkyl, heteroarylalkyl, -NO₂, alkyloxyalkyl, alkyloxyalkyloxyalkyl, C₃ - C₇ cycloalkyl, aryl, -CN, heterocycloalkyl, =O, -OH, amino, substituted amino, nitro and halo;
or R¹⁵ is 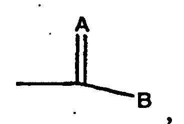 wherein B is OH or NH₂ and A is NH O, NOH or NCN, or R¹⁵ is NR¹⁶R¹⁷, wherein R¹⁶ is H or alkyl and R¹⁷ is H, alkyl, SO₂CH₃, or C(O)NH₂; or R^{e} and R^{f} together with the nitrogen to which they are bound, form a 5 or 6 membered heterocycloalkyl ring which is optionally substituted by OH, NH₂, NHR¹⁶, NHR¹⁷, NR¹⁶R¹⁷, or (CH₂)ₙR¹⁸R¹⁹, wherein R¹⁶ and R¹⁷ are as defined above, R¹⁸ is H or C₁-C₆ alkyl, and R¹⁹ is selected from H, C₁-C₆ alkyl, substituted alkyl, arylalkyl, acyl (e.g., acetyl, benzoyl, etc.), carboxamido, alkyloxycarbonyl (e.g., methoxycarbonyl), arylalkyloxycarbonyl (e.g., benzyloxycarbonyl), amido derivatives derived from amino acids (e.g., glycine,alnine, serine,etc.), imidate (e.g., phenoxyimidate), cyanide, imidamido (e.g., C(=NH)NH₂, (C=NSO₂NH₂)NH₂, etc.), sulfonamido (e.g., SO₂NH₂, SO₂N(CH₃)₂) sulfonyl (e.g., SO₂CH₃, SO₂C₆H₅, SO₂CH₂C₆H₅, etc.), phosphinate (e.g. P(=O)(CH₃)₂), heterocyclyl and imidamido (e.g., (C=NC₆H₅)C₆H₅), (C=NH)C₆H₅ ,etc.), wherein n is as defined above; and R^{h} is H or =O; with the further proviso that when R^{h} is H and R^{b} and R^{d} are 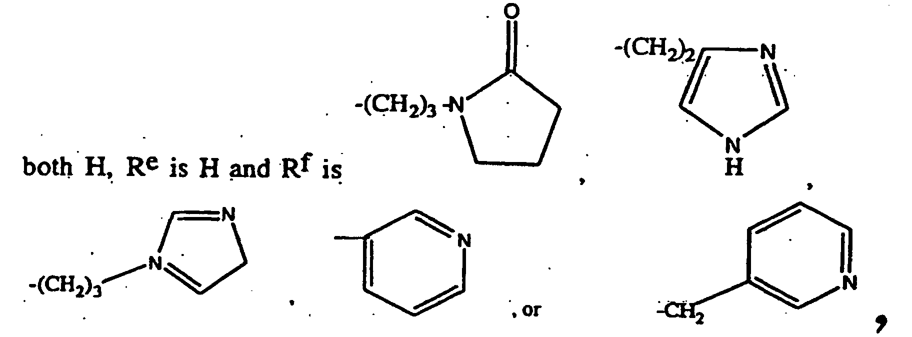 wherein, unless indicated otherwise, the terms have the following meanings:
"alkyl" (including the alkyl portions of alkoxy, alkylamino and dialkylamino) represents straight and branched carbon chains and contains from one to twenty carbon atoms;
"alkyloxyalkyl" represents alkyl bound to an oxygen atom which in turn is bound to alkyl;
"alkyloxyalkyloxyalkyl" represents alkyl bound to oxygen, which in turn is bound to alkyl, which in turn is bound to oxygen, which in turn, is bound to alkyl;
"cycloalkyl" represents saturated carbocyclic rings branched or unbranched of from 3 to 20 carbon atoms'';
''heterocycloalkyl" represents a saturated, branched or unbranched carbocyclic ring containing from 3 to 15 carbon atoms, which carbocyclic ring is interrupted by 1 to 3 hetero groups selected from -O-, -S- or -NR¹⁰-, wherein R¹⁰ is H, alkyl, aryl or arylalkyl;
"aryl" (including the aryl portion of aryloxy and aralkyl) represents a carbocyclic group containing from 6 to 15 carbon atoms and having at least one aromatic ring, with all available substitutable carbon atoms of the carbocyclic group being intended as possible points of attachment, said carbocyclic group being optionally substituted (e.g. 1 to 3) with one or more of halo, alkyl, hydroxy, alkoxy, phenoxy, CF₃, amino, alkylamino, dialkylamino, -COOR¹⁰ or -NO₂;
"halo" represents fluoro, chloro, bromo and iodo; and
"heteroaryl" represents cyclic groups, and having at least one heteroatom selected from O, S or N, said heteroatom interrupting a carbocyclic ring structure and having a sufficient number of delocalized pi electrons to provide aromatic character, with the aromatic heterocyclic groups containing from 2 to 14 carbon atoms.

2. The Compound of Claim 1 wherein Re is H, R^{f} is -(CH₂)ₙR¹⁵ and R¹⁵ is selected from :

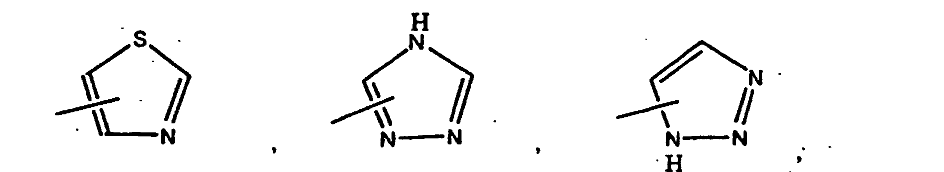

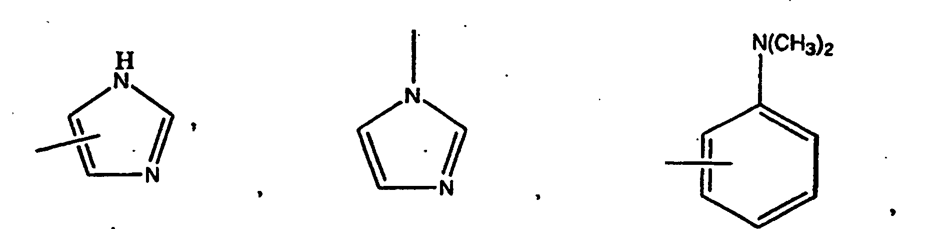

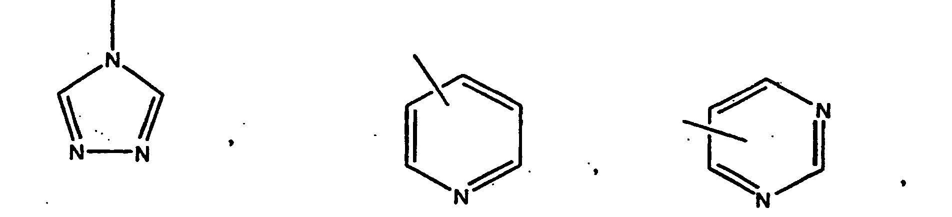

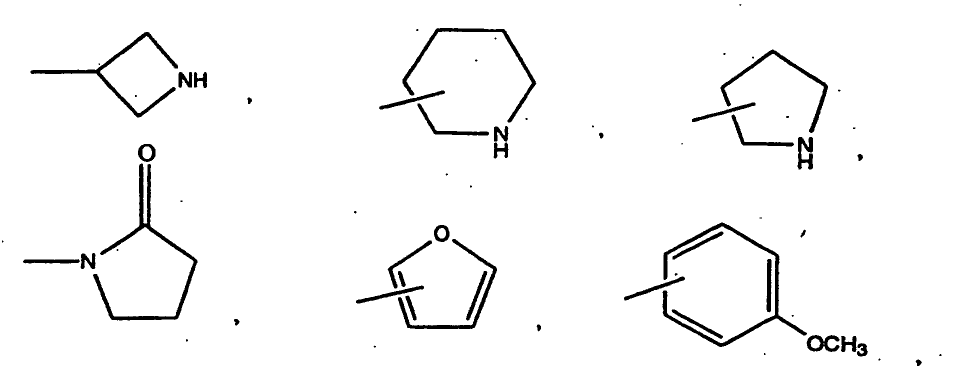

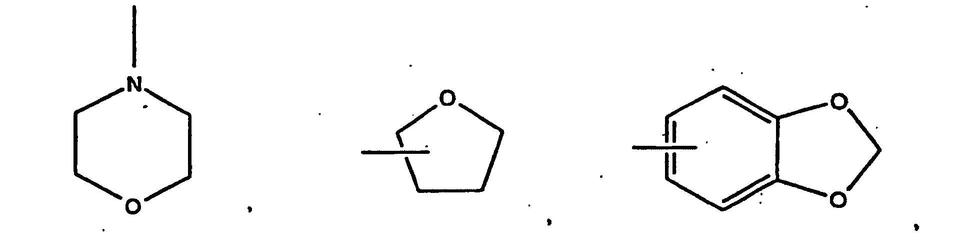

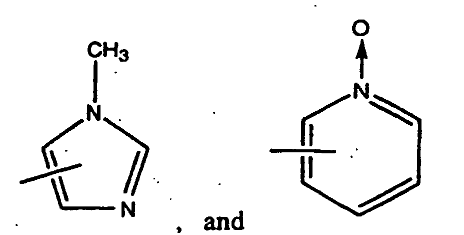

3. The compound of claim 1, wherein a is N and R^{h} is H.

4. The compound of claim 1, wherein R^{a} and R^{d} are Br and R^{c} is Cl.

5. The compound of claim 2, wherein R is selected from the group consisting of H, -C(O)(CH₂)₃CH₃, -S(O)₂CH₃, -C(O)-CH₂-O-(CH₂)₂-O-CH₃, -C(O)-CH₂-OCH₃, -C(O)NH(CH₂)₃CH₃,

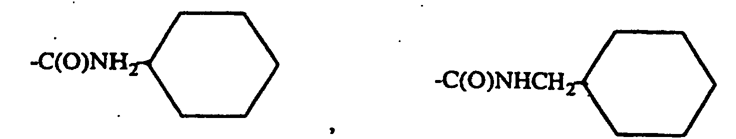

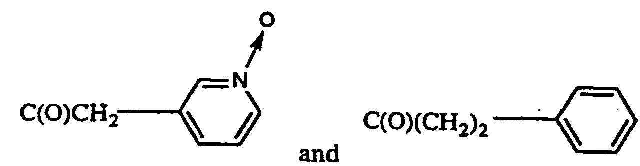

6. The compound of claim 1, wherein R^{h} is O=.

7. The compound of claim 1, wherein R^{e} is H and R^{f} is
-(CH₂)ₙ-R¹⁵.

8. The compound of claim 1, wherein R^{f} is

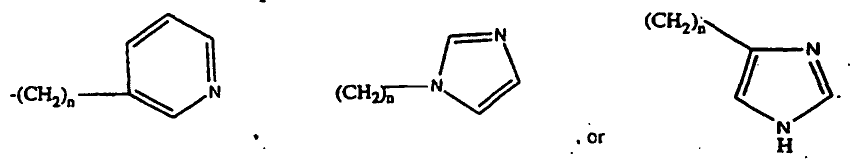

9. A compound, or a pharmaceutically acceptable salt or solvate thereof, selected from the group consisting of:

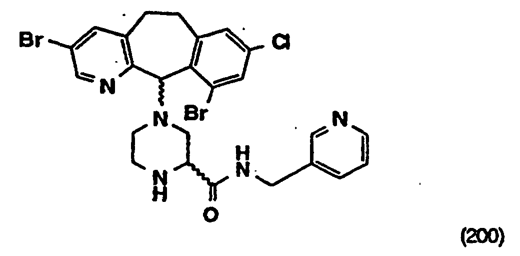

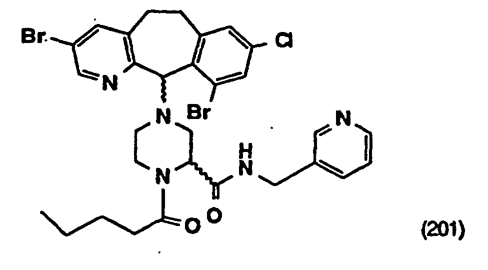

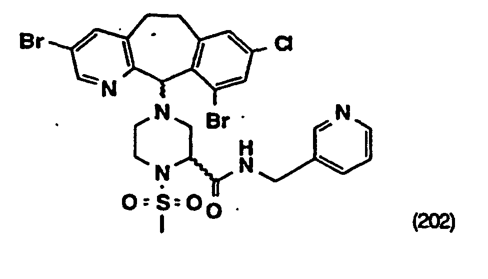

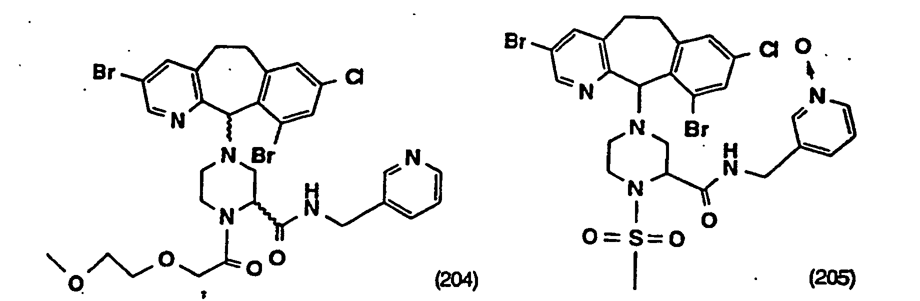

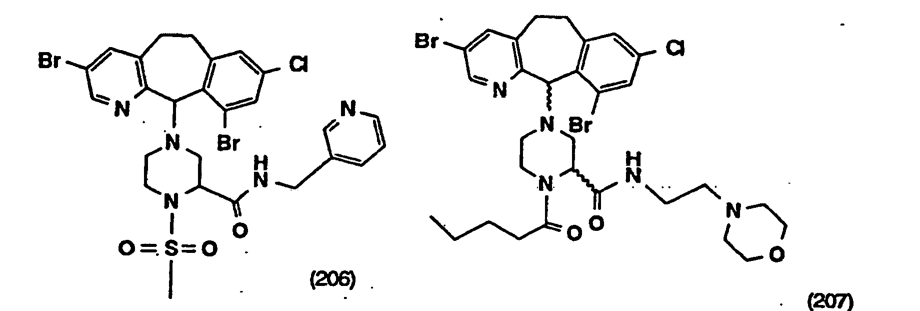

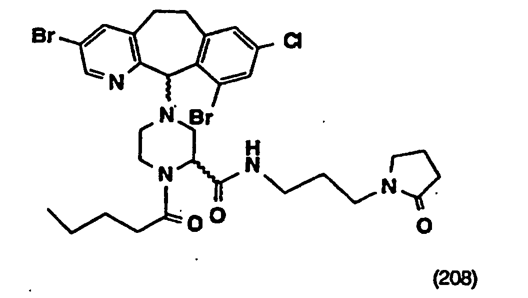

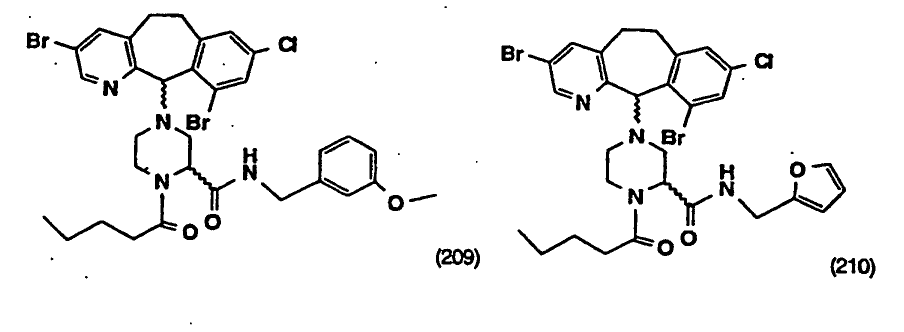

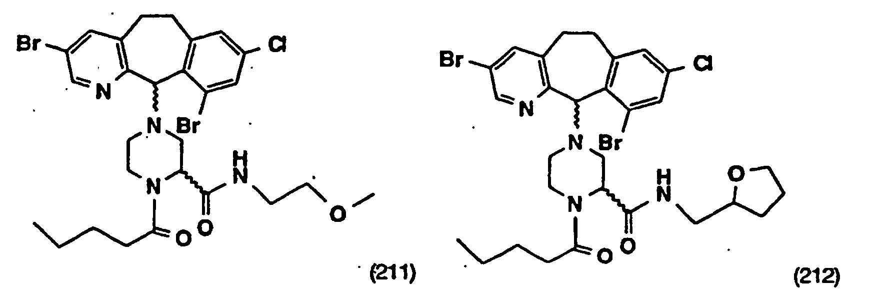

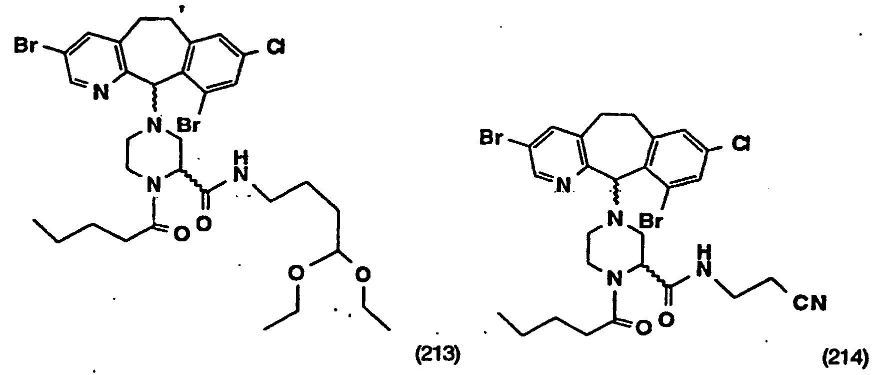

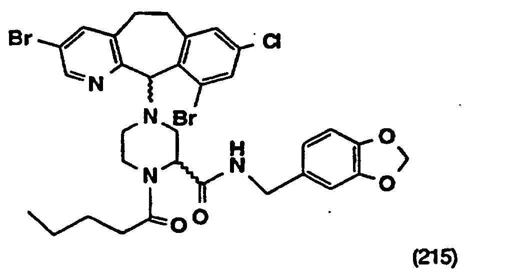

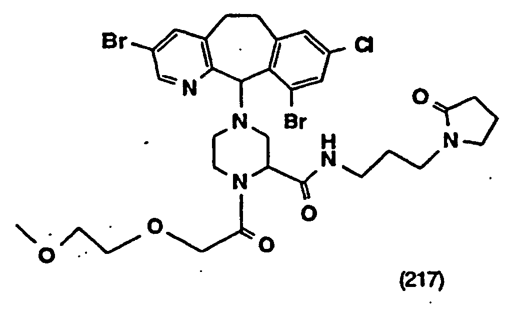

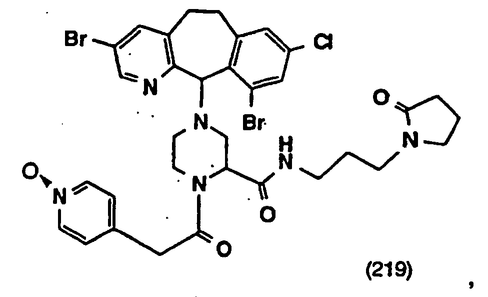

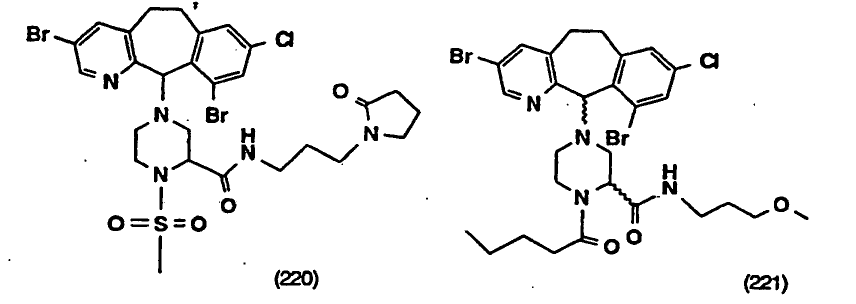

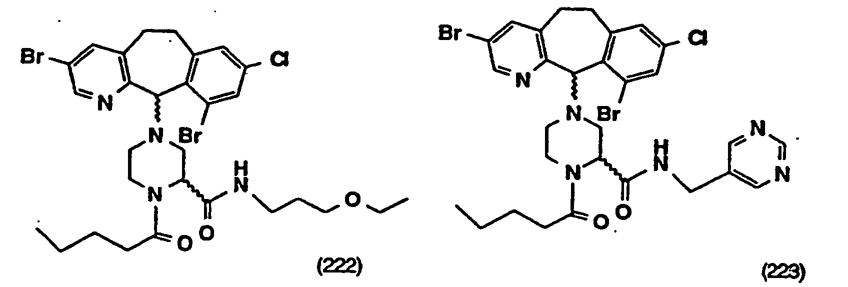

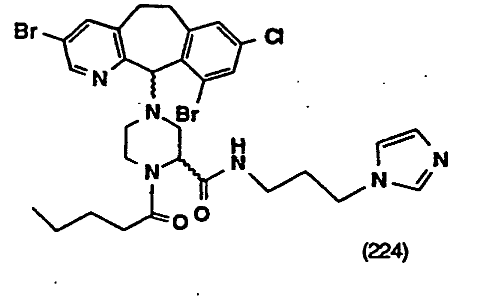

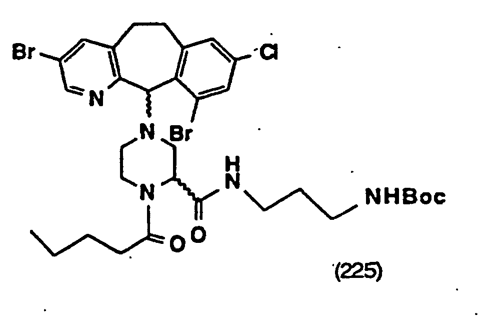

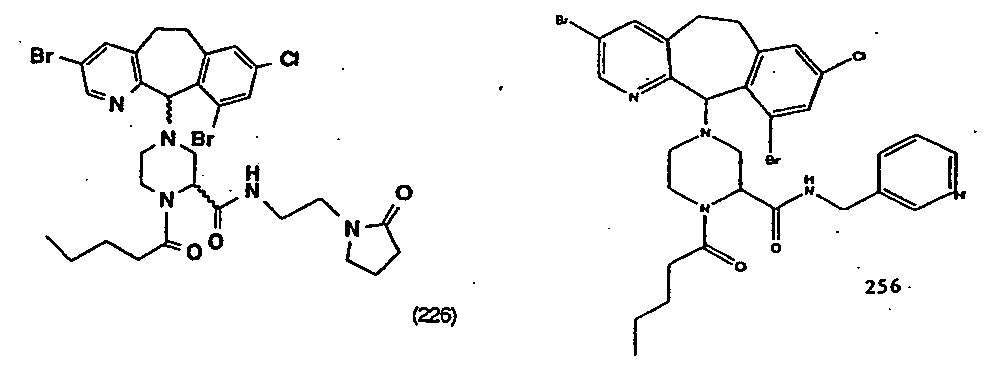

10. Use of a compound according to any preceding claim for the manufacture of a medicament for inhibiting the abnormal growth of cells.

11. Use according to claim 10 wherein the cells inhibited are tumor cells expressing an activated ras oncogene.

12. Use according to Claim 10 or Claim 11 wherein the cells inhibited are pancreatic tumor cells, lung cancer cells, myeloid leukemia tumor cells, thyroid follicular tumor cells, myelodysplastic tumor cells, epidermal carcinoma tumor cells, bladder carcinoma tumor cells, or colon tumor cells.

## Patentansprüche

1. Verbindung mit der Formel 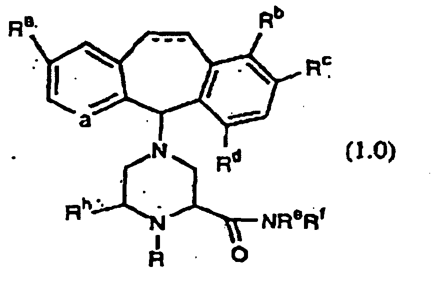 oder pharmazeutisch annehmbares Salz oder Solvat davon, worin
a für N oder NO⁻ steht;
R^{a}, R^{c} und R^{d} Halogen sind und R^{b} H ist;
die punktierte Linie (...) für eine optionale Doppelbindung steht;
R ausgewählt ist aus der Gruppe bestehend aus H, -S(O)₂R¹, -S(O)₂NR¹R², -C(O)R¹ und -C(O)NR¹R², wobei R¹ und R² unabhängig ausgewählt sind aus der Gruppe bestehend aus H, Alkyl, Aryl, Arylalkyl, Heteroaryl, Heteroarylalkyl, (C₃- bis C₇)-Cycloalkyl, Cycloalkylalkyl, Heterocycloalkyl, substituiertem Alkyl, substituiertem Aryl, substituiertem Arylalkyl, substituiertem Heteroaryl, substituiertem Heteroarylalkyl, substituiertem (C₃- bis C₇)-Cycloalkyl, substituiertem Cycloalkylalkyl, substituiertem Heterocycloalkyl, wobei die substituierten Gruppen einen oder mehrere Substituenten ausgewählt aus Alkyl, Alkoxy, Aralkyl, Heteroarylalkyl, -NO₂, Alkyloxyalkyl, Alkyloxyalkyloxyalkyl, C₃- bis C₇-Cycloalkyl, Aryl, -CN, Heteroaryl, Heterocycloalkyl, =O, -OH, Amino, substituiertem Amino, Nitro und Halogen aufweisen;
R^{e} und R^{f} unabhängig ausgewählt sind aus H, Alkyl, Alkyloxyalkyl, Alkyloxyalkyloxyalkyl, Aryl, Arylalkyl, Heteroaryl, Heteroarylalkyl, (C₃- bis C₇)-Cycloalkyl,- Cycloal,kylalkyl, Heterocycloalkyl, substituiertem Alkyl, substituiertem Alkyloxyalkyl, substituiertem Alkyloxyalkyloxyalkyl, substituiertem Aryl, substituiertem Arylalkyl, substituiertem Heteroaryl, substituiertem Heteroarylalkyl, substituiertem (C₃- bis C₇)-Cycloalkyl, substituiertem Cycloalkylalkyl, substituiertem Heterocycloalkyl, wobei die substituierten Gruppen einen oder mehrere Substituenten ausgewählt aus Alkyl, Alkoxy, Aralkyl, Heteroarylalkyl, -NO₂, Alkyloxyalkyl, Alkyloxyalkyloxyalkyl, C₃- bis C₇-Cycloalkyl, Aryl, -CN, -Heteroaryl, Heterocycloalkyl, =O, -OH, Amino, substituiertem Amino, Nitro und Halogen aufweisen; oder R^{e} ausgewählt ist aus der Gruppe bestehend aus H, Alkyl und Aryl, und R^{f} durch - (CH₂)ₙ-R¹⁵ wiedergegeben wird, wobei n eine ganze Zahl von 0 bis 8 ist und R¹⁵ ausgewählt ist aus -C(O)NH₂, -SO₂NH₂, Aryl, Heteroaryl, Cycloalkyl, Heterocycloalkyl, gegebenenfalls substituiert durch Alkyl, Alkoxy, Aralkyl, Heteroarylalkyl, -NO₂, Alkyloxyalkyl, Alkyloxyalkyloxyalkyl, C₃- bis C₇-Cycloalkyl, Aryl, -CN, Heterocycloalkyl, =O, -OH, Amino, substituiertem Amino, Nitro und Halogen;
oder R¹⁵ 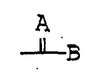 ist, wobei B OH oder NH₂ ist und A NH, O, NOH oder NCN ist, oder R¹⁵ NR¹⁶R¹⁷ ist, wobei R¹⁶ H oder Alkyl ist und R¹⁷ H, Alkyl, SO₂CH₃ oder C(O)NH₂ ist; oder R^{e} und R^{f} zusammen mit dem Stickstoff, an den sie gebunden sind, einen 5- oder 6-gliedrigen Heterocycloalkylring bilden, der gegebenenfalls durch OH, NH₂, NHR¹⁶, NHR¹⁷, NR¹⁶R¹⁷ oder (CH₂)ₙR¹⁸R¹⁹ substituiert ist, wobei R¹⁶ und R¹⁷ wie oben definiert sind, R¹⁸ H oder C₁- bis C₆-Alkyl ist und R¹⁹ ausgewählt ist aus H, C₁- bis C₆-Alkyl, substituiertem Alkyl, Arylalkyl, Acyl (z. B. Acetyl, Benzoyl, usw.), Carboxamido, Alkyloxycarbonyl (z. B. Methoxycarbonyl), Arylalkyloxycarbonyl (z. B. Benzyloxycarbonyl), Amidoderivaten, die von Aminosäuren (z. B. Glycin, Alanin, Serin, usw.) abgeleitet sind, Imidat (z. B. Phenoxyimidat), Cyanid, Imidamido (z. B. C(=NH)NH₂, (C=NSO₂NH₂)NH₂, usw.), Sulfonamido (z. B. SO₂NH₂, SO₂N(CH₃)₂), Sulfonyl (z. B. SO₂CH₃, SO₂C₆H₅, SO₂CH₂C₆H₅, usw.), Phosphinat (z. B. P(=O) (CH₃)₂), Heterocyclyl und Imidamido (z.B. (C=NC₆H₅) C₆H₅), (C=NH) C₆H₅, usw.), wobei n wie oben definiert ist; und R^{h} H oder =O ist; mit der weiteren Maßgabe, dass, wenn R^{h} H ist und R^{b} und R^{d} beide H sind, R^{e} H ist und R^{f} 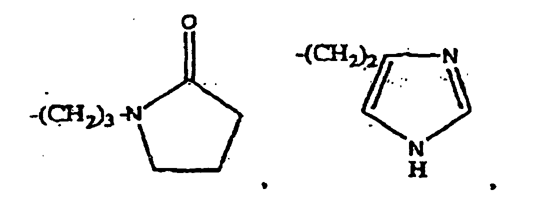 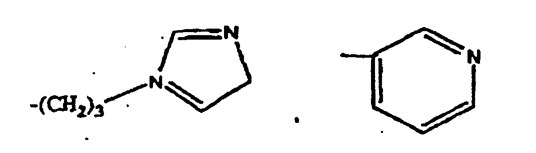 oder 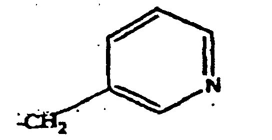 ist, wobei, wenn nicht anders angegeben, die Begriffe die folgenden Bedeutungen haben:
"Alkyl" (einschließlich der Alkylanteile von Alkoxy, Alkylamino und Dialkylamino) für geradkettige und verzweigte Kohlenstoffketten steht und ein bis zwanzig Kohlenstoffatome enthält;
"Alkyloxyalkyl" für Alkyl gebunden an ein Sauerstoffatom steht, das wiederum an Alkyl gebunden ist;
"Alkyloxyalkyloxyalkyl" für Alkyl gebunden an ein Sauerstoffatom steht, das wiederum an Alkyl gebunden ist, das wiederum an Sauerstoff gebunden ist, das wiederum an Alkyl gebunden ist;
"Cycloalkyl" für gesättigte carbocyclische Ringe steht, die verzweigt oder unverzweigt sind und 3 bis 20 Kohlenstoffatome aufweisen;
"Heterocycloalkyl" für gesättigte, verzweigte oder unverzweigte carbocyclische Ringe steht, die 3 'bis 15 Kohlenstoffatome enthalten, wobei der carbocyclische Ring durch 1 bis 3 Heterogruppen ausgewählt aus -O-, -S- oder -NR¹⁰unterbrochen ist, wobei R¹⁰ H, Alkyl, Aryl oder Arylalkyl ist;
"Aryl" (einschließlich des Arylanteils von Aryloxy und Aralkyl) für eine carbocyclische Gruppe steht, die 6 bis 15 Kohlenstoffatome enthält und mindestens einen aromatischen Ring aufweist, wobei alle verfügbaren substituierbaren Kohlenstoffatome der carbocyclischen Gruppe als mögliche Bindungspunkte vorgesehen sind, wobei die carbocyclische Gruppe gegebenenfalls mit einem oder mehreren von Halogen, Alkyl, Hydroxy, Alkoxy, Phenoxy, CF₃, Amino, Alkylamino, Dialkylamino, -COOR¹⁰ oder -NO₂ substituiert (z. B. 1 bis 3) ist;
"Halogen" für Fluor, Chlor, Brom und Iod steht; und
"Heteroaryl" für cyclische Gruppen steht, die mindestens ein Heteroatom ausgewählt aus 0, S oder N aufweisen, wobei das Heteroatom eine carbocyclische Ringstruktur unterbricht und eine ausreichende Anzahl delokalisierter n-Elektronen aufweist, um aromatischen Charakter zu liefern, wobei die aromatischen heterocyclischen Gruppen 2 bis 14 Kohlenstoffatome enthalten.

2. Verbindung nach Anspruch 1, bei der Re H, ist, R^{f} -(CH₂)ₙR¹⁵ ist und R¹⁵ ausgewählt ist aus

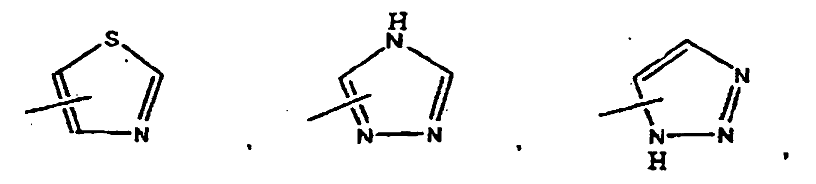

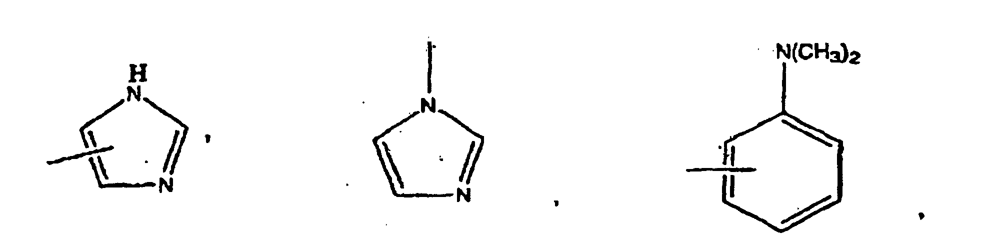

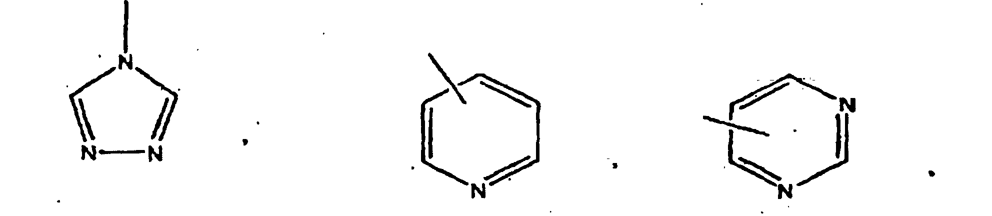

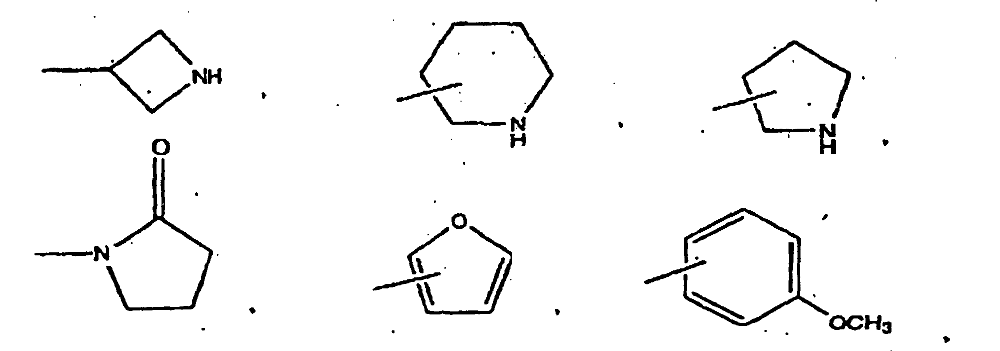

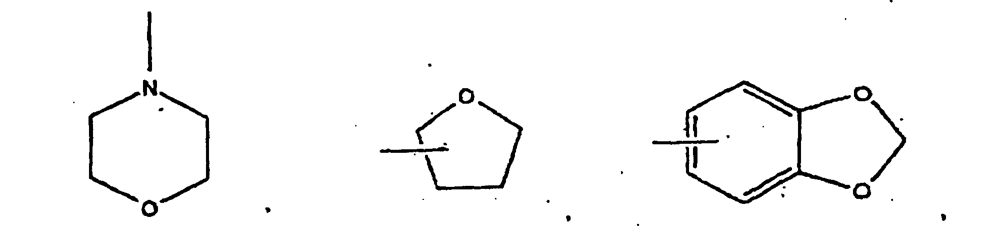

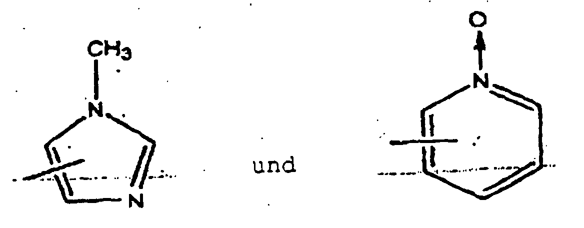

3. Verbindung nach Anspruch 1, bei der a N ist und R^{h} H ist.

4. Verbindung nach Anspruch 1, bei der R^{a} und R^{d} Br sind und R^{c} Cl ist.

5. Verbindung nach Anspruch 2, bei der R ausgewählt ist aus der Gruppe bestehend aus H, -C(O) (CH₂)₃CH₃, -S(O)₂CH₃, -C(O)-CH₂-O-(CH₂)₂-O-CH₃, -C(O)-CH₂-OCH₃, -C(O)NH(CH₂)₃CH₃,

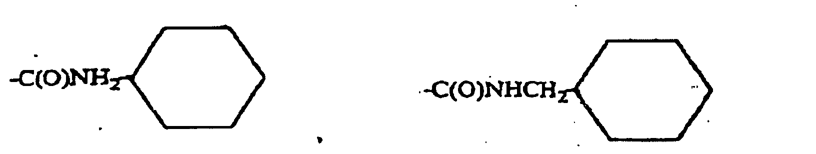

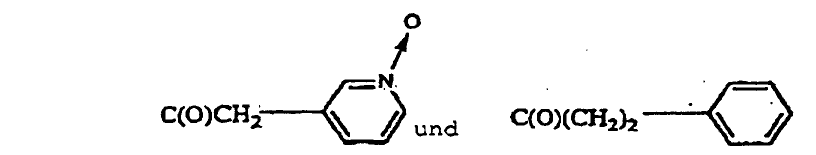

6. Verbindung nach Anspruch 1, bei der R^{h} O= ist.

7. Verbindung nach Anspruch 1, bei der R^{e} H ist und R^{f} -(CH₂)ₙ-R¹⁵ ist.

8. Verbindung nach Anspruch 1, bei der R^{f} 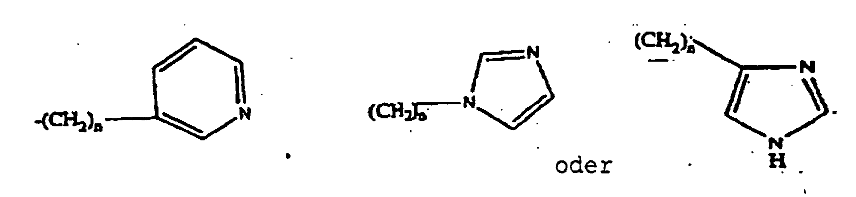 ist.

9. Verbindung, oder pharmazeutisch annehmbares Salz oder Solvat davon, ausgewählt aus der Gruppe bestehend aus

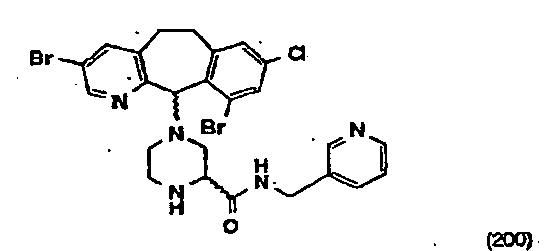

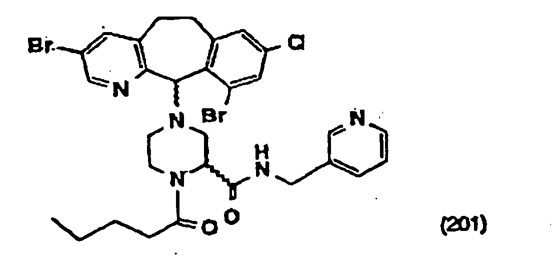

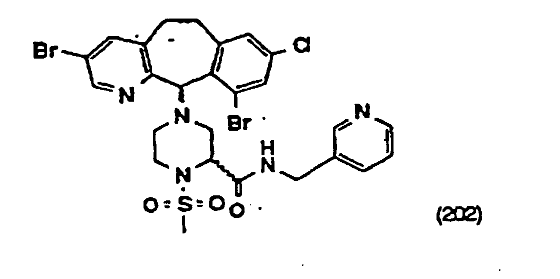

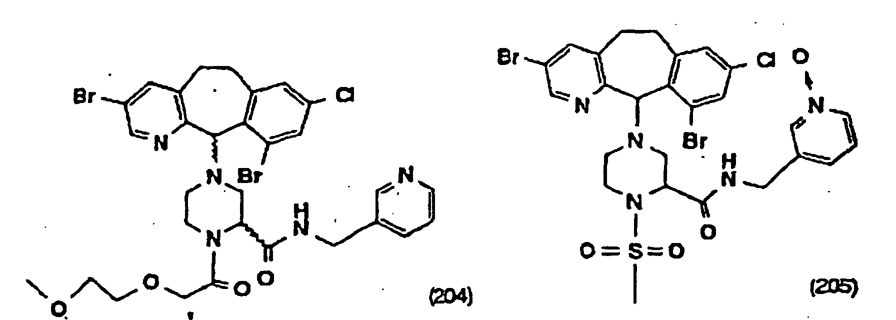

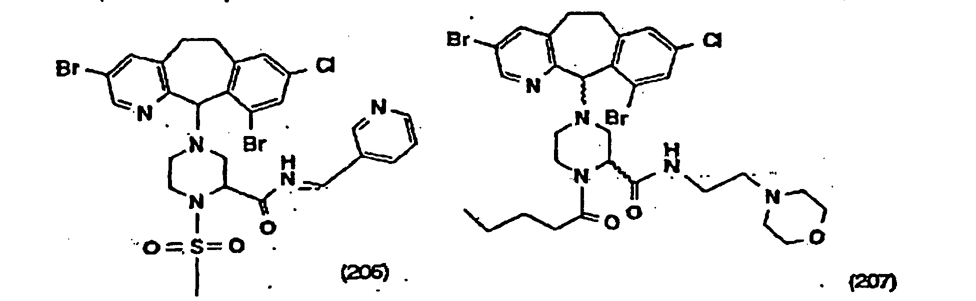

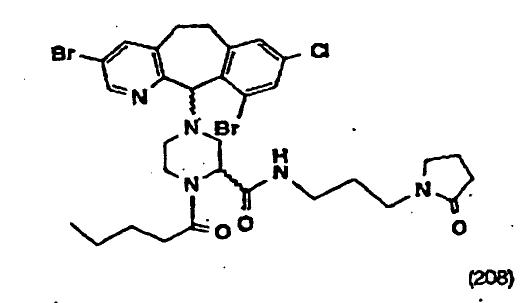

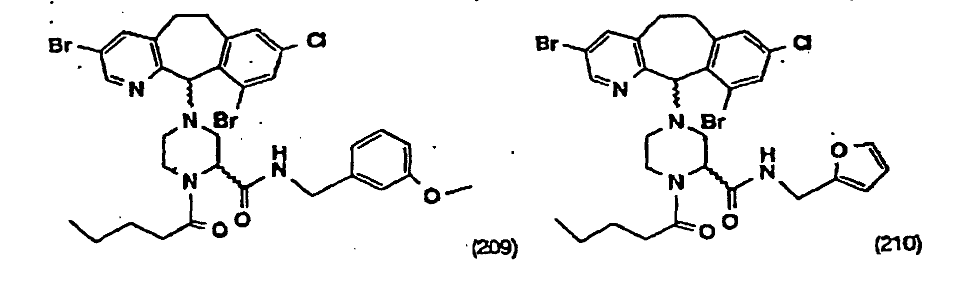

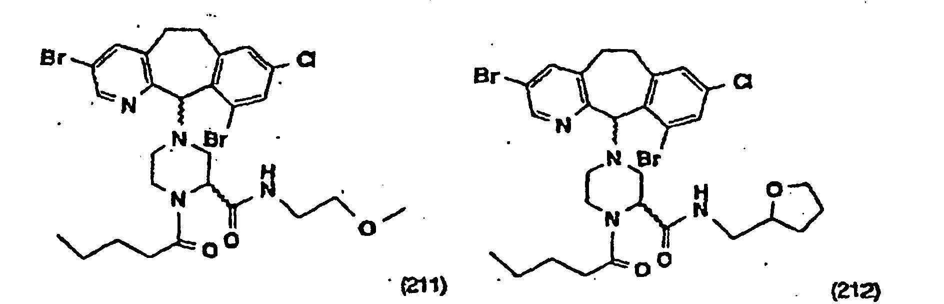

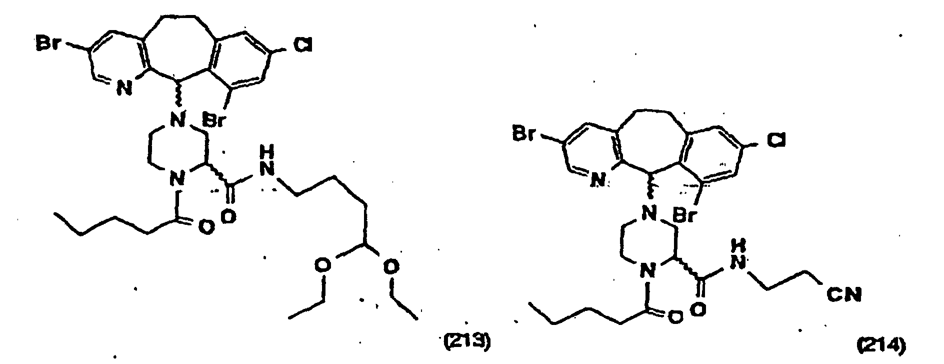

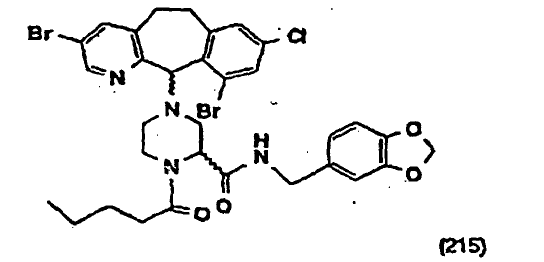

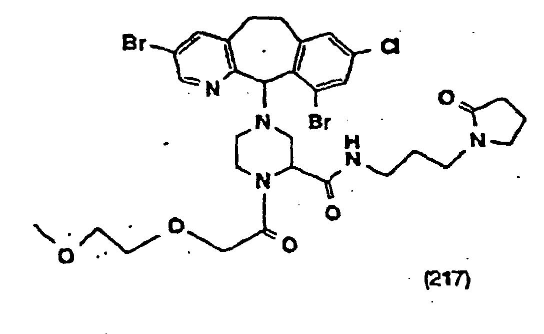

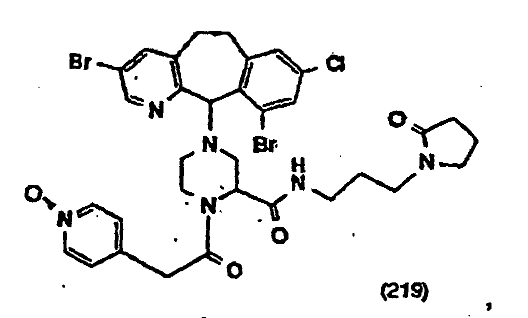

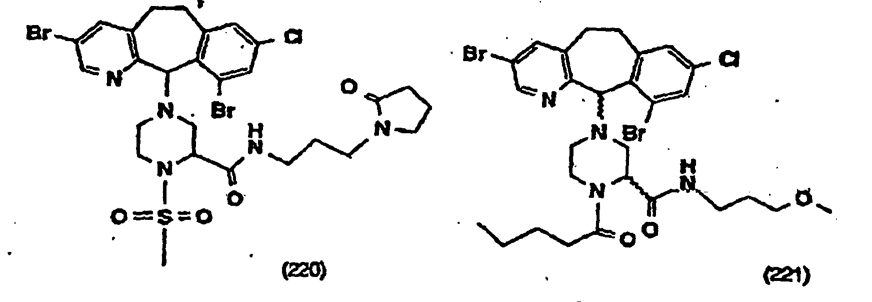

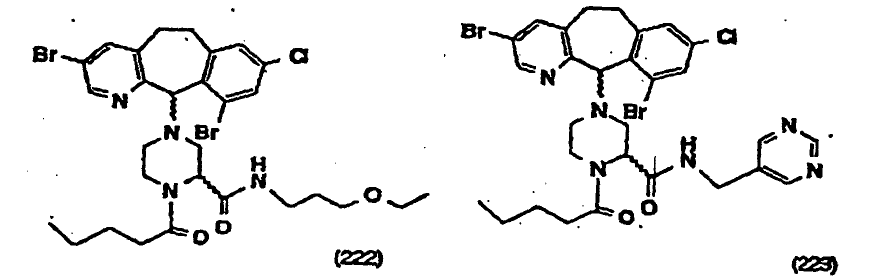

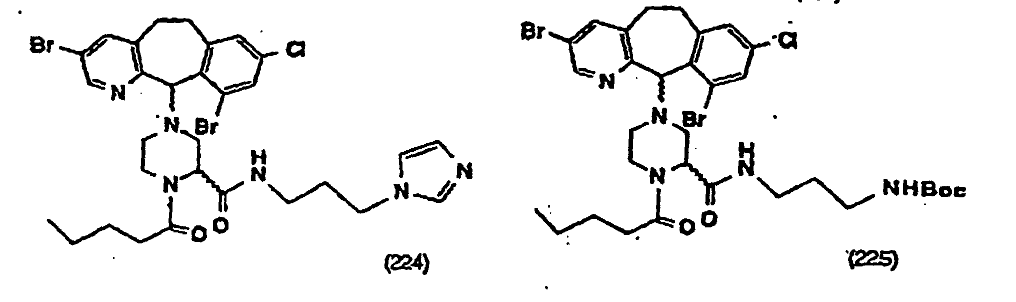

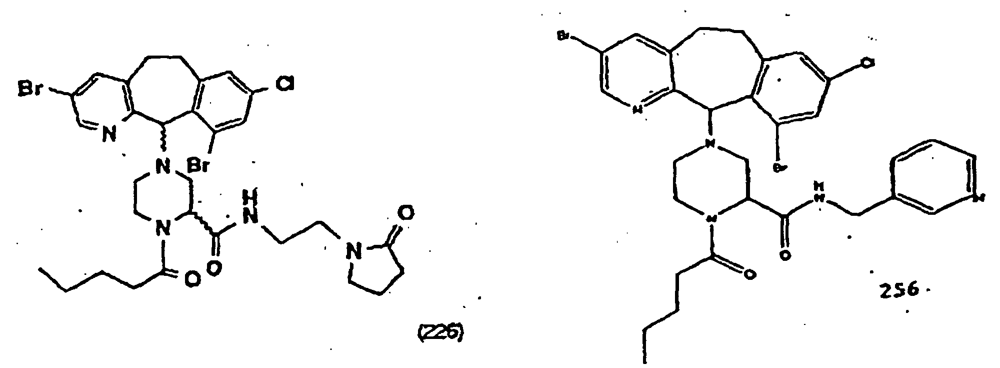

10. Verwendung einer Verbindung nach einem der vorhergehenden Ansprüche zur Herstellung eines Medikaments zur Inhibierung des abnormalen Wachstums von Zellen.

11. Verwendung nach Anspruch 10, bei der die inhibierten Zellen Tumorzellen sind, die ein aktiviertes ras-Onkogen exprimieren.

12. Verwendung nach Anspruch 10 oder 11, bei der die inhibierten Zellen Pankreastumorzellen, Lungenkrebszellen, myeloide Leukämietumorzellen, Thyroidfollikel-Tumorzellen, myelodysplastische Tumorzellen, Epidermalkarzinom-Tumorzellen, Blasenkarzinom-Tumorzellen oder Colon-Tumcrzellen sind.

## Revendications

1. Composé de formule : 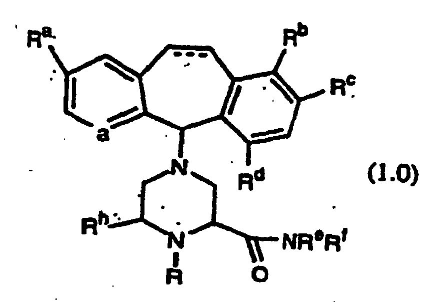 ou un sel ou un produit d'addition de solvant pharmaceutiquement acceptable de ce dernier, dans laquelle :
a représente N ou NO- ;
R^{a}, R^{c} et R^{d} représentent un atome d'halogène et R^{b} représente H ;
les traits en pointillés (... ) représente une double liaison optionnelle ;
R est choisi dans le groupe formé par H-, -S(O)₂R¹, -S(O)₂NR¹R²,-C(O)R¹, et -C(O)NR¹R², dans lesquels R¹ et R² sont indépendamment choisis dans le groupe formé par un atome d'hydrogène, un alkyle, un aryle, un arylalkyle, un hétéroaryle, un hétéroarylalkyle, un cycloalkyle en C₃-C₇, un cycloalkylalkyle, un hétérocycloalkyle, un alkyle substitué, un aryle substitué, un arylalkyle substitué, un hétéroaryle substitué, un hétéroarylalkyle substitué, un cycloalkyle en C₃-C₇ substitué, un cycloalkylalkyle substitué, un hétérocycloalkyle substitué dans lesquels lesdits groupes substitués portent un ou plusieurs substituants choisis parmi un alkyle, un alcoxy, un aralkyle, un hétéroarylalkyle, -NO₂, un alkyloxyalkyle, un alkyloxyalkyloxyalkyle, un cycloalkyle en C₃-C₇, un aryle, -CN, un hétéroaryle, un hétérocycloalkyle, =O, -OH, un amino, un amino substitué, un nitro, et un halogène,
R^{e} et R^{f} sont indépendamment choisis parmi un atome d'hydrogène, un alkyle, un alkyloxyalkyle, un alkyloxyalkyloxyalkyle, un aryle, un arylalkyle, un hétéroaryle, un hétéroarylalkyle, un cycloalkyle en C₃-C₇, un cycloalkylalkyle, un hétérocycloalkyle, un alkyle substitué, un alkyloxyalkyle substitué, un alkyloxyalkyloxyalkyle substitué, un aryle substitué, un arylalkyle substitué, un hétéroaryle substitué, un hétéroarylalkyle substitué, un cycloalkyle en C₃-C₇ substitué, un cycloalkylalkyle substitué, un hétérocycloalkyle substitué, dans lesquels les groupes substitués portent un ou plusieurs substituants choisis parmi un alkyle, un alcoxy, un aralkyle, un hétéroarylalkyle, -NO2, un alkyloxyalkyle, un alkyloxyalkyloxyalkyle, un cycloalkyle en C₃-C₇, un aryle, -CN, un hétéroaryle, un hétérocycloalkyle, =O, -OH, un amino, un amino substitué, un nitro et un halogène, ou R^{e} est choisi dans le groupe formé par un atome d'hydrogène, un alkyle et un aryle, et R^{f} est représenté par -(CH₂)ₙ-R¹⁵, dans laquelle n est un entier de 0 à 8, et R¹⁵ est choisi parmi -C(O)NH₂, -SO₂NH₂, un aryle, un hétéroaryle, un cycloalkyle, un hétérocycloalkyle éventuellement substitués par un alkyle, un alcoxy, un aralkyle, un hétéroarylalkyle, -NO₂, un alkyloxyalkyle, un alkyloxyalkyloxyalkyle, un cycloalkyle en C₃-C₇, un aryle, -CN, un hétérocycloalkyle, =O, -OH, un amino substitué, un nitro, et un atome d'halogène,
ou R¹⁵ est représenté par 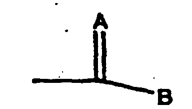 dans laquelle B représente OH ou NH₂ et A représente NH, O, NOH ou NCN, ou R¹⁵ représente NR¹⁶R¹⁷ dans laquelle R¹⁶ représente un atome d'hydrogène, ou un alkyle, et R¹⁷ représente un atome d'hydrogène, un alkyle, SO₂CH₃, ou C(O)NH₂, ou R^{e} et R^{f} forment avec l'atome d'azote auquel ils sont liés un cycle hétérocycloalkyle à 5 ou 6 chaînons qui est éventuellement substitué par OH, NH₂, NHR¹⁶, NHR¹⁷, NR¹⁶R¹⁷ ou (CH₂)ₙR¹⁸R¹⁹, dans lesquels R¹⁶ et R¹⁷ sont tels que définis ci-dessus, R¹⁸ représente un atome d'hydrogène ou un alkyle en C₁-C₆ et R¹⁹ est choisi dans le groupe formé par un atome d'hydrogène, un alkyle en C₁-C₆, un alkyle substitué, un arylalkyle, un acyle (par exemple, un acétyle, benzoyle etc.), un carboxamido, un alkyloxycarbonyle (par exemple un méthoxycarbonyle), un arylalkyloxycarbonyle (par exemple un benzyloxycarbonyle), des dérivés amido dérivés d'acides aminés (par exemple une glycine, une alnine, une sérine, etc.), un imidate (par exemple un phénoxyimidate), un cyanure, un imidamido (par exemple C(=NH)NH₂, (C=NSO₂NH₂)NH₂, etc.), un sulfonamido (par exemple SO₂NH₂, SO₂N(CH₃)₂), un sulfonyle (par exemple SO₂CH₃, SO₂C₆H₅, SO₂CH₂C₆H₅, etc.), un phosphinate (par exemple P(=O)(CH₃)₂), un hétérocyclyle et imidamido (par exemple (C=NC₆H₅)C₆H₃), (C=NH)C₆H₅, etc.), dans lesquels n est tel que défini ci-dessus et R^{h} représente un atome d'hydrogène ou =O ; à condition en outre que lorsque R^{h} représente un atome d'hydrogène, et R^{b} et R^{d} représentent tous deux un atome d'hydrogène, R^{e} représente un atome d'hydrogène et R^{f} représente : 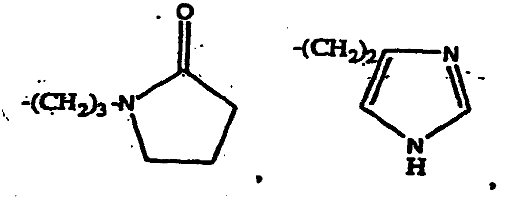 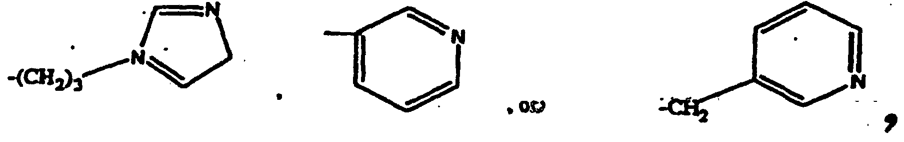 dans lesquelles, sauf indication contraire, les expressions ont les significations suivantes :
« alkyle » (y compris les fragments alkyle d'alcoxy, d'alkylamino et de dialkylamino) représentent des chaînes hydrocarbonées linéaires et ramifiées, et comprenant entre 1 et 20 atomes de carbone ;
« alkyloxyalkyle » représente un alkyle lié à un atome d'oxygène qui à son tour est lié à un alkyle ;
« alkyloxyalkyloxyalkyle » représente un alkyle lié à un atome d'oxygène qui à son tour est lié à un alkyle qui à son tour est lié à un atome d'oxygène qu à son tour est lié à un alkyle ;
« cycloalkyle » représente des cycles carbocycliques saturés, ramifiés ou non-ramifiés, comprenant de 3 à 20 atomes de carbone ;
« hétérocycloalkyle » représente un cycle carbocyclique saturé ramifié ou non-ramifié, contenant de 3 à 15 atomes de carbone, ledit cycle carbocyclique étant interrompu par 1 à 3 groupes hétéro choisis parmi -O-, -S- ou -NR¹⁰-, dans lesquels R¹⁰ représente un atome d'hydrogène, un alkyle, un aryle ou un arylalkyle ;
« aryle » (y compris le fragment aryle d'aryloxy et d'aralkyle) représente un groupe carbocyclique contenant de 6 à 15 atomes de carbone, et possédant au moins un cycle aromatique avec tous les atomes de carbone disponibles du groupe carbocyclique pouvant porter un substituant étant de possibles points de liaison, ledit groupe carbocyclique étant substitué de façon optionnelle (par exemple 1 à 3) par un ou plusieurs atomes d'halogène, groupes alkyle, hydroxy, alcoxy, phénoxy, CF₃, amino, alkylamino, dialkylamino, -COOR¹⁰ ou -NO₂ ;
« halogène » représente le fluor, le chlore, le brome et l'iode ; et
« hétéroaryle » représente des groupes cycliques possédant au moins un hétéroatome choisi parmi O, S ou N, ledit hétéroatome interrompant la structure du cycle carbocyclique et possédant un nombre suffisant d'électrons pi délocalisés pour que la structure soit aromatique, avec les groupes hétérocycliques aromatiques contenant de 2 à 14 atomes de carbone.

2. Composé selon la revendication 1, dans lequel R^{e} représente un atome d'hydrogène, R^{f} représente -(CH₂)ₙR¹⁵ et R¹⁵ est choisi parmi :

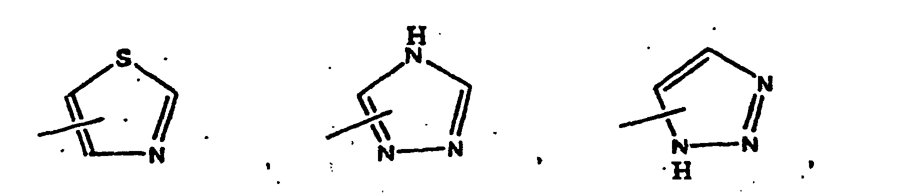

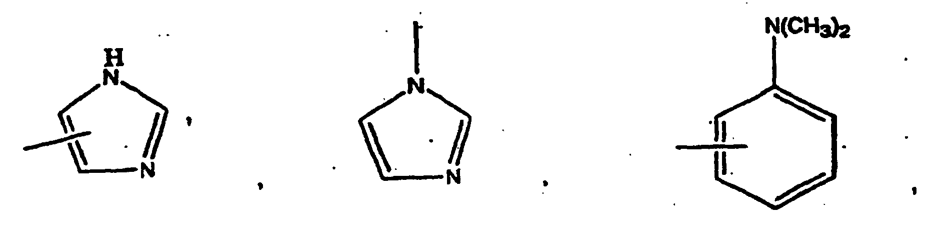

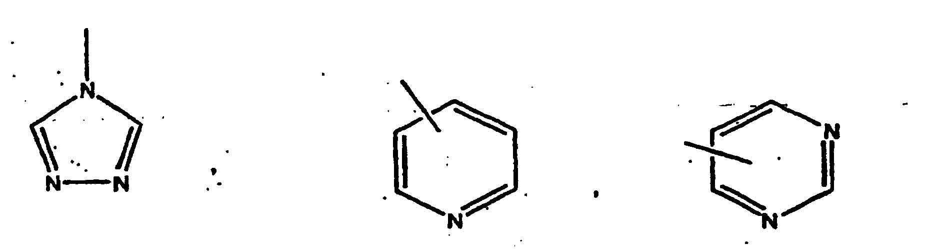

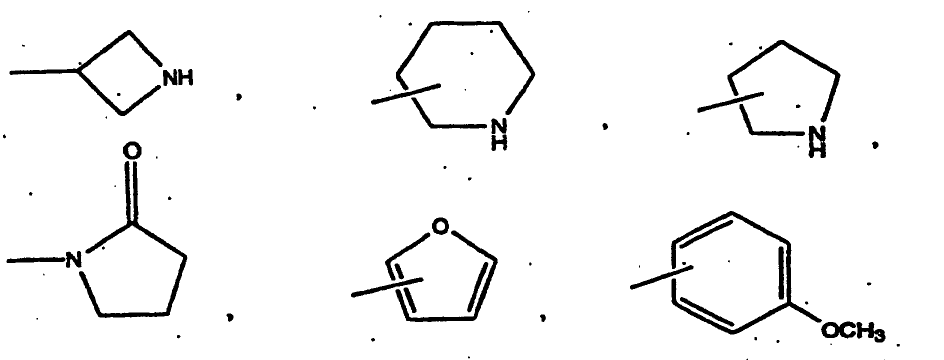

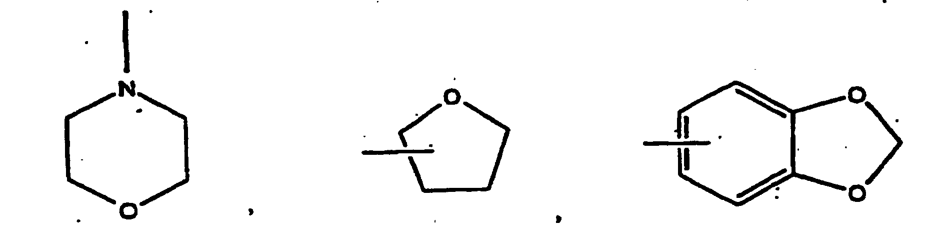

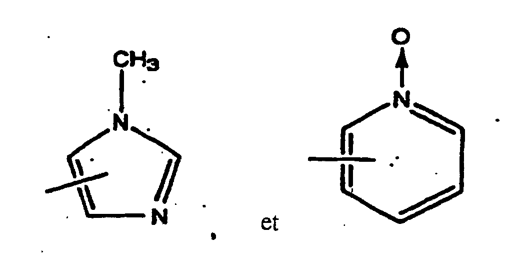

3. Composé selon la revendication 1, dans lequel a représente un atome d'azote et R^{h} représente un atome d'hydrogène.

4. Composé selon la revendication 1, dans lequel R^{a} et R^{d} représentent Br et R^{c} représente Cl.

5. Composé selon la revendication 2, dans lequel R est choisi dans le groupe formé par un atome d'hydrogène, -C(O)(CH₂)₃CH₃, -S(O)₂CH₃, -C(O)-CH₂-O-(CH₂)₂-O-CH₃, -C(O)-CH₂-OCH₃, -C(O)NH(CH₂)₃CH₃,

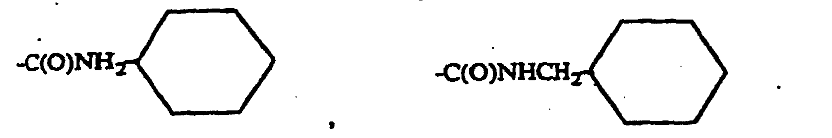

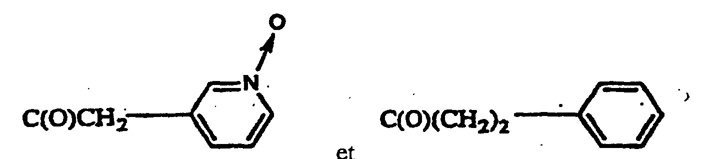

6. Composé selon la revendication 1, dans lequel R^{h} représente =O.

7. Composé selon la revendication 1, dans lequel R^{e} représente un atome d'hydrogène, et R^{f} représente -(CH₂)ₙ-R¹⁵.

8. Composé selon la revendication 1, dans lequel R^{f} représente :

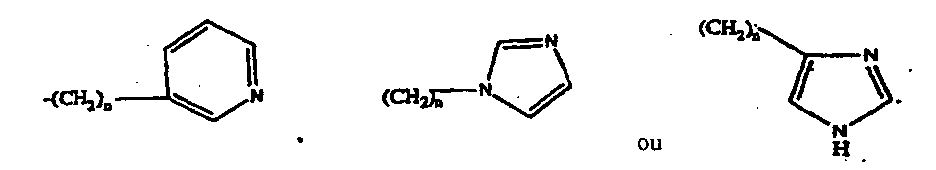

9. Composé ou sel ou produit d'addition de solvant pharmaceutiquement acceptable de ce dernier, choisi dans le groupe constitué de :

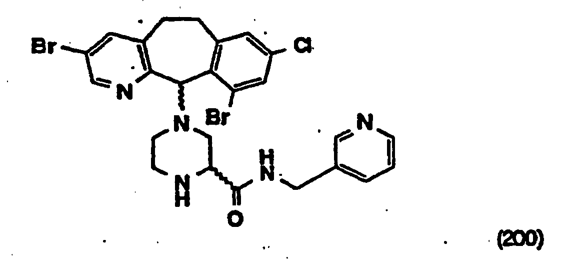

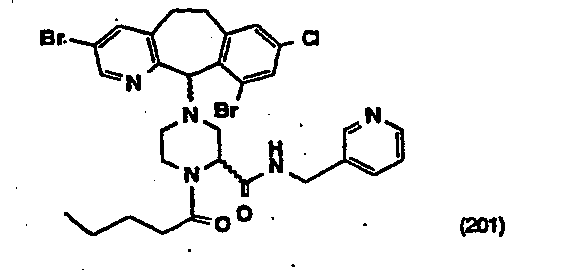

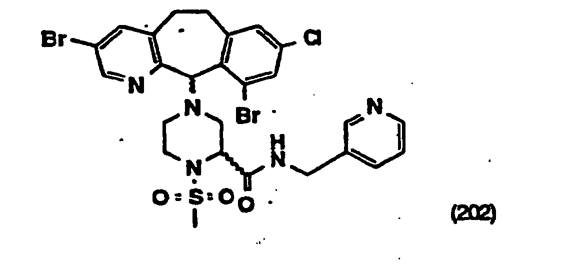

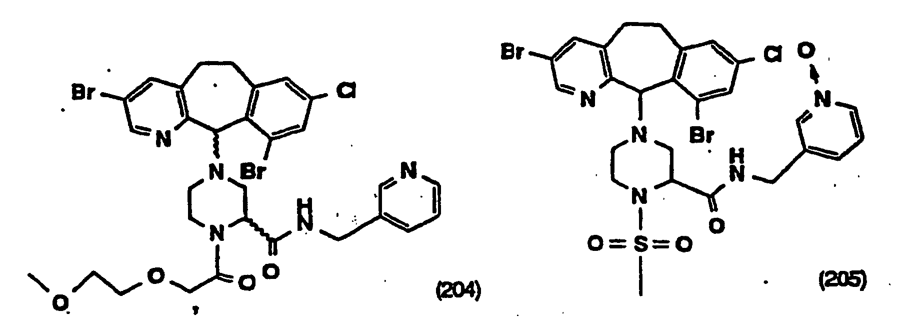

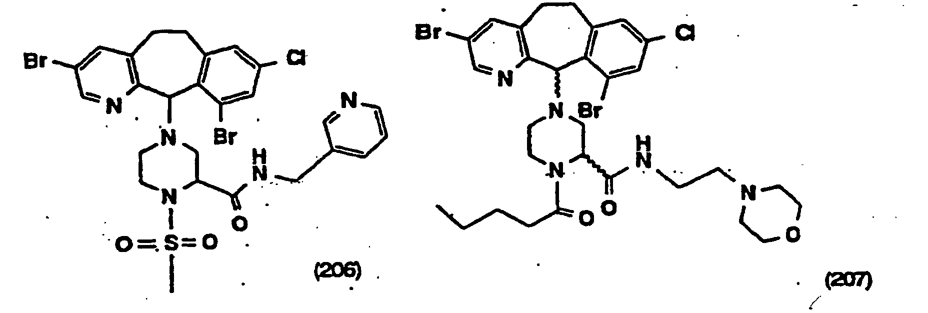

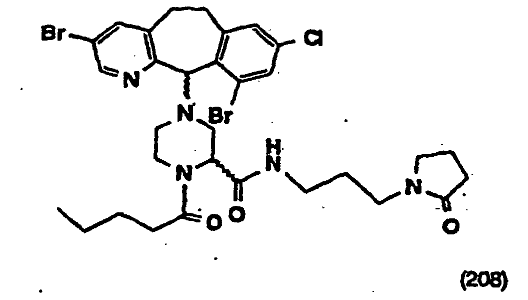

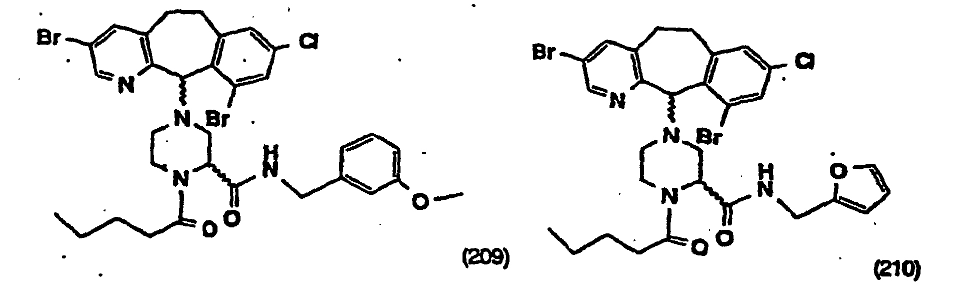

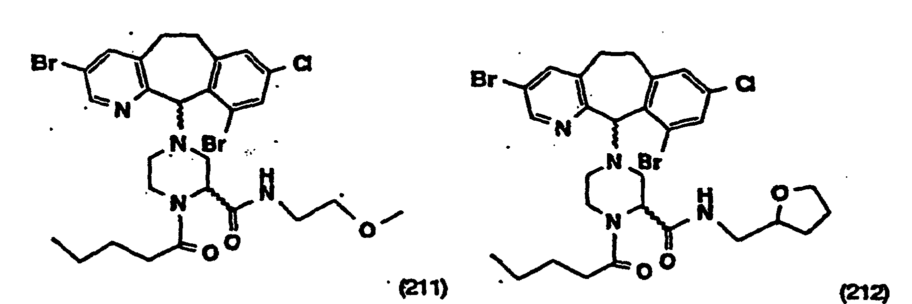

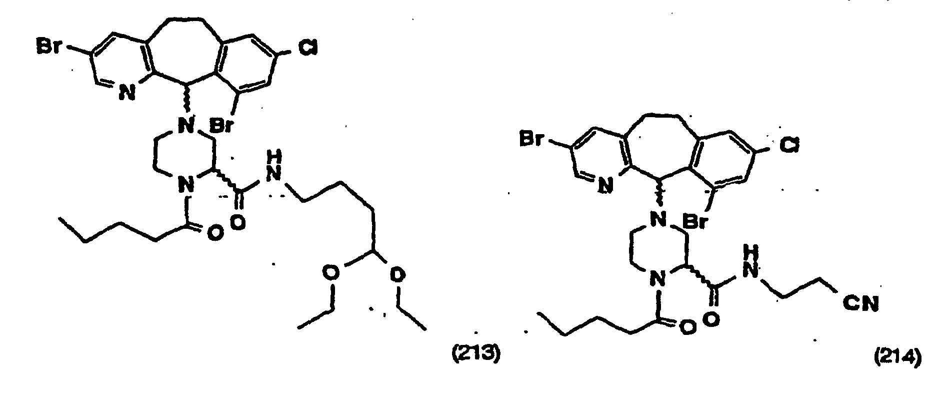

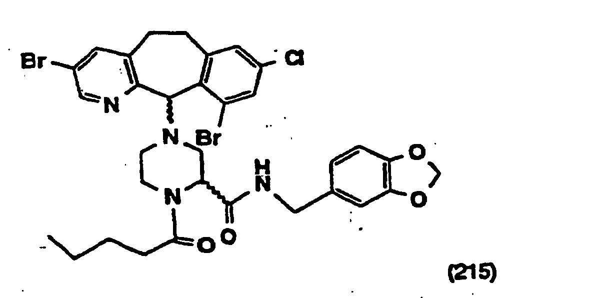

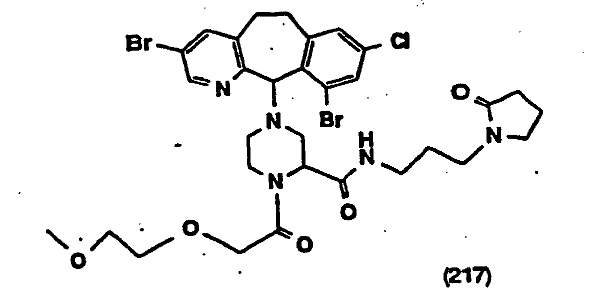

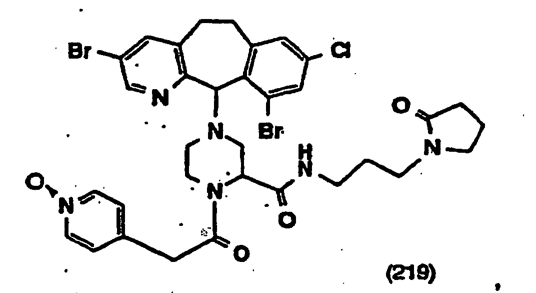

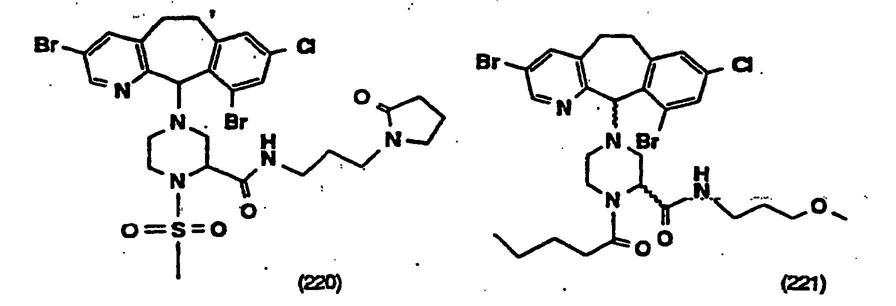

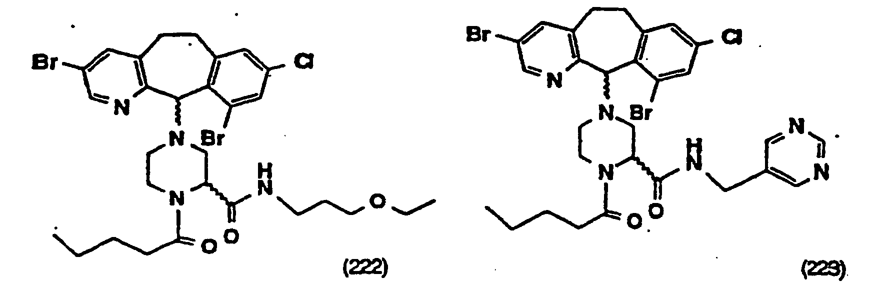

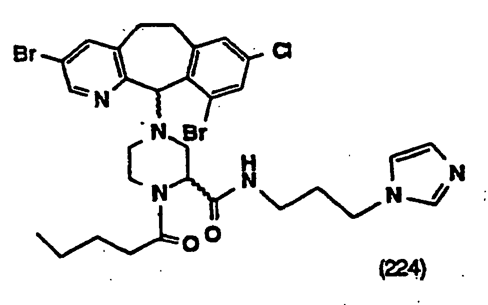

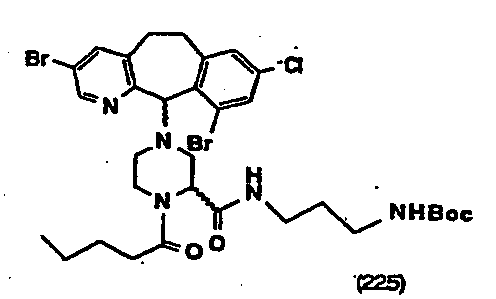

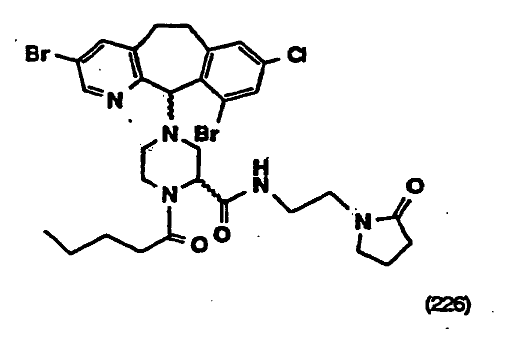

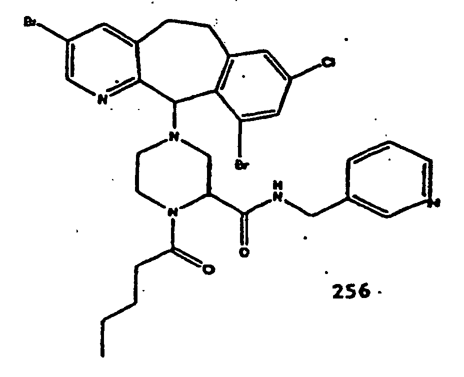

10. Utilisation d'un composé selon l'une quelconque de revendications précédentes, pour la préparation d'un médicament destiné à l'inhibition de la prolifération anormale de cellules.

11. Utilisation selon la revendication 10, dans laquelle les cellules inhibées sont des cellules tumorales symptôme d'un oncogène *ras* activé.

12. Utilisation selon la revendication 10 ou 11, dans laquelle les cellules inhibées sont des cellules de tumeur du pancréas, des cellules cancéreuses pulmonaires, des cellules tumorales de leucémie myéloïde, des cellules de tumeur folliculaire de la thyroïde, des cellules tumorales de syndrome myélodysplasique, des cellules tumorales de carcinome épidermoïde, des cellules tumorales de cancer de la vessie ou des cellules de tumeur du côlon.
